# EUROPEAN PATENT APPLICATION

(11) **EP 4 538 380 A2**
(43) Date of publication of application: **16.04.2025**
(21) Application number: 24219807.5
(22) Date of filing: 21.02.2020
(51) Int. Cl.: C12N 15/85

(54) **METHODS OF MODULATING IMMUNE RESPONSE VIA SNVS OF A20 THAT CAN "TUNE" THE IMMUNE SYSTEM OF A SUBJECT**

(30) Priority: 22.02.2019 AU 2019900574; 26.02.2019 AU 2019900607
(62) Divisional of application: 20759904.4
(71) Applicant: Garvan Institute of Medical Research, Darlinghurst, NSW 2010 (AU)
(72) Inventor: GREY, Shane T, Darlinghurst New South Wales 2010 (AU)
(74) Representative: Haseltine Lake Kempner LLP

(57) **Abstract**

The present disclosure relates generally to the field of immunotherapy. In particular, the present disclosure describes 13 single nucleotide variants (SNVs) within the A20 coding sequence that can differentially impact the immune system. Differential expression of the identified A20 SNVs can be used to "tune" the immune system of a subject e.g., tune up or tune down the sensitivity and/or strength of the immune system. Methods of modulating the immune system of a subject by modulating the A20 SNV expression profile in the subject are disclosed. The methods and reagents of the application are used in the treatment and/or management of diseases/ conditions such as, for example, cancer, autoimmune disease, pathogenic infection and transplant rejection.

## Description

### Cross-Reference to Related Applications

The present application claims priority from Australian Provisional Application No. 20199900574 filed on 22 February 2019 and Australian Provisional Application No. 2019900607 filed on 26 February 2019, the entire contents of which are incorporated herein by way of reference.

### Technical Field

The present disclosure relates to the field of immunotherapy and methods of modulating the immune response.

### Background

Immune balance determines the difference between the immune system recognizing self from non-self. It is recognised that loss, or a breakdown in the inhibitory signal can contribute to autoimmune disease, chronic inflammatory disease and transplant rejection. In cancer, these inhibitory signals are enhanced, thus suppressing the ability of the immune response to efficiently target and destroy tumours. In contrast, in autoimmune disease, these inhibitory signals are diminished, thus enhancing the ability of the immune system to attack when it should not otherwise.

In recent times the clinical development of novel immunotherapies has revolutionised the way many cancers are being treated. Immune checkpoint inhibitors including drugs that target anti-PD-1/PD-L1 and anti-CTLA-4, or immune activators like anti-CD40, have shown great improvements in patient outcomes for several types of cancers including melanoma of the skin, bladder cancer and Hodgkin's lymphoma. These results highlight the key principle that boosting the natural immune response either by removing immune suppressive pathways, or by directly enhancing the immune response, is a promising new approach to improve patient outcomes that could be applied to the treatment of cancer. Further, it highlights that the converse is true for the treatment of autoimmune disease.

### Summary

A20 is encoded by the *TNFAIP3* gene. It promotes microbial tolerance as a negative regulator of NF-κB signaling: an evolutionarily ancient and central pathway for activating innate and adaptive immune responses. It has a critical role in maintaining immune reactivity and function. However, little was known about the ability to modulate the immune response via controlling A20. The inventors have identified and functionally evaluated more than 20 SNVs within the A20 coding sequence that can differentially impact the immune system. The inventors have shown for the first time in both cell and animal models that expression of the identified A20 SNVs can be used to "tune" the immune system of a subject *i.e.,* tune up or tune down the sensitivity and/or strength of the immune system of a subject in response e.g., to a treatment or pathogen. On the basis of these findings, the present inventors have developed new methods for modulating the immune system of a subject comprising modulating the A20 SNV expression profile in the subject, thereby "tuning" the immune system. The inventors have also developed reagents for use in such methods. The inventors envisage that the methods and reagents may have application in the treatment and/or management of diseases/conditions such as, for example, cancer, autoimmune disease, pathogenic infection, complement deficiency, and transplant rejection, where the strength of a subject's immune system plays an important role.

The present inventors have also shown that the A20 SNVs may be used as biomarkers to stratify subjects according to the strength of their immune system *e.g*., strong or poor immune response relative to a reference A20 genotype. The inventors therefore envisage that diagnostic methods which are designed to predict the strength of a subject immune response on the basis of A20 SNVs may be useful in formulating a treatment plan for a subject, as well as ongoing patient management.

Accordingly, the present disclosure provides a method of stratifying a subject according to the strength of their immune response, said method comprising:
(i) determining the nucleotide sequence of the A20 gene in the subject;
(ii) comparing the nucleotide sequence of the A20 gene determined at (i) with a reference A20 gene nucleotide sequence; and
(iii) predicting the strength of the subject's immune response on the basis of the subject's A20 gene nucleotide sequence being identical to the reference sequence at one or more nucleotide positions or on the basis of one or more differences in the nucleotide sequence of the A20 gene in the subject relative to the reference sequence.

In one example, the presence of a A20 single nucleotide variant (SNV) *e.g.,* an A20 sequence comprising a non-synonymous SNV, in the subject relative to the reference A20 nucleotide sequence is indicative that the subject's immune response will be increased or decreased relative to if the subject had the reference A20 nucleotide sequence.

In one example, the presence of a A20 SNV corresponding to an A20 variant set forth in Table 1 in the subject is indicative that the subject's immune response will be increased or decreased relative to if the subject had a nucleotide sequence encoding the reference A20 protein.

In one example, the presence of an A20 SNV corresponding to an A20 variant set forth in Table 2 in the subject is indicative that the subject's immune response will be increased relative to if the subject had a nucleotide sequence encoding the reference A20 protein.

In one example, the presence of an A20 SNV corresponding to an A20 variant set forth in Table 3 in the subject is indicative that the subject's immune response will be decreased relative to if that subject had a nucleotide sequence encoding the reference A20 protein.

The present disclosure also provides a method of modifying the strength of an immune response in a subject, the method comprising administering to, or expressing in, the subject an A20 variant which will increase or decrease the strength of the subject's immune response relative to the subject's immune response in the absence of the A20 variant being administered or expressed.

In one example, the method comprises stratifying the subject according to the strength of their immune response based on the subject's A20 gene nucleotide sequence prior to administering to, or expressing in, the subject the A20 variant. For example, stratifying the subject according to the strength of their immune response may be performed according to the method described herein.

In one example, the A20 variant administered to, or expressed in, the subject is set forth in Table 1. For example, the A20 variant administered to, or expressed in, the subject may be a variant set forth in Table 2. For example, the A20 variant administered to, or expressed in, the subject may be a variant set forth in Table 3.

The A20 variant may be administered to, or expressed in, the subject in any number of ways. In one example, the method comprises administering to the subject an expression vector comprising a nucleic acid coding for the A20 variant. In one example, the method comprises administering to the subject the A20 variant protein. In one example, the method comprises administering to the subject a cell modified to express the A20 variant.

In accordance with an example in which a cell has been modified to express the A20 variant, the cell may be an autologous cell.

In accordance with another example in which a cell has been modified to express the A20 variant, the cell may be an allogeneic cell.

In one example, the cell has been modified to express the A20 variant using a meganuclease, a zinc finger nuclease (ZFN), a transcription activator-like effector-based nuclease (TALEN) or a clustered regularly interspaced short palindromic repeats (CRISPR/Cas) system.

In one example, the cell which has been modified to express the A20 variant is a T cell. For example, the T cell may be a chimeric antigen receptor (CAR) T cell.

In one example, the A20 variant is set forth in Table 2 and the strength of the subject's immune response is increased relative to if the subject expressed a wildtype A20 only or the subject expressed an A20 allele comprising the reference A20 nucleotide sequence only. In accordance with this example, the increase in the subject's immune response comprises an increase in NF-κB and/or c-Jun amino-terminal kinase (JNK) signalling relative to if the subject expressed a wildtype A20 allele only or the subject expressed an A20 allele comprising the reference A20 nucleotide sequence only.

In accordance with an example in which the strength of the subject's immune response is increased by performing the method, the subject may be suffering from cancer. For example, the subject may be suffering from cancer and is receiving treatment by immunotherapy or has been prescribed a treatment by immunotherapy. Accordingly, the present disclosure provides a method and reagents for treating cancer.

In accordance with another example in which the strength of the subject's immune response is increased by performing the method, the subject may be suffering from, or is at risk of, infection by a pathogen. Accordingly, the present disclosure provides a method and reagents for treating or preventing pathogenic infection. For example, the pathogen may be a disease-causing bacteria, virus, protist or fungus. In one example the pathogen is a bacteria. In one example the pathogen is a virus. In one example the pathogen is a protist. In one example the pathogen is a fungus.

In accordance with another example in which the strength of the subject's immune response is increased by performing the method, the subject may be suffering from complement deficiency. Accordingly, the present disclosure provides a method and reagents for treating complement deficiency.

In another example, the A20 variant is set forth in Table 3 and the subject's immune response is decreased relative to if the subject expressed a wildtype A20 allele only or the subject expressed an A20 allele comprising the reference A20 nucleotide sequence only. In accordance with this example, the decrease in the subject's immune response comprises a decrease in NF-κB and/or c-Jun amino-terminal kinase (JNK) signalling relative to if the subject expressed a wildtype A20 allele only or the subject expressed an A20 comprising the reference A20 nucleotide sequence only.

In accordance with an example in which the strength of the subject's immune response is decreased by performing the method, the subject may be suffering from an autoimmune disease.

In accordance with another example in which the strength of the subject's immune response is decreased by performing the method, the subject may have undergone or is about to undergo organ or tissue transplantation.

The present disclosure also provides a method of treating cancer in a subject in need thereof, comprising increasing a subject's immune response by performing the method of the disclosure and administering to the subject an immunotherapeutic agent.

In one example, the immunotherapeutic agent is selected from the group consisting of checkpoint inhibitors, monoclonal antibodies, chimeric antigen receptor (CAR) T cells, and small molecule immune agonists.

The present disclosure also provides a method of treating an autoimmune disease in a subject, comprising repressing a subject's immune response by performing the method of the disclosure.

In one example, the autoimmune disease is associated with activation of the innate immune system or auto-inflammatory response e.g., familial Mediterranean fever; Behcet disease. In another example, the autoimmune disease is associated with activation of the adaptive immune response or autoimmunity e.g., type-1 diabetes, lupus, irritable bowel syndrome (IBS), rheumatoid arthritis, multiple sclerosis, Hashimoto's disease, Celiac disease, Crohn's disease and asthma, for example.

The present disclosure also provides an isolated nucleic acid molecule comprising a nucleotide sequence encoding an A20 variant set forth in Table 1. In one example, the isolated nucleic acid may comprise a nucleotide sequence encoding an A20 variant set forth in Table 2. In another example, the isolated nucleic acid may comprise a nucleotide sequence encoding an A20 variant set forth in Table 3.

The present disclosure also provides an expression vector comprising the isolated nucleic acid molecule of the disclosure.

The present disclosure also provides a delivery system e.g., a viral delivery system, comprising am isolated nucleic acid or expression vector described herein. For example, the delivery system may be an adeno-associated virus (AAV), a lentivirus, or an adenovirus.

The present disclosure also provides isolated protein comprising an A20 variant set forth in Table 1.

The present disclosure also provides isolated protein comprising an A20 variant set forth in Table 2.

The present disclosure also provides isolated protein comprising an A20 variant set forth in Table 3.

The present disclosure also provides T cell which is modified to express an A20 variant selected from Table 2. For example, the T-cell may be a chimeric antigen receptor (CAR) T cell.

The present disclosure also provides a pharmaceutical composition comprising (i) one or more of an isolated nucleic acid of the disclosure or an expression vector of the disclosure or an isolated protein of the disclosure or a T-cell of the disclosure, and (ii) a pharmaceutically acceptable carrier, diluent or excipient.

The present disclosure also provides a vaccine comprising an A20 protein selected from Table 2 or an isolated nucleic acid molecule comprising a nucleotide sequence encoding an A20 variant set forth in Table 2.

### Brief description of the drawings

The following figures form part of the present specification and are included to further demonstrate certain aspects of the present disclosure. The disclosure may be better understood by reference to one or more of these figures in combination with the detailed description of specific embodiments presented herein.

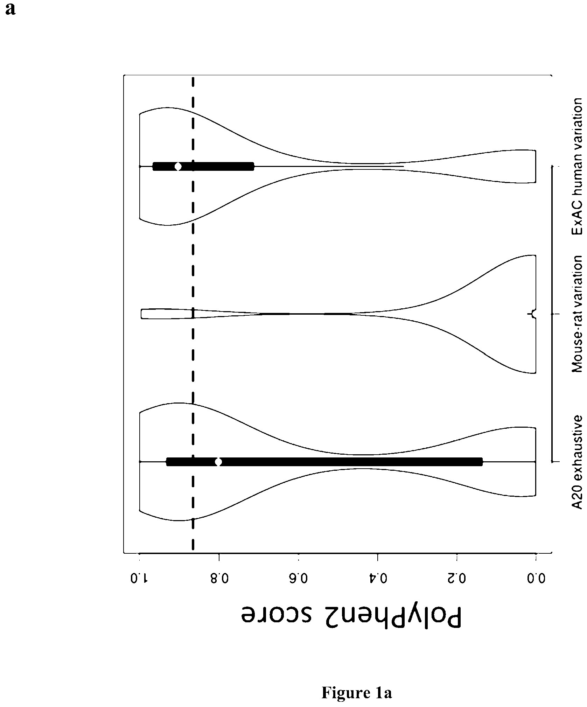

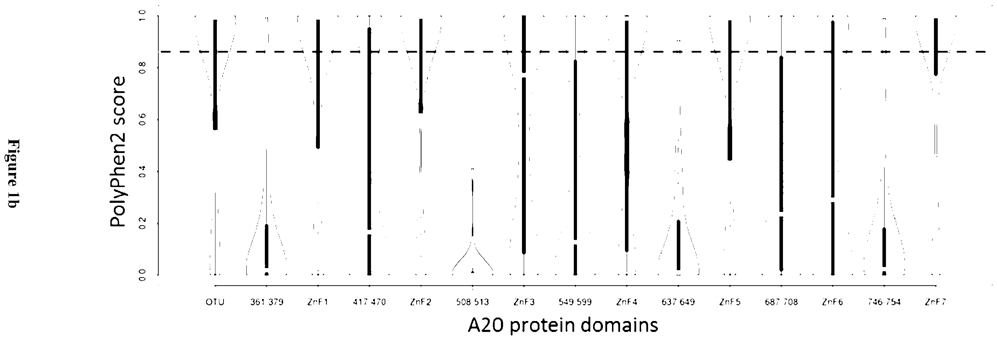

**Figure 1** is a violin plot showing the distribution of non-synonymous single nucleotide variations (nsSNVs) classified as benign or deleterious within the A20 locus from human genome sequence data using the ExAC database PolyPhen2.

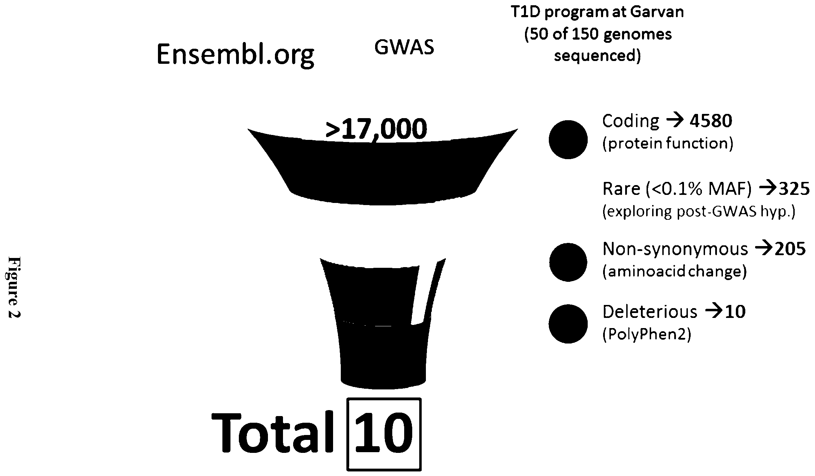

**Figure 2** illustrates the filtering strategy that was used for evaluation of human A20 variants.

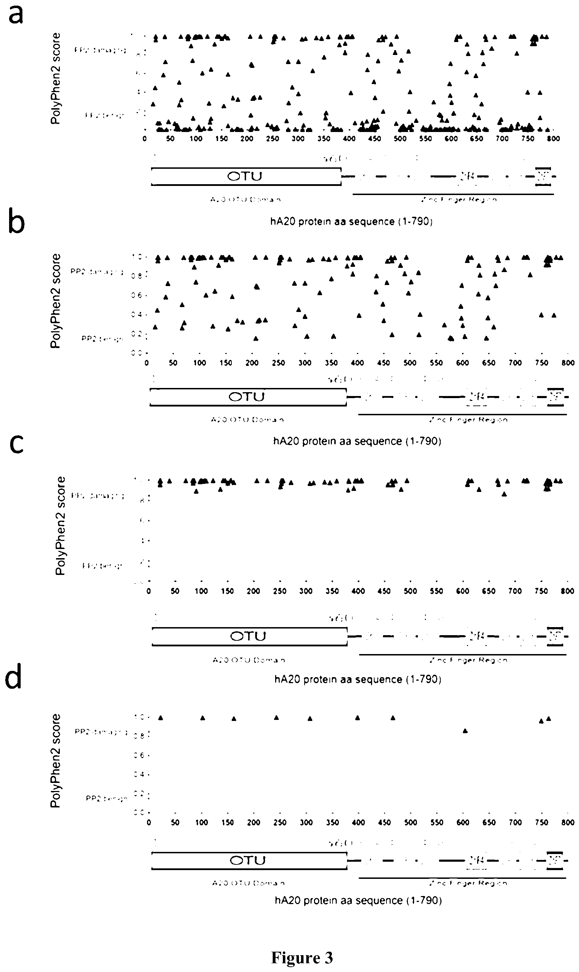

**Figure 3** shows individual human A20 variant functional consequences as predicted by PolyPhen2 plotted against the human A20 protein sequence and domains. **(a)** All 205 coding non-synonymous variants with a PolyPhen2 score present in *TNFAIP3* canonical transcript of (ENST00000612899.4). The following figures show, with increasing order of deleteriousness and rarity in the human genome, the filtering of human A20 variants. **(b)** All possibly and probably damaging variants (PolyPhen2 score 0.15-1.0), **(c)** all probably damaging variants (PolyPhen2 score ≥0.85-1.0), and **(d)** all damaging variants (PolyPhen2 ≥0.85-1.0) that are also rare (MAF 0-0.1). Data collected from Ensembl.org database.

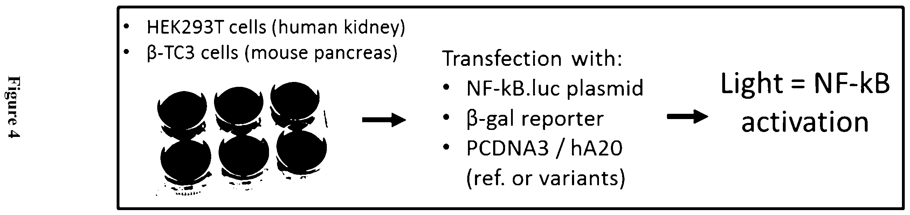

**Figure 4** shows the functional testing performed for human A20 variants in the cell reporter assay for assessment of NF-κB activation.

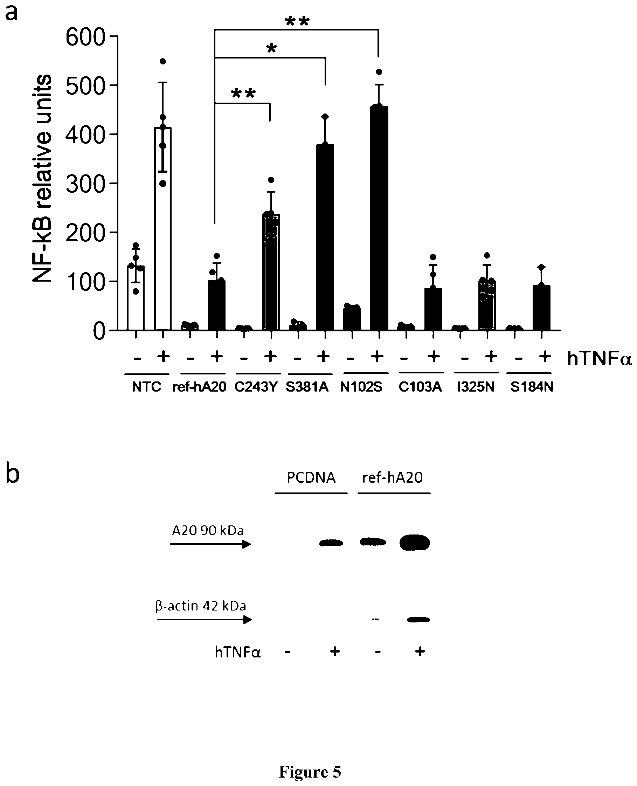

**Figure 5** shows NF-κB activation response across HEK293T cells transiently transfected with human A20 variants. **(a)** Shown from left to right are representative results from cells transiently transfected with NTC, reference human-A20, hA20-C243Y (rare-deleterious), hA20-S381A (LoF), hA20-N102S (rare-deleterious), hA20-C103A (LoF), hA20-I325N (ENU mutant) and hA20-S184N (rare-benign). Data represented as mean ± SD, *P-*values were determined using Mann-Whithney test. **P* < 0.05, ***P* < 0.01. Each dot represents an individual experiment. These data are representative of N = 3 to 5 experiments. **(b)** Representative western blot showing the level of expression of A20 in cells transfected with PCDNA (NTC) and ref-hA20, harvested prior hTNFα stimulation and 8 hours after hTNFα treatment.

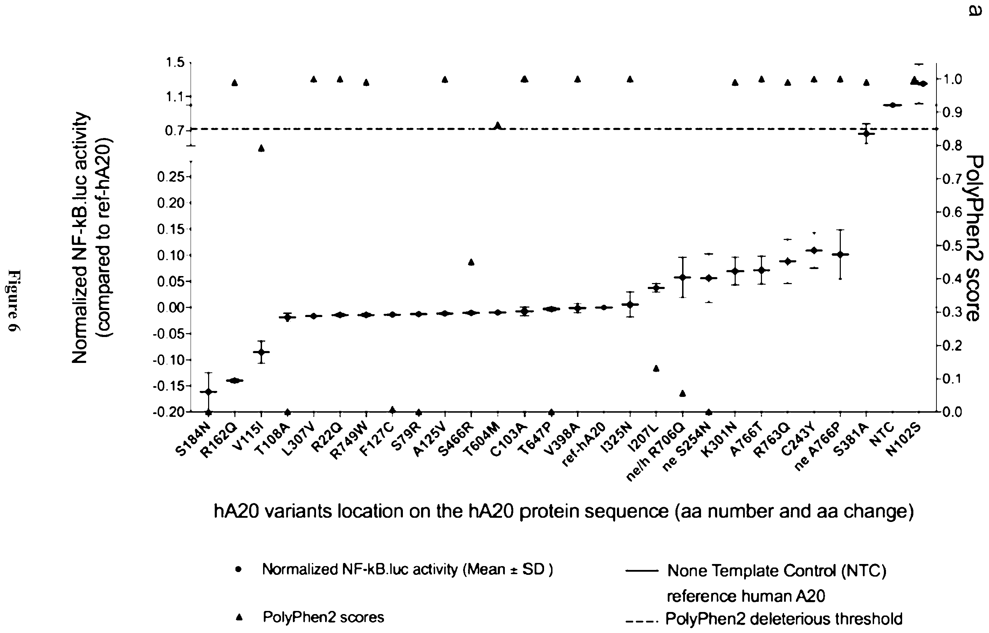

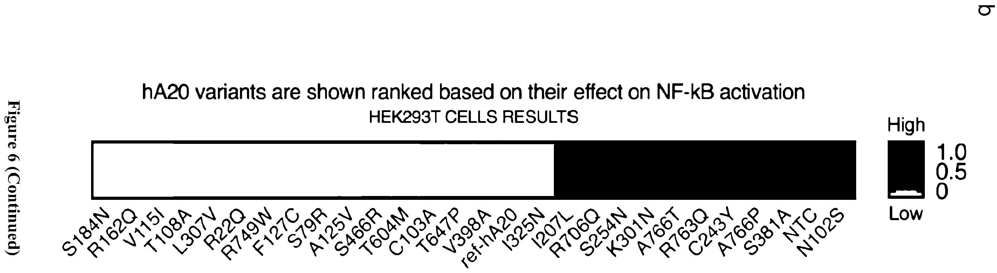

**Figure 6** shows the cumulative data of normalized NF-κB luciferase activity per each tested A20 variant vs its respective PolyPhen2 score in the HEK293T cell line and normalized NF-κB luciferase activity in the form of a heat map. **(a)** The y-axis on the left shows the normalized NF-κB luciferase values for each of the variants tested. Values were normalized against each experiment's NTC (hTNFα stimulated), set to a value of 1 (red line), and reference human A20 (hTNFα stimulated), set to a value of 0 (green line). The y-axis on the right shows the PolyPhen2 scores (purple triangles) for each variant ranging from 0 to 1. The dotted purple line is the threshold above which variants are predicted to be deleterious by PolyPhen2 (set to a value of 0.85). The x-axis shows the amino acid position of the A20 variants tested.**(b)** NF-κB luciferase activity for each of the variants tested was normalized against each experiment's NTC+ (hTNFα stimulated), set to a value of 1, and reference human-A20+ (hTNFα stimulated), set to a value of 0. Below each heat map are reported the variants IDs, shown with the classic nomenclature for amino acid change and position in the protein sequence. The '+' next to each variant is to indicate that the normalized value was taken from its stimulated state. NTC+, ref-hA20 and A20 variants tested in HEK293T cells were normalized and organized in order of appearance in the human A20 protein sequence. Each dot represents an experiment with an N of 3-5 replicates per variant.

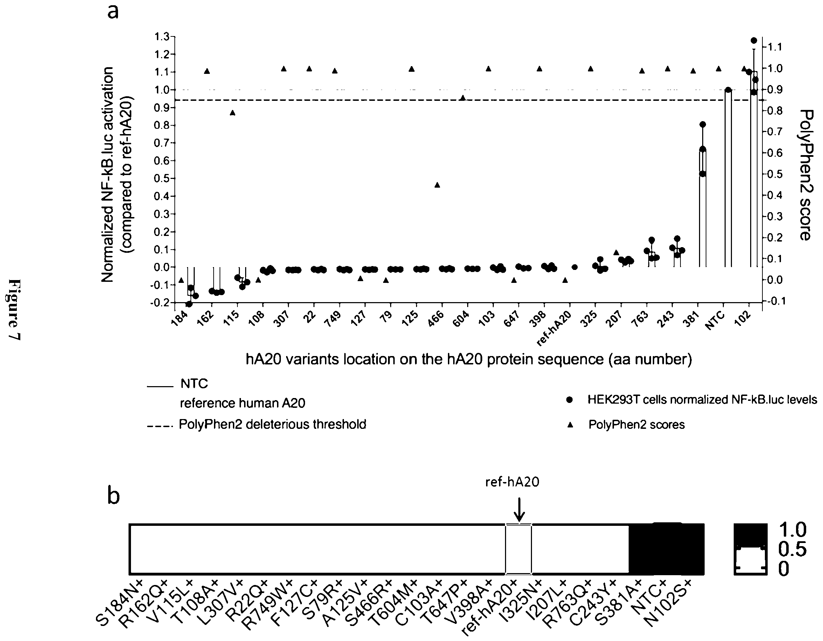

**Figure 7** shows the Cumulative data of normalized NF-κB luciferase activity per each tested A20 variant vs their respective PolyPhen2 score in the HEK293T cell line and normalized NF-κB luciferase activity in the form of a heat map. **(a)** The y-axis on the left shows the normalized NF-κB luciferase values for each of the variants tested. Values were normalized against each experiment's NTC (hTNFα stimulated), set to a value of 1 (red line), and reference human A20 (hTNFα stimulated), set to a value of 0 (green line). The y-axis on the right shows the reported the PolyPhen2 scores (purple triangles) for each variant ranging from 0 to 1. The dotted purple line is the threshold above which variants are predicted to be deleterious by PolyPhen2 (set to a value of 0.85). The x-axis shows the amino acid position of the A20 variants tested. Variants are ordered based on their ability to suppress NF-κB (most suppressive on the left, least suppressive on the right). **(b)** NF-κB luciferase activity for each of the variants tested was normalized against each experiment's NTC+ (hTNFα stimulated), set to a value of 1, and reference human-A20+ (hTNFα stimulated), set to a value of 0. Below each heat map are reported the variants IDs, shown with the classic nomenclature for amino acid change and position in the protein sequence. The '+' next to each variant is to indicate that the normalized value was taken from its stimulated state. A20 variants tested in HEK293T cells were ordered based on their ability to suppress NF-κB. Each square represents the mean NF-κB luminescence value from a minimum of three experiment in which a variant has been tested three times.

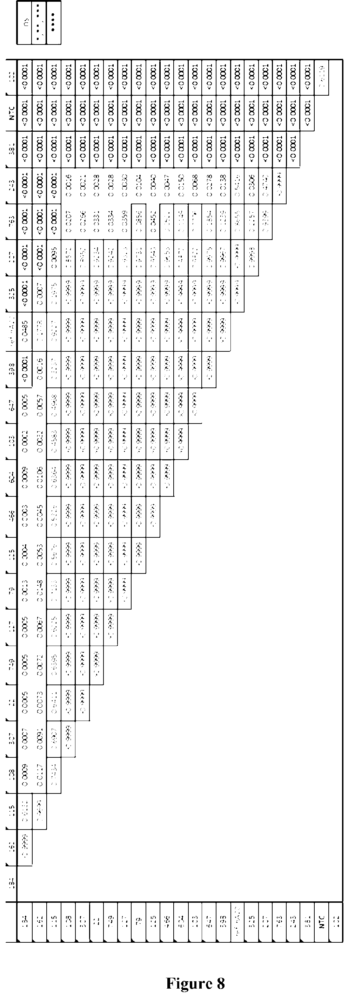

**Figure 8** shows the statistical correlation between variants tested in HEK293T cells. Variants are ordered based on the normalized functional ability to inhibit NF-κB, from the most effective (top left corner) to the most impaired (far left / bottom). In green are highlighted ref-hA20 (in respect to the variants activity) and *P-values* that are not significant. In red are highlighted NTC (in respect to the variants' activity) and ***** P-values <* 0.0001. In orange are highlighted *, ** and **** P-values* 0.05, 0.01 and 0.001 respectively. Statistical analyses were carried out using One-way ANOVA.

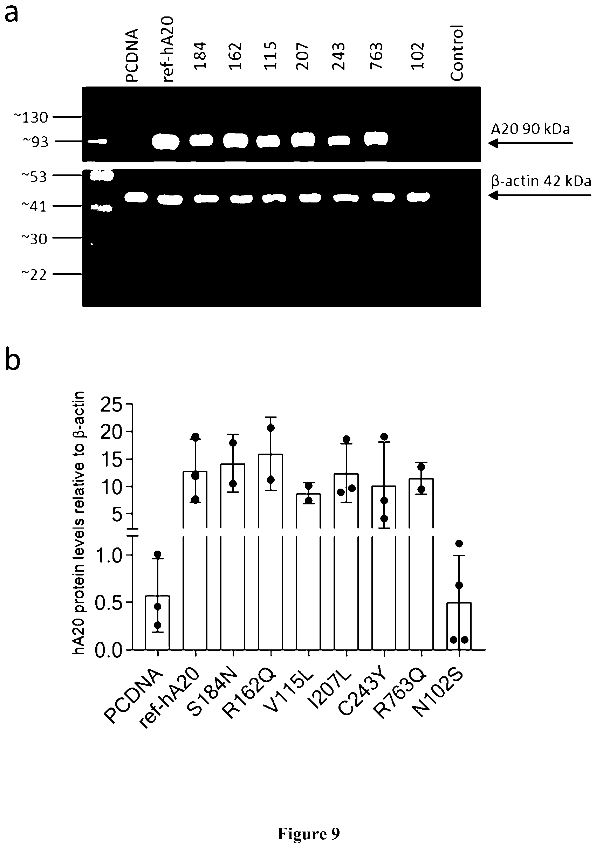

**Figure 9** shows A20 protein expression as determined by western blot of core GoF and impaired A20 variants. **(a)** Representative western blot membrane highlighting the presence of hA20 in the cell lysates from HEK293T cells transfected with PCDNA, ref-hA20 and hA20 variants. Below, β-actin protein present in the same lysates used to quantify hA20's protein levels. **(b)** Human A20 protein levels relative to β-actin. This graph shows that only the endogenous hA20 is present in the PCDNA transfected cells, whereascomparable hA20 protein levels are expressed in cells expressing A20 variatns, with the exception of N102S transfected cells. The low expression level for N102S might indicate a misfolding and subsequent degradation of the protein bearing this mutation, allowing for the detection only of the endogenous hA20 in the cells. This data represents N = 2-3 experiments.

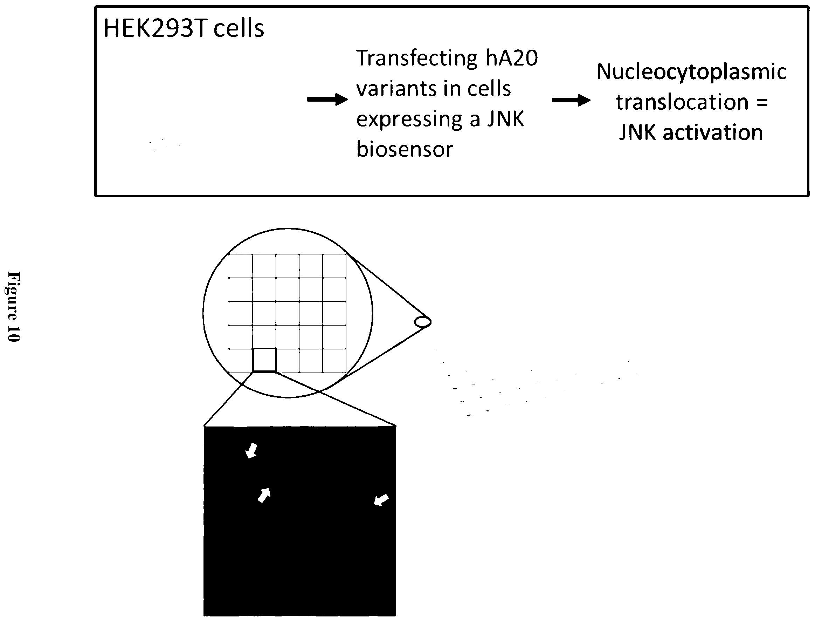

**Figure 10** illustrates the functional testing performed for human A20 variants in the cell reporter assay to assess JNK activation.

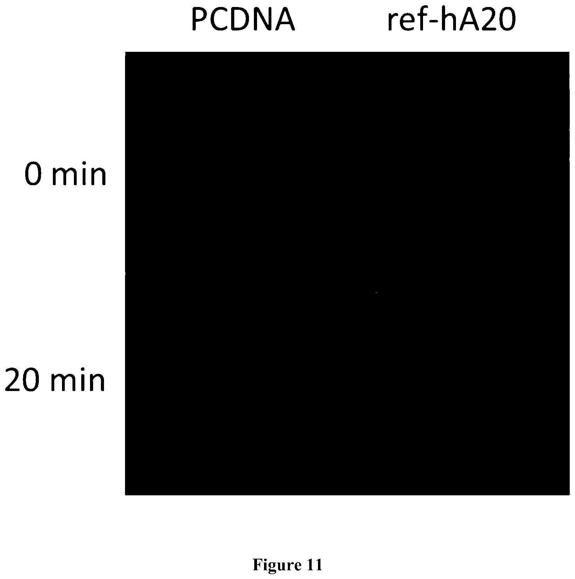

**Figure 11** is a representative image of JNK mCherry marker activation in HEK293T cells transfected with PCDNA (left column) and reference-humanA20 (right column). Cells snapshot taken at time 0, prior hTNFα stimulation, and at 20 minutes post-hTNFα stimulation, where PCDNA transfected cells reach their maximum peak of JNK activation. Images are false colors representations of the field captured by the Thermo Scientific Cellomics Arrayscan machine.

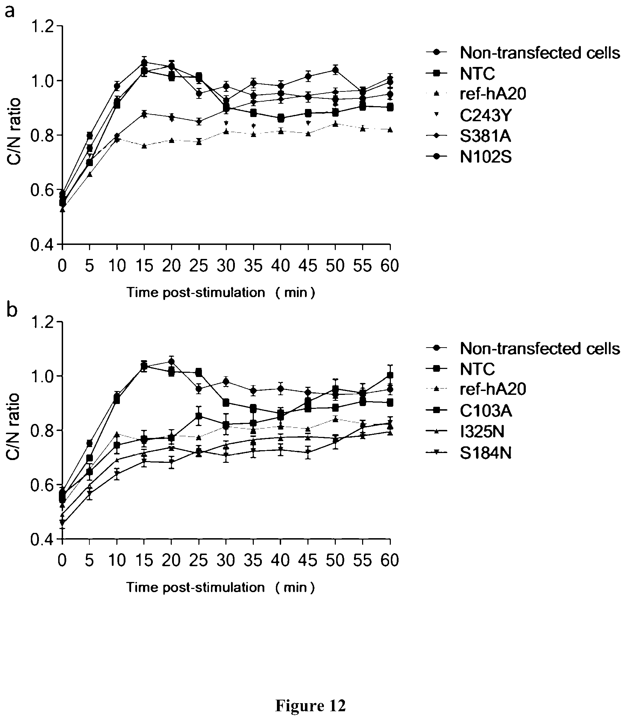

**Figure 12** shows JNK activation kinetics of HEK293T mCherry-JNK cells upon hTNFα stimulation. These representative graphs show the activation dynamics for JNK, deleterious **(a)** or benign **(b),** when cells are transiently transfected with hA20 variants and stimulated with hTNFα for 1 hour at 5 minutes intervals. The induction peak of non-transfected and NTC transfected cells is at 20 minutes post-stimulation, making it the static reference point for assessing variants. Each of the two graphs reports the dynamics for non-transfected cells, NTC and ref-hA20. The graphs have been split into two for clarity. Data represented as mean ± standard error.

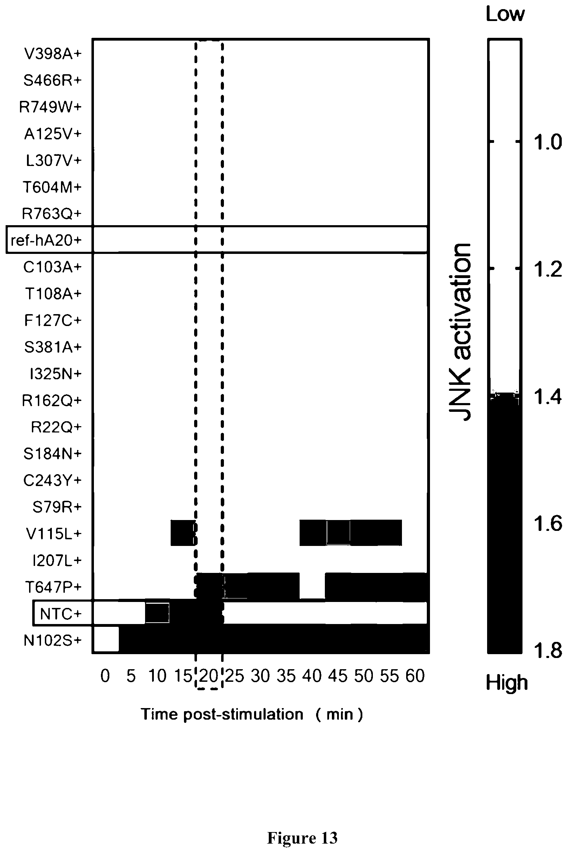

**Figure 13** is a heatmap showing normalised JNK activation through time for each of the transfected hA20 variants in relation to the ref-hA20 and NTC transfected cells. JNK activity is reported for each of the hA20 variants and NTC transfected cells upon human TNFα stimulation. Variants were ordered based on JNK activation at the 20 minute time point (highest activation peak in the NTC transfected cells). Top of the graph, variants showing the weakest JNK activation at 20 min, bottom of the graph, variants with the highest JNK activation at 20 min. On the right of each row are reported the variants IDs, shown with the classic nomenclature for amino acid change and position in the protein sequence. The '+' next to each variant is to indicate that the normalized value was taken from its stimulated state. Each square represents the mean of at least two experiments performed in duplicate and assessing a minimum of 150k cells up to 360k cells.

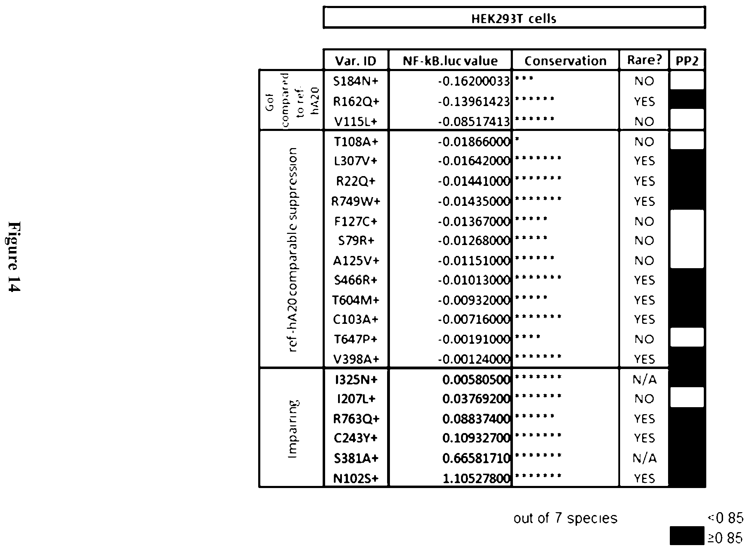

**Figure 14** shows the normalized NF-κB luciferase activity of the human A20 variants tested ordered based on their ability to suppress NF-κB. Groups were identified based on the clear separation of variants effect. GoF variants compared to ref-hA20 (in blue); ref-hA20 comparable variants (in green); impairing variants (in red).

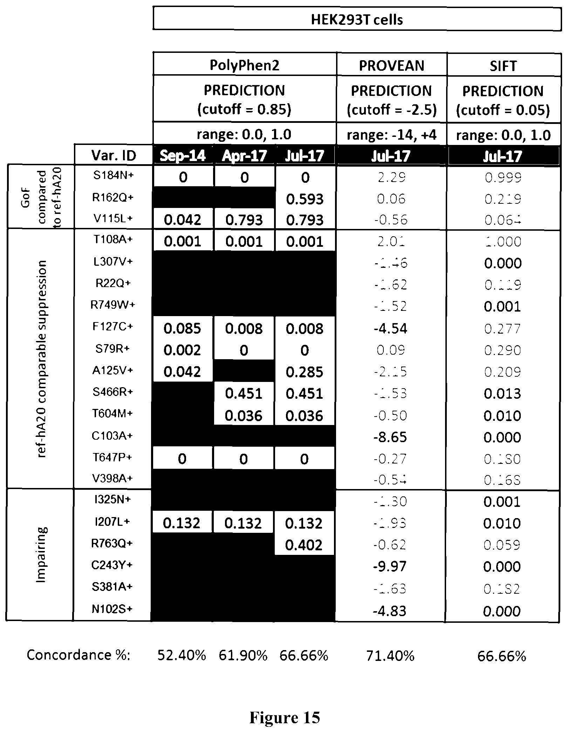

**Figure 15** shows the deleteriousness predictions for each of the tested human A20 variants according to PROVEAN, SIFT and PolyPhen2 algorithms. A20 variants tested in HEK293T cells were ordered based on their ability to suppress NF-κB, with A20 variants exhibiting the highest NF-κB inhibitory activity represented at the top and A20 variants exhibiting the most impaired NF-κB inhibitory activity at the bottom. GoF variants compared to ref-hA20 (in blue); ref-hA20 comparable variants (in green); impairing variants (in red). Each variant was compared to the predictions of three deleteriousness assessing algorithms: PROVEAN, SIFT and PolyPhen2.

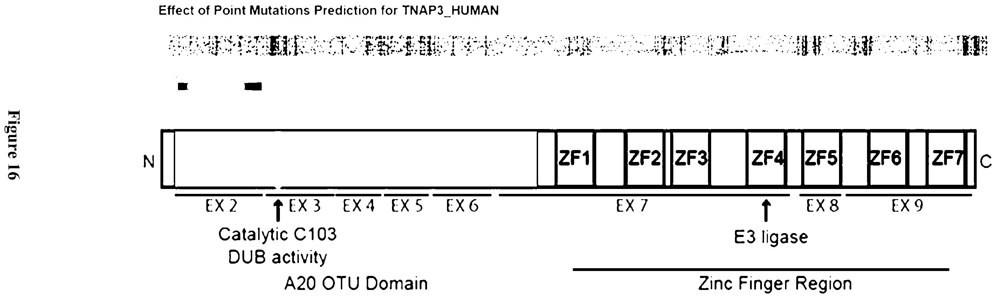

**Figure 16** shows the effect of point mutation predictions according to ProteinPredict online tool. Here is shown A20 amino acid sequence and all the possible amino acid substitution (top), and A20 cartoon with the relevant domains (bottom). Each substitution is presented as a heatmap. Dark red indicates a high score (score > 50, strong signal for effect), white indicates weak signals (-50 < score < 50), and green a low score (score < -50, strong signal for neutral/no effect). Black marks the corresponding wildtype residues.

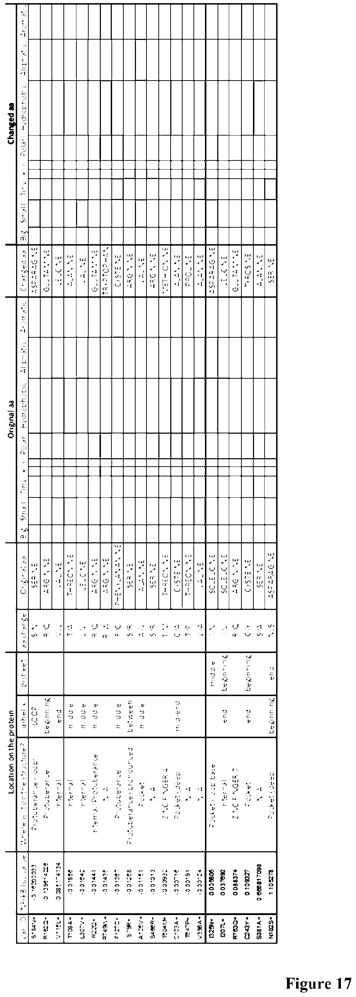

**Figure 17** shows a summary of the human A20 variants tested according to their normalized NF-κB luciferase activity and their position in the protein structure (where available). A20 variants tested in HEK293T cells were ordered based on their ability to suppress NF-κB. GoF variants compared to ref-hA20 presentedin blue), ref-hA20 comparable variants are presented in green, and impairing variants are presented in red. For each variant, the relative position in the protein structure is indicated (where available), as is whether the variant is located on alpha-helix or beta-sheet structures. Amino acid characteristics for each A20 variants are also presented in this figure.

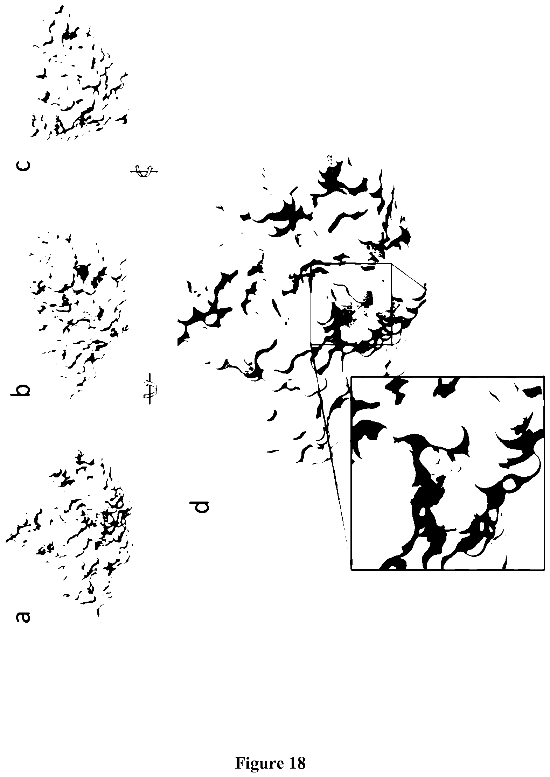

**Figure 18** shows the 3D structure of the A20's OTU domain and variants tested falling in this domain. A20's OTU domain protein structure represented as semi-transparent surface for amino acid identification: **(a)** frontal, **(b)** rotated 90° along the horizontal plane, **(c)** rotated 180° along the vertical plane. **(d)** Close-up of the catalytic site of A20 (C103). The different colours of each amino acid and their relative surface exposure are: GWAS variants A125 and F127 (blue), catalytic site C103 (pink), benign variants scored according to the NF-κB luciferase assay (green) and impaired variants scored according to the NF-κB luciferase assay (red). Images generated with PyMOL software.

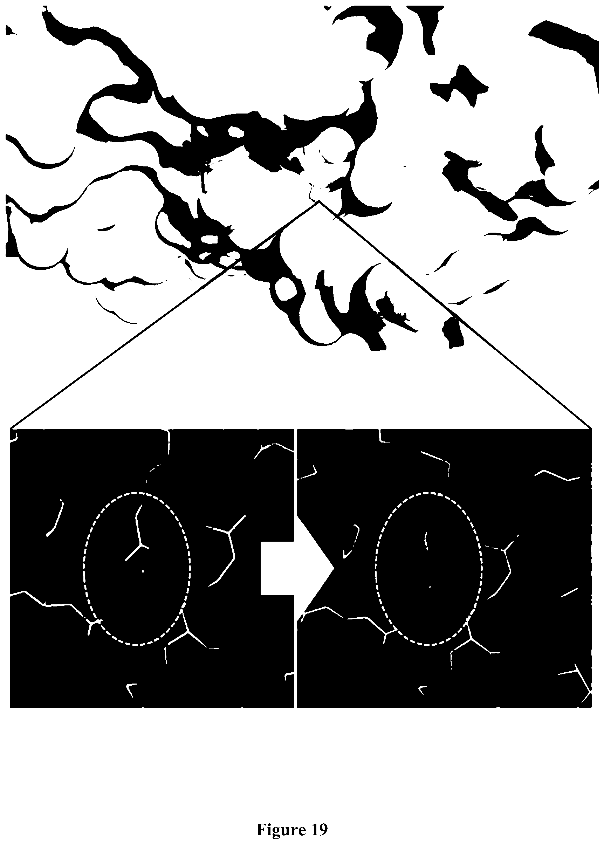

**Figure 19** shows the effect of amino acid change for the A20 variant N102S. A20 variant N102S, Asparagine to Serine, structural change shows interaction impairment with two other A20's residues (A117 and A123). The latter residues, previously interacting with covalent bonds with the two arms of the asparagine are too distant to interact with the one arm of the serine. Images generated with PyMOL and Coot softwares.

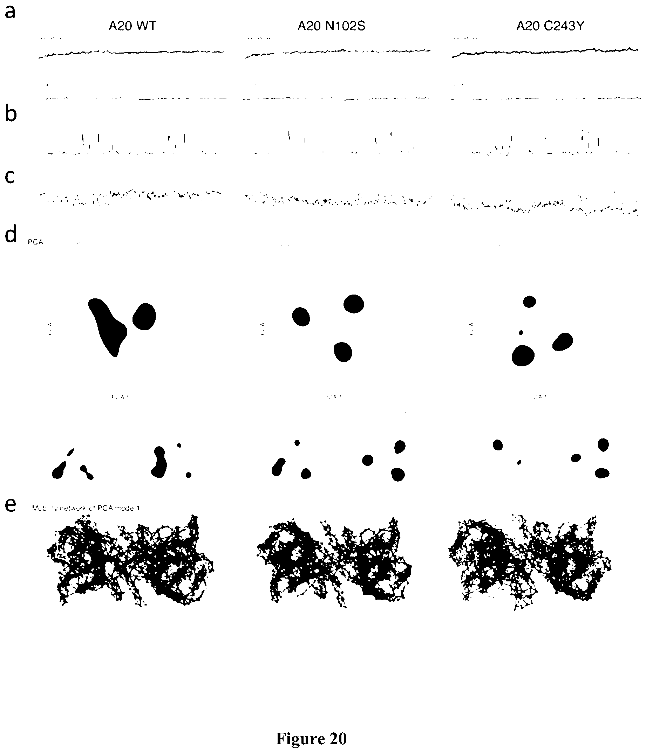

**Figure 20** shows molecular dynamics simulations of A20-WT, N102S and C243Y variants. A20 variants N102S and C243Y molecular dynamics were simulated over a theoretical period of 500 ns at 300 K. (a-b) Root-mean-square-deviation and -fluctuation (RMSD and RMSF respectively) showed an increased atomic fluctuation of the C243Y mutant compared to A20-WT and N102S. **(c)** The radius of gyration of the A20 variants homodimers shows a similar level of compactness for A20-WT and N102S but a reduction in gyration for the C243Y variant. **(d)** Principal component analysis (PCA) simulations show smoother transitions (both in homodimers and single chains) for the A20-WT. In contrast, N102S and C243Y indicate that less time is spent transitioning from one position to the other, resulting in a more constrained overall geometry. **(e)** PCA mapped over an interaction network shows local flexibility of the structure, located around the catalytic site for both A20-WT and N102S with the latter exhibiting a reduced dynamics. C243Y displays an increased dynamic around the loops surrounding the mutation.

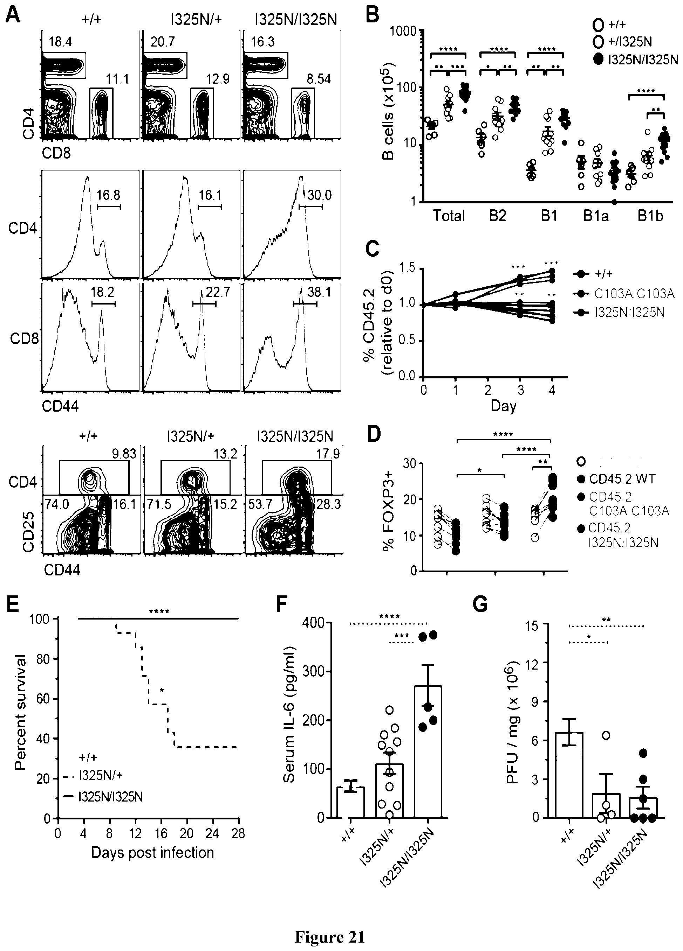

**Figure 21** illustrates that the I325N OTU variant of A20 confers heightened immunity in mice. **(A)** Representative flow cytometric analysis of splenocytes, showing percentage of CD4⁺ or CD8⁺ T cells, and percentage CD44^{hi} activated/memory cells within these subsets or percentage CD25⁺ CD44^{int} regulatory T- cells or CD25⁻ CD44^{high} effector/memory T cells among CD4⁺ cells. **(B)** Numbers of indicated B cells in peritoneum of individual *Tnfaip3* mice (circles). **(C, D)** Wild-type CD45.1⁺ mice transplanted with equal mixtures of congenic bone marrow from *Tnfaip3*^{*+*/*+*} CD45.1⁺ donors and CD45.2⁺ donors of *Tnfaip3*^{I325N/I325N}*, Tnfaip3*^{C103A/C103A} or *Tnfaip3*^{*+*/*+*} genotypes. **(C)** Splenocytes from chimeras cultured with 0.1 µg/ml LPS for 0-4 days, and the frequency of CD45.2⁺ cells of the indicated *Tnfaip3* genotypes was measured among B cells from individual chimeras (lines), expressed relative to their frequency on day 0. **(D)** Pairwise comparison of frequency of FOXP3⁺ cells amongst CD45.2⁺ and CD45.1⁺ CD4⁺ splenocytes from the same chimera. **(E-G)** Female *Tnfaip3*^{*+*/}*⁺ (n=15), Tnfaip3*^{I325N/+} (*n=14*) or *Tnfaip*^{3I325N/I325N} (*n=10*) C57BL/6 mice were injected with Coxsackie B4 virus strain E2 on day 0. **(E)** Kaplan-Meier survival data (Log-rank test). **(F)** Plasma IL-6 and **(G)** virus titers (plaque forming units, PFU) per mg of pancreas on day 3. Error bars represent SEM and one-way ANOVA used for significance analysis unless otherwise stated, **P* < 0.05; ***P* < 0.01; ****P* < 0.001; *****P* < 0.0001.

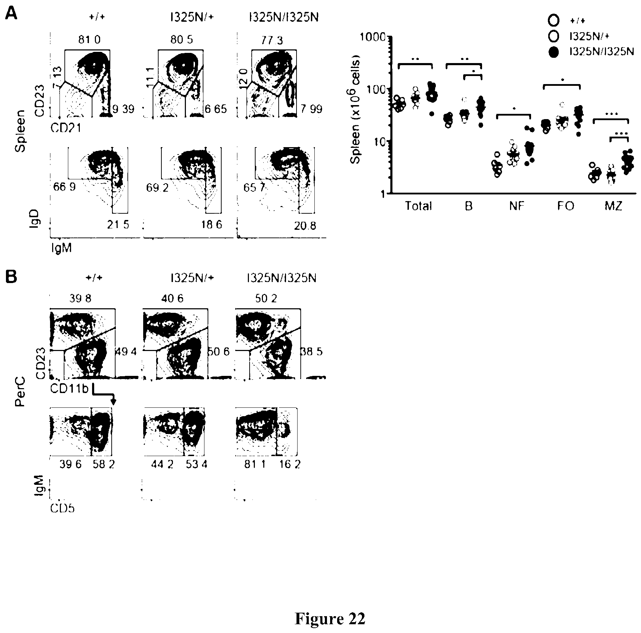

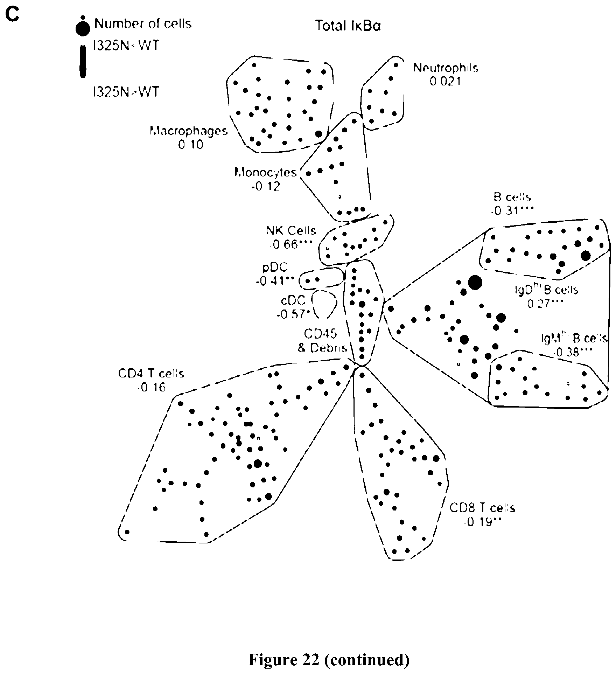

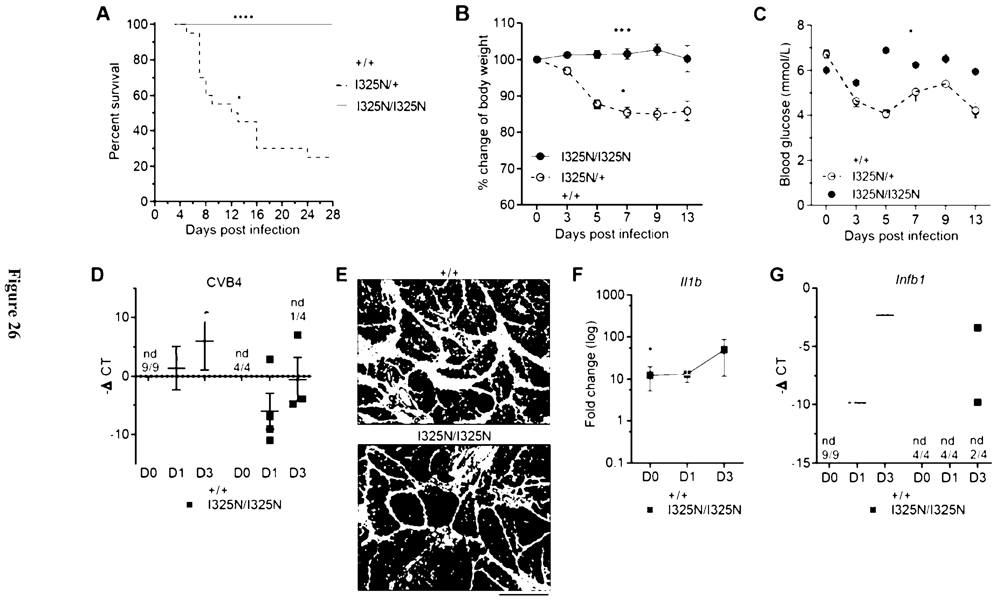

**Figure 22** shows B cell lymphocytosis induced by the Tnfaip3^{I325N} mutation. **(A)** Representative flow cytometry plots from mice of the indicated genotypes showing total frequency of splenic CD23⁺ and CD21⁺ positive cells (top panel). The bottom panel shows cells subsetted into IgD⁺ and IgM⁺ B cells. Note that the number of splenocyte B cell subsets is increased in *Tnfaip3*^{I325N/I325N} mice, shown to the right. **(B)** Representative flow cytometry plots from mice of the indicated genotypes showing the frequency of CD23⁺ and CD21⁺ positive cells in the peritoneal cavity (top panel). The bottom panel shows frequency of IgM⁺ and CD45⁺ B cells. Number of peritoneal cavity B cell subsets in mice of indicated genotypes is shown in Figure 26B. **(C)** CYTOF analysis of intracellular IκBα in immune cells. Unstimulated spleen cells from *Tnfaip3*^{*+*/*+*} or *Tnfaip3*^{I325N/I325N} mice (n=4 per genotype) were individually labeled with mass-barcodes, mixed, permeabilized and stained with mass-labeled antibodies to a panel of cell surface markers and intracellular proteins including IκBα, and analyzed by CYTOF mass spectrometry. Spanning-tree Progression Analysis of Density-normalized Events (SPADE) analysis was used to resolve leukocyte lineages and subsets, and the relative intensity of IκBα in each subset was compared between *Tnfaip3*^{I325N/I325N} and wild-type cell counterparts. Shown by colour and numbers is the mean hyperbolic arcsine (arcsinh) ratio in minor and major leukocyte subsets. Note that IκBα levels are reduced in B cell subsets indicating increased NF-κB activation. Significant differences indicated by Student's t-test comparison of major subsets are marked: *P < 0.05; **P < 0.01; ***P < 0.001.

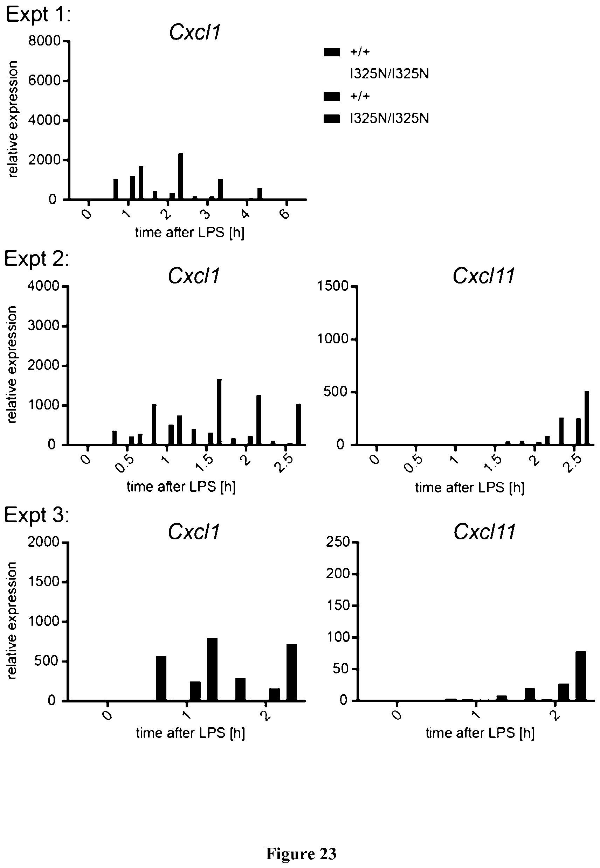

**Figure 23** shows exaggerated cytokine production by bone marrow-derived macrophages with the *Tnfaip3*^{I325N} mutation. Bone marrow macrophages derived from conditionally Hoxb8-immortalized bone marrow progenitor cells were derived from two separate pairs of wild-type and I325N homozygous mice, stimulated with LPS (10ng/ml) for the indicated times, and Cxcl1 or Cxcl11 mRNA determined by qPCR relative to Efla expression. Data are shown from three independent experiments, each with two different mouse donors of each genotype.

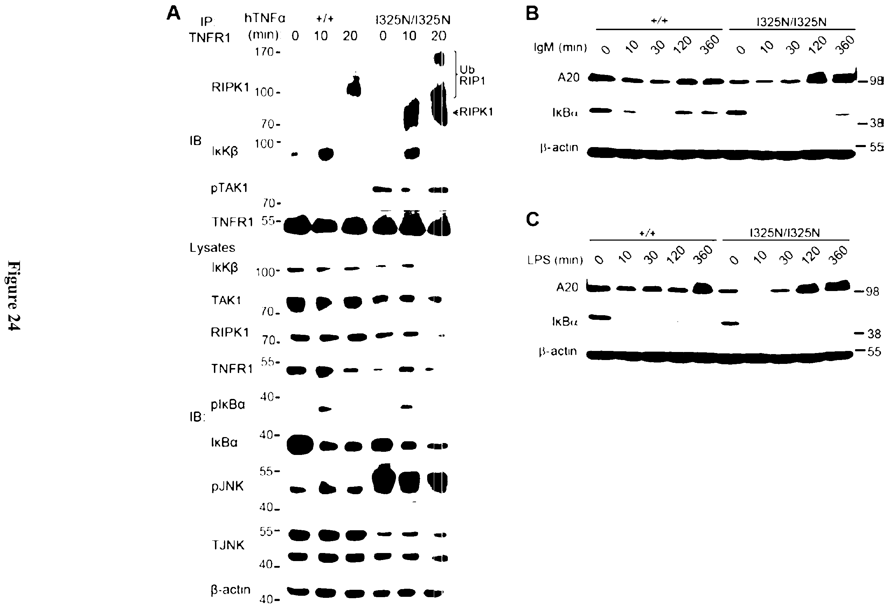

**Figure 24** shows prolonged NF-κB signaling in thymocytes and B cells extracted from I325N variant C57BL/6 mice. **(A)** Representative immunoblot analysis (IB; n=3 independent experiments) of canonical NF-κB components following immunoprecipitation (IP) of TNFR1 from lysates of *Tnfaip3*^{*+*/*+*} or *Tnfaip3*^{I325N/I325N} thymocytes treated with 200 U/ml hTNFα for the indicated times. Following TNFα stimulation RIPK1 was recruited to the TNFR1 complex, with increased high molecular weight polyubiquitinated forms of RIPK1 (UbRIP1) and auto-phosphorylated transforming growth factor β activated kinase-1 (TAK1) in A20^{I325N/I325N} thymocytes (A). Mutant thymocytes exhibited a modest increase in IκBα degradation relative to wild-type, and increased JNK phosphorylation (Lysate blots). A similar pattern of enhanced TAK1 activation and JNK phosphorylation was seen in murine embryonic fibroblasts and bone marrow derived macrophages expressing catalytically dead A20 OTU or ZnF4 mutants (18). **(B, C)** Wild-type (+/+) and I325N homozygous (I325N/I325N) Tnfaip3 splenic B lymphocytes were stimulated for the indicated times with anti-IgM **(B),** or lipopolysaccharide (LPS) **(C)** and lysates analyzed by immunoblotting for A20, IκBα and β-actin (loading control). As observed for mutant thymocytes, B cells exhibited a modest increase in IκBα degradation relative to wild-type.

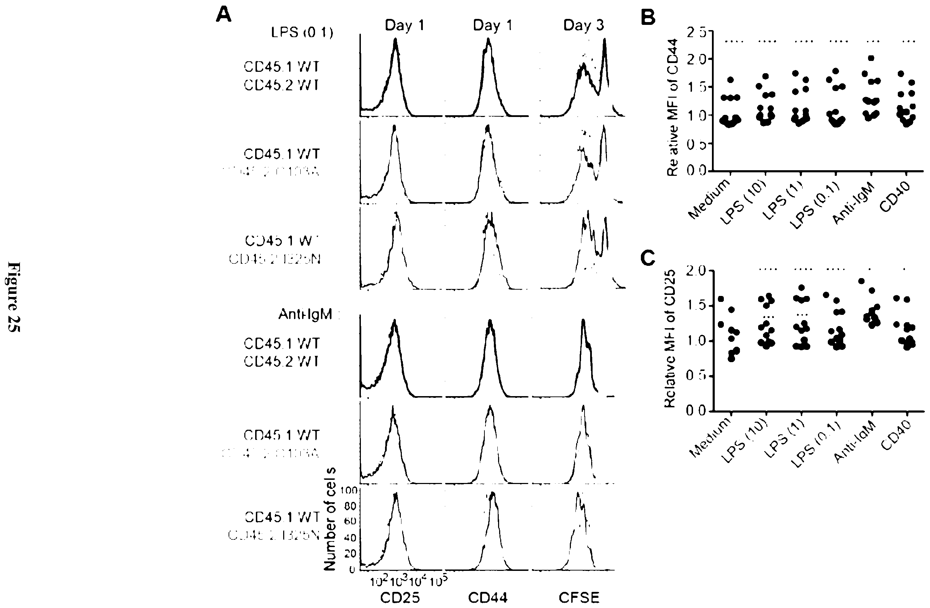

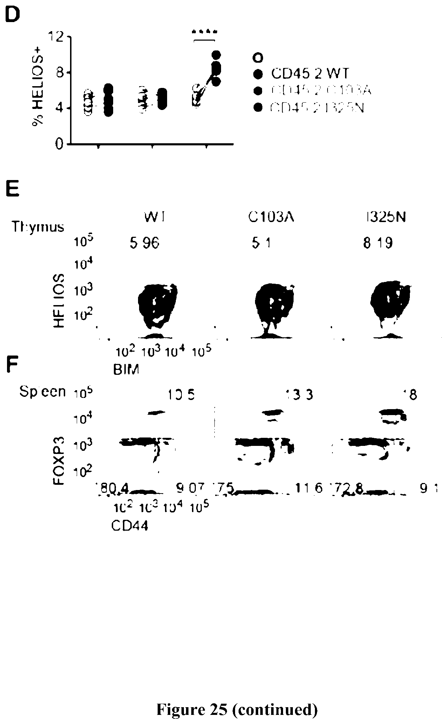

**Figure 25** shows cell autonomous exaggeration of B cell activation and Treg formation by *Tnfaip3* I325N more than C103A mutations. B6.CD45.1⁺ wild-type mice were transplanted with a congenic bone marrow mixture from *Tnfaip3*^{*+*/*+*} CD45.1⁺ donors, to provide wild-type lymphocytes as an internal control, and CD45.2⁺ donors of *Tnfaip3*^{I325N/I325N} *Tnfaip3*^{C103A/C103A} or *Tnfaip3*^{*+*/*+*} genotypes. **(A)** Representative flow cytometric histograms of CD25 and CD44 expression on B cells from chimeric mice cultured for 1 day and CFSE dilution after 3 days with 0.1 ug/ml LPS or anti-IgM. Black or colored histograms, CD45.2⁺ B cells of the indicated Tnfaip3 genotype; grey line unfilled histograms, CD45.1⁺ control B cells in the same stimulated culture; grey filled histograms, B cells in a parallel unstimulated culture. **(B, C)** Data from independent mixed chimeric animals showing relative mean fluorescence intensity (MFI) of CD25 or CD44 on CD45.2⁺ B cells of the indicated genotypes (red, I325N; blue, C103A; black, WT) compared to the co-cultured CD45.1⁺ wild-type B cells. Statistical analysis by ANOVA: *P < 0.05; **P < 0.01; ***P < 0.001; ****P < 0.0001. Figure 26C shows the percentage of CD45.2⁺ cells of the indicated genotypes among viable B cells, relative to starting percentage, in cultures from individual mixed chimera donors stimulated with 0.1 ug/ml LPS. **(D)** Pairwise comparison of percent HELIOS⁺ cells among the indicated CD45.2⁺ and CD45.1⁺ subsets of CD4⁺ CD8⁻ CCR7⁺ CD24⁺ FOXP3⁻ thymocytes from the same chimera. Analysis by paired Student's t-test; ****P < 0.0001. Compared to the C103A mutation, the I325N mutation exaggerated thymic formation of FOXP3⁺ CD4⁺ cells and their Helios⁺ FOXP3⁻ precursors, which depend on TCR signaling through CARD11 to NF-κB. **(E, F)** Representative profiles gated on CD45.2⁺ cells of the indicated *Tnfaip3* genotypes. **(E)** Analysis of CD4⁺ CD8⁻ CCR7⁺ CD24⁺ FOXP3⁻ thymocytes, showing the percentage of immature medullary CD4 T cells induced by strong self-reactivity to express high levels of HELIOS and BIM. **(F)** Percentage of FOXP3⁺ CD44⁺ cells among CD4⁺ splenic T cells.
**Figure 26** shows increased resistance to Coxsackievirus B4 in A20 I325N mice. Infection of C57BL/6 male mice of the indicated *Tnfaip3* genotypes with 20 plaque forming units (PFU) of Coxsackievirus B4 (CVB4) E2 strain by intraperitoneal injection. **(A)** Kaplan-Meier survival curve showing a mean survival time of 6.5 and 12.5 days for *Tnfaip3*^{*+*/*+*} and *Tnfaip3*^{+/I325N} mice, respectively. No *Tnfaip3*^{I325N/I325N} mice succumbed to infection. Significance determined by Log-rank test. **(B)** Percent change in body weight after CVB4 infection and **(C)** Blood glucose levels of mice with indicated *Tnfaip3* genotype, compared to wild-type by area under the curve analysis. **(D)** CVB4 mRNA abundance on indicated days (D0-3) post infection. **(E)** Hematoxylin & eosin stained sections of pancreas from *Tnfaip3*^{*+*/*+*} and *Tnfaip3*^{I325N/I325N} mice at post-infection day 9. Note better preserved pancreatic architecture in *Tnfaip3*^{I325N/I325N} mice. Scale bar = 200 µm. **(F, G)** RTPCR analysis for inflammatory genes Il1b **(F)** or Infb1 **(G)** at post-infection day 0, 1 and 3. Error bars represent SEM and Student t-test used for significance analysis unless otherwise stated, *P < 0.05; ***P < 0.001.

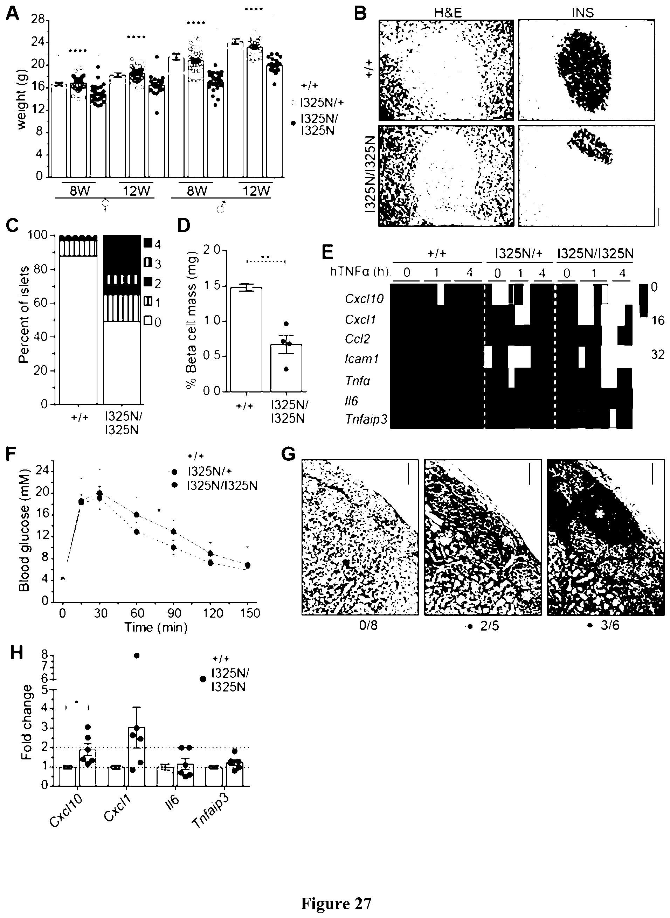

**Figure 27** shows the subclinical inflammatory and metabolic consequences of the I325N variant. **(A)** Body weights of *Tnfaip3*^{*+*/}*⁺, Tnfaip3*^{I325N/+}, *Tnfaip*^{3I325N/I325N} mice of the indicated age and sex. Statistical analysis by unpaired one-way ANOVA: ****P < 0.0001. **(B)** Hematoxylin and eosin (H&E) and insulin (INS) stained pancreas sections (scale = 100 µm) with **(C)** cumulative insulitis scores (4 represents >75% islet mononuclear cell infiltration, and 0 an absence of infiltrating cells) and **(D)** calculated beta cell mass for *Tnfaip3*^{*+*/*+*} and *Tnfaip3*^{I325N/I325N} mice. **(E)** Expression of TNFα-induced genes in islets from *Tnfaip3*^{*+*/}*⁺, Tnfaip3*^{I325N/+}, *Tnfaip3*^{I325N/I325N} mice. Data represents 3 independent islet preparations with 3-4 biological replicates. **(F)** Glucose tolerance test in diabetic wild-type mice transplanted with *Tnfaip3*^{*+*/}*⁺, Tnfaip3*^{I325N/+} or *Tnfaip3*^{I325N/I325N} islets, N>8 mice per group. **(G)** H&E sections of islet grafts at post-operative day 30 (scale = 50 µm) with the fraction of grafts exhibiting immune infiltrate shown below. **(H)** Islet grafts isolated on post-operative day 10 were analysed for indicated mRNAs by RT-qPCR. P values represent Student's t-test unless otherwise stated, *P < 0.05; **P < 0.01; ****P < 0.0001.

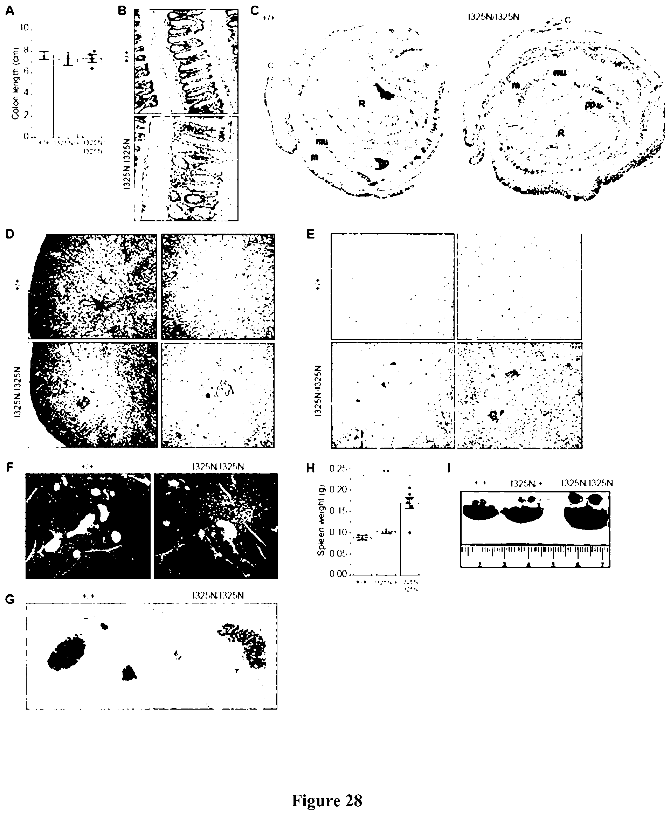

**Figure 28** shows the histological analysis of tissues from A20I325N mice. **(A)** Colon length (cm) and **(B)** Hematoxylin and eosin (H&E) stained sections from *Tnfaip3*^{*+*/*+*} and *Tnfaip3*^{I325N/I325N} mice colon (20 X magnification; scale bar = 200 µm) and **(C)** power-mosaic image of whole colon cross section (R=rectum; C=Caecum; m=muscularis; mu=mucosa; pp=Peyer's patches; scale = 1 mm). **(D)** H&E sections of kidney (4 X left images, scale bar = 500 µm; 10 X right images, scale bar = 200 µm) and **(E)** liver (4 X left images, scale bar = 500 µm; 10 X right images, scale bar = 200 µm). **(F)** H&E stained sections of pancreas (10 X images, scale bar = 200 µm) or, **(G)** insulin stained sections (20 X images, scale bar = 100 µm). (H) Weight of spleens in grams (g). **(I)** Gross appearance of spleen and salivary glands from *Tnfaip3*^{*+*/*+*} and *Tnfaip3*^{I325N/I325N} mice. Images are representative from n=4 *Tnfaip3*^{*+*/*+*} and n=4 *Tnfaip*3^{I325N/I325N} 16 week old mice. Error bars represent SEM, **P < 0.01.

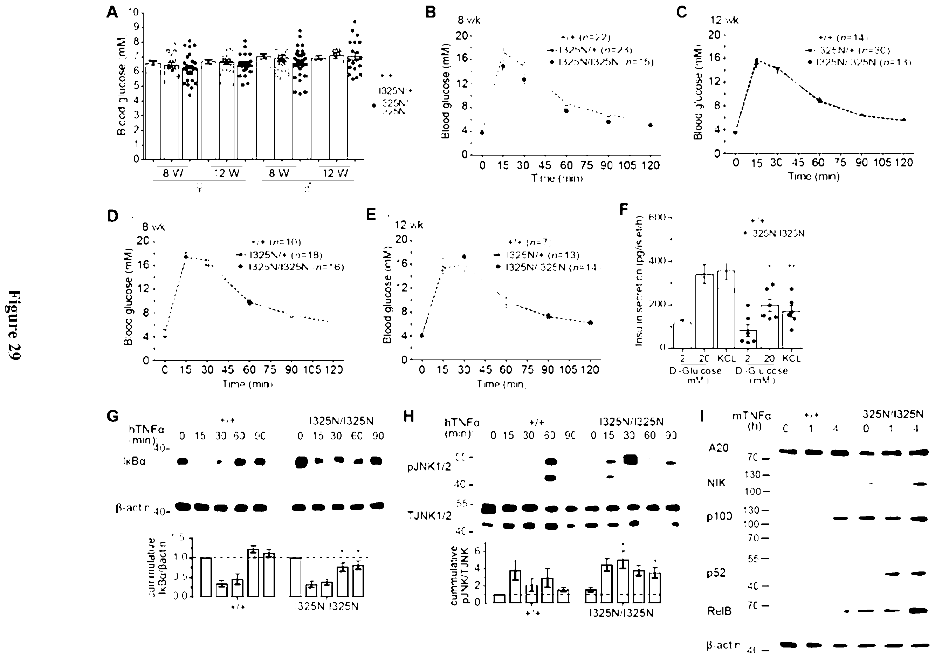

**Figure 29** shows the glucose homeostasis of A20I325N mice and isolated islets. **(A)** Random blood glucose levels of *Tnfaip3*^{*+*/}*⁺, Tnfaip3*^{I325N/+} or *Tnfaip3*^{I325N/I325N} female (♀) or male (♂) mice at 8 or 12 weeks of age. **(B-E)** Blood glucose levels (mM) were monitored following an intraperitoneal injection of glucose (2 g/kg) in 8 **(B, D)** or 12 week-old **(C, E)** mice. **(F)** Pancreatic islets were isolated from individual mice of the indicated A20 genotypes and incubated overnight. Following incubation an in vitro Glucose-Stimulated Insulin Secretion (GSIS) response in conditions of 2 mM, 20 mM D-glucose, or 25 mM KCl was conducted in separate groups of islets. Error bars represent SEM and Student's t-test used for significance between treatments, *P < 0.05; **P < 0.01. (G-H) Pancreatic islets were isolated from individual mice of the indicated A20 genotypes, incubated overnight, and treated with 200 U/ml TNFα for the indicated times. **(G)** Representative immunoblot for IκBα and β-actin (loading control) or **(H)** phosphorylated JNK (pJNK) and total JNK (TJNK, loading control). Cumulative densitometry from 5 independent experiments is shown below (n=5 *Tnfaip3*^{*+*/*+*} and n=6 *Tnfaip3*^{I325N/I325N} biological replicates). **(I)** Immunoblot for non-canonical NF-κB components NIK, p100/p52 and RelB; representative of 2 independent experiments. *Tnfaip3*^{I325N/I325N} islets exhibited increased activation of the non-canonical NF-xB pathway that can alter beta cell transcriptional programs to favor reduced insulin output.

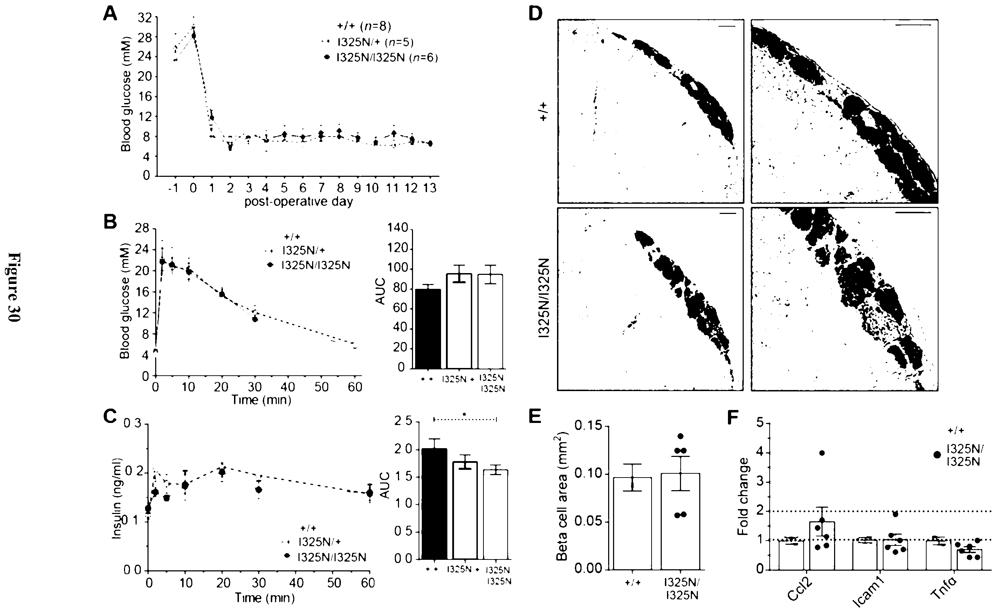

**Figure 30** shows that A20I325N islet grafts exhibit a reduced first-phase insulin secretory response compared to wild-type A20 islet grafts independent to beta cell area. **(A)** Islets isolated from B6 mice of the indicated genotypes were transplanted under the kidney capsule of *Tnfaip3*^{*+*/*+*} B6 recipients that had been rendered diabetic with streptozotocin. Mean and SEM blood glucose on the indicated days relative to islet transplantation is shown. **(B-C)** At post-operative day 14 when euglycemia was established, mice were challenged with an **(B)** intravenous injection of glucose (1 g/kg), and blood glucose monitored over time (min). **(C)** Blood insulin levels (ng/ml) were also measured at the same time points via an enzyme-linked immunosorbent assay from samples in **(B).** AUC = area under the curve. Error bars represent SEM and ANOVA used for significance, *P < 0.05. **(D)** Insulin immunostaining of wild-type (*Tnfaip3*^{*+*/*+*}) or A20 I325N homozygous mutant (*Tnfaip3*^{I325N/I325N}) islet grafts transplanted under the kidney capsule of diabetic C57BL/6 mice for 30 days (*Tnfaip3*^{*+*/}*⁺ > Tnfaip3*^{*+*/}*⁺ n* = 5; *Tnfaip3*^{I325N/I325N} > *Tnfaip3*^{*+*/*+*} n=5). (E) Islet graft beta cell area was determined by insulin-positive area quantification in continuous serial graft sections. *Tnfaip3*^{I325/I325N} grafts exhibited equivalent insulin-positive graft area, confirming that loss of glucose tolerance **(B, C)** was due to a defect in insulin secretion. **(F)** *Tnfaip3*^{*+*/*+*} or *Tnfaip3*^{I325N/I325N} islet grafts from an independent cohort of recipients were isolated on post-operative day 10 and analyzed for the indicated mRNAs by RT-qPCR. The difference in gene expression of indicated islet grafts is shown. Fold change calculated using average wild-type ΔCt value.

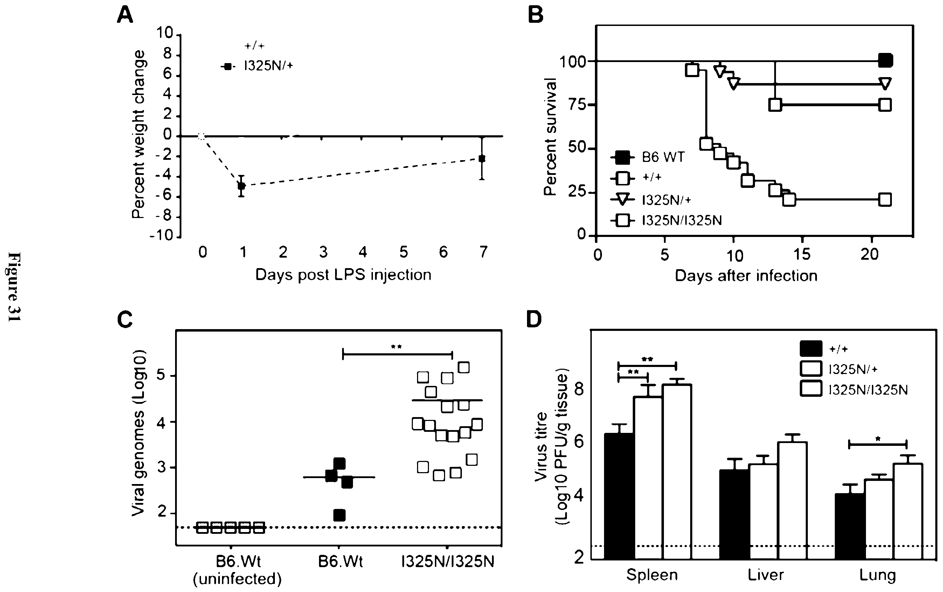

**Figure 31** shows that A20 I325N mice are susceptible to LPS injection and poxvirus infection. **(A)** Daily body weights for *Tnfaip3*^{*+*/*+*} or *Tnfaip3*^{I325N/+} mice administered 50 µg bacterial LPS by intraperitoneal injection. **(B-D)** Ectromelia virus infection of B6 male siblings of the indicated *Tnfaip3* genotypes and wild-type B6 control mice, showing **(B)** Kaplan-Meyer survival plots and **(C)** viral load in blood on day 8; **P < 0.005 by unpaired t test. (D) Virus load in spleen, liver and lung, respectively. Data expressed as mean ± SEM; data was log-transformed and statistical significance determined by 2-way ANOVA; *P < 0.05, **P < 0.01. Dotted lines in **(C, D)** denote limit of detection.

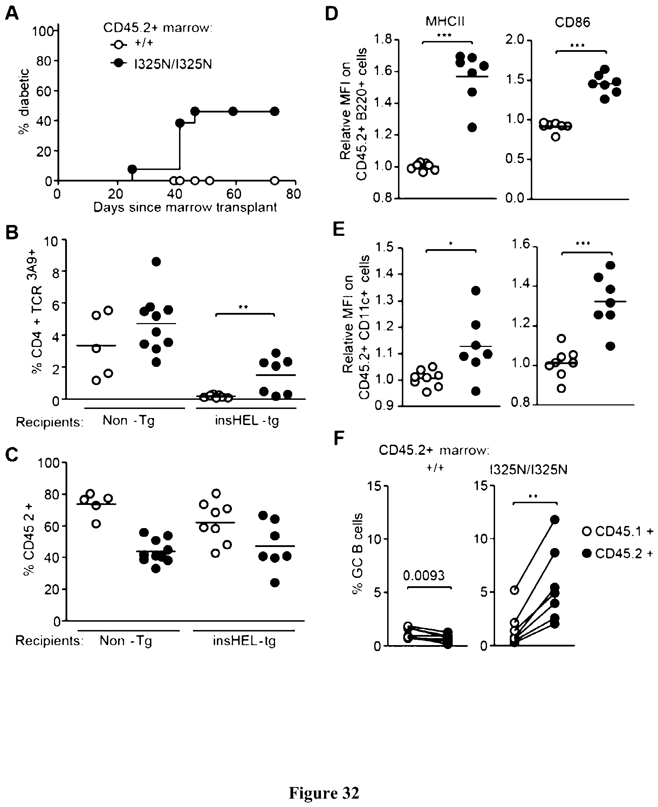

**Figure 32** shows that there is a diminished tolerance to self-antigen caused by mutant A20. InsHEL-transgenic mice or non-transgenic controls, both with wild-type A20 in pancreatic islets, were transplanted with a congenic bone marrow mixture from *Tnfaip3*^{*+*/*+*} CD45.1⁺ TCR^{3A9}-transgenic donors, to serve as an internal control, and CD45.2 TCR^{3A9}-transgenic donors with either *Tnfaip3*^{I325N/I325N} (black circles) or *Tnfaip3*^{*+*/*+*} (grey circles) genotype. **(A)** Incidence of diabetes in insHEL-transgenic recipients as a function of time after transplantation (n=12 chimeras for +/+ and 14 chimeras for I325N/I325N pooled from 2 separate experiments). **(B)** Frequency of TCR^{3A9} positive CD4⁺ T cells among CD45.2⁺ *Tnfaip3*^{*+*/*+*} (grey circles) or *Tnfaip3*^{I325N/I325N} (black circles) lymphocytes in pancreatic lymph nodes. Test recipients expressed the insHEL antigen in pancreatic islets (insHEL-transgenic), while parallel non-transgenic recipients lacked the autoantigen. **(C)** Percentage of CD45.2⁺ *Tnfaip3*^{*+*/*+*} (grey circles) or *Tnfaip3*^{I325N/I325N} (black circles) cells among TCR^{3A9+} CD4⁺ T cells in spleen. **(D-F)** Analysis of insHEL transgenic recipients. Relative mean fluorescence intensity (MFI) of staining for cell surface MHC class II or CD86 on CD45.2⁺ *Tnfaip3*^{*+*/*+*} (grey circles) or *Tnfaip3*^{I325N/I325N} (black circles) B cells **(D)** or CD11c⁺ dendritic cells **(E),** compared to CD45.1⁺ *Tnfaip3*^{*+*/*+*} B cells or dendritic cells in the same sample. **(F)** Frequency of germinal center (GC) B cells among CD45.2⁺ B cells (black circles) compared to the frequency among CD45.1⁺ *Tnfaip3*^{*+*/*+*} B cells in the same animal (linked by lines). Left panel, CD45.2 *Tnfaip3+*/*+* marrow donor; right panel, CD45.2 *Tnfaip3*^{I325N/I325N} marrow donor. In **(B-F),** each symbol, or pair of symbols joined by a line, represents one chimeric mouse, all analyzed in a single experiment. Statistical analysis by 2-tailed Student's t-tests: *P < 0.05; **P < 0.01; ***P < 0.001.

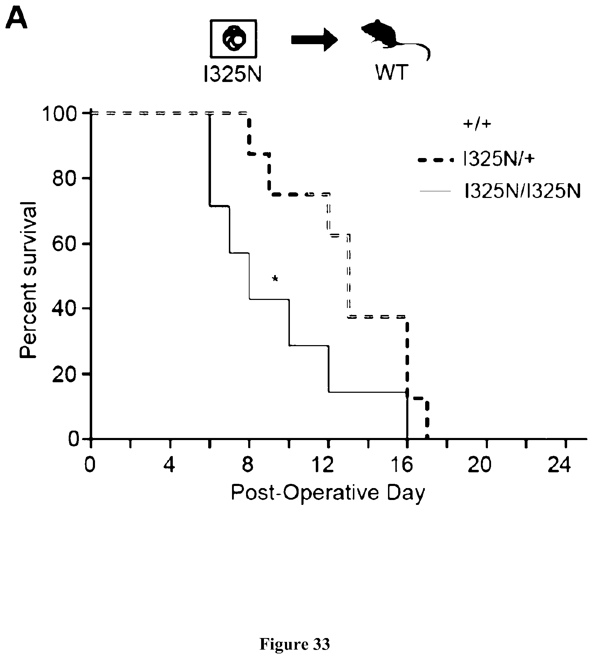

**Figure 33** shows that I325N mice reject transplanted grafts faster.

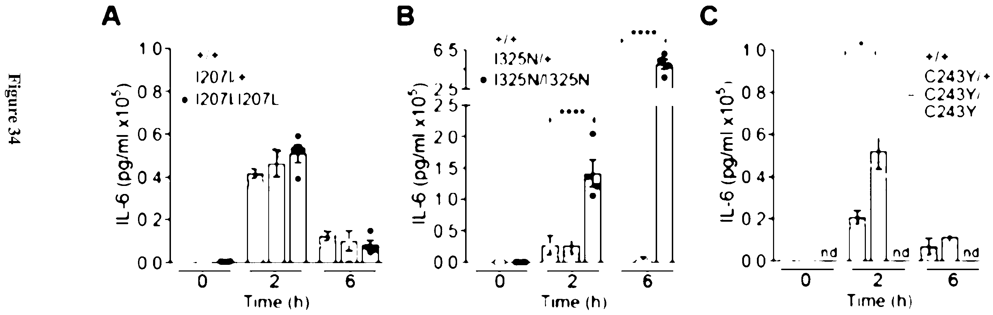

**Figure 34** shows Serum IL-6 concentration in **(A)** *Tnfaip3*^{I207L} mice, **(B)** *Tnfaip3*^{I325N} mice, and **(C)** *Tnfaip3*^{C243Y} mice administered 2.5 mg/kg LPS by intraperitoneal injection at time=0. Statistical analysis by Students *t*-test and one-way ANOVA: **P* < 0.05, ****P* < 0.001, *****P* < 0.0001.

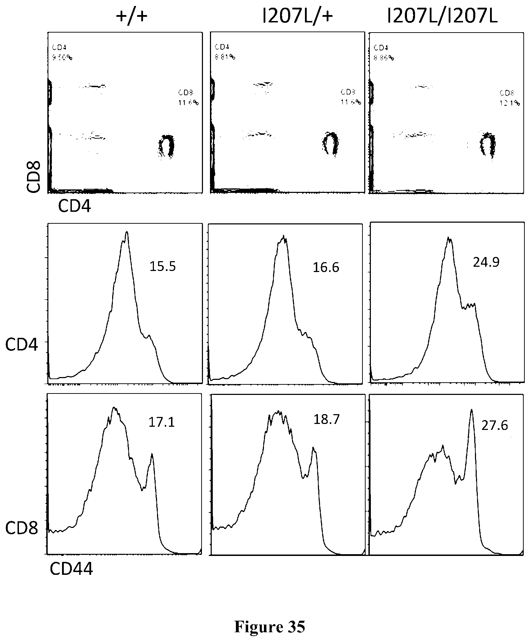

**Figure 35** shows that mice homozygous for I207L exhibit increased frequency of activated (CD44 high) CD4 and CD8 immune cells relative to wildtype mice.

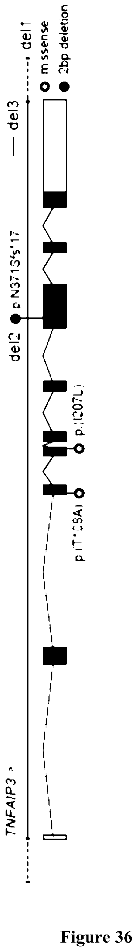

**Figure 36** is a schematic illustrating missense variants *in cis,* p.(T108A) and p.(I207L), within the OTU domain of A20. These missense variants form part of a larger haplotype consisting of multiple coding and non-coding variants (called 'hap').

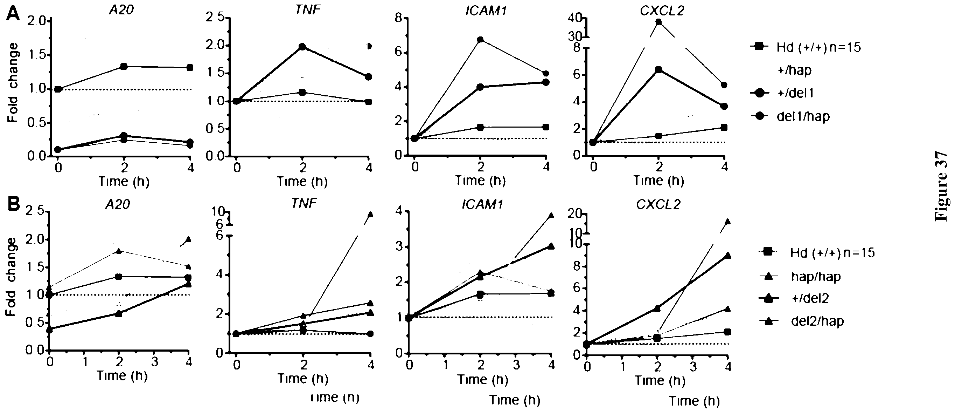

**Figure 37(A)** and **(B)** illustrate that expression of human A20 variants is able to time the human immune response. In particular, this figures show the results of RT-PCR analyses for TNFAIP3, TNF, ICAM1 and CXCL2 gene expression in PBMCs from Trios of subjects. Probands (red circles), +/hap (blue circles), hap/hap (purple circles), +/del (black) and 15 healthy donors (green circles) +/- range (shaded light green).

### Key to the Sequence Listing

| | |
|---|---|
| SEQ ID NO: 1 | Amino acid sequence for reference human A20 protein |
| SEQ ID NO: 2 | cDNA sequence encoding reference human A20 protein |

### Detailed description

### General Definitions

Unless specifically defined otherwise, all technical and scientific terms used herein shall be taken to have the same meaning as commonly understood by one of ordinary skill in the art (e.g. in immunology, molecular biology, oncology and biochemistry).

The present disclosure is performed without undue experimentation using, unless otherwise indicated, conventional techniques of molecular biology, microbiology, virology, recombinant DNA technology, peptide synthesis in solution, solid phase peptide synthesis, and immunology. Such procedures are described, for example, in Sambrook, Fritsch & Maniatis, Molecular Cloning: A Laboratory Manual, Cold Spring Harbor Laboratories, New York, Second Edition (1989), whole of Vols I, II, and III; DNA Cloning: A Practical Approach, Vols. I and II (D. N. Glover, ed., 1985), IRL Press, Oxford, whole of text; Oligonucleotide Synthesis: A Practical Approach (M. J. Gait, ed, 1984) IRL Press, Oxford, whole of text, and particularly the papers therein by Gait, ppl-22; Atkinson *et al,* pp35-81; Sproat *et al,* pp 83-115; and Wu *et al,* pp 135-151; 4. Nucleic Acid Hybridization: A Practical Approach (B. D. Hames & S. J. Higgins, eds., 1985) IRL Press, Oxford, whole of text; Immobilized Cells and Enzymes: A Practical Approach (1986) IRL Press, Oxford, whole of text; Perbal, B., A Practical Guide to Molecular Cloning (1984); Methods In Enzymology (S. Colowick and N. Kaplan, eds., Academic Press, Inc.), whole of series; J.F. Ramalho Ortigao, "The Chemistry of Peptide Synthesis" In: Knowledge database of Access to Virtual Laboratory website (Interactiva, Germany); Sakakibara, D., Teichman, J., Lien, E. Land Fenichel, R.L. (1976). Biochem. Biophys. Res. Commun. 73 336-342; Merrifield, R.B. (1963). J. Am. Chem. Soc. 85, 2149-2154; Barany, G. and Merrifield, R.B. (1979) in The Peptides (Gross, E. and Meienhofer, J. eds.), vol. 2, pp. 1-284, Academic Press, New York. 12. Wiinsch, E., ed. (1974) Synthese von Peptiden in Houben-Weyls Metoden der Organischen Chemie (Miler, E., ed.), vol. 15, 4th edn., Parts 1 and 2, Thieme, Stuttgart; Bodanszky, M. (1984) Principles of Peptide Synthesis, Springer-Verlag, Heidelberg; Bodanszky, M. & Bodanszky, A. (1984) The Practice of Peptide Synthesis, Springer-Verlag, Heidelberg; Bodanszky, M. (1985) Int. J. Peptide Protein Res. 25, 449-474; Handbook of Experimental Immunology, Vols. I-IV (D. M. Weir and C. C. Blackwell, eds., 1986, Blackwell Scientific Publications); and Animal Cell Culture: Practical Approach, Third Edition (John R. W. Masters, ed., 2000), ISBN 0199637970, whole of text.

The skilled artisan will appreciate that the present disclosure is susceptible to variations and modifications other than those specifically described. It is to be understood that the disclosure includes all such variations and modifications. The disclosure also includes all of the steps, features, compositions and compounds referred to or indicated in this specification, individually or collectively, and any and all combinations of any two or more of said steps or features.

The present disclosure is not to be limited in scope by the specific embodiments described herein, which are intended for the purpose of exemplification only. Functionally equivalent products, compositions and methods are clearly within the scope of the disclosure, as described herein.

Each feature of any particular aspect or embodiment or embodiment of the present disclosure may be applied *mutatis mutandis* to any other aspect or embodiment or embodiment of the present disclosure.

Throughout this specification, unless specifically stated otherwise or the context requires otherwise, reference to a single step, composition of matter, group of steps or group of compositions of matter shall be taken to encompass one and a plurality (i.e. one or more) of those steps, compositions of matter, groups of steps or group of compositions of matter.

As used herein, the singular forms of "a", "and" and "the" include plural forms of these words, unless the context clearly dictates otherwise. For example, a reference to "a bacterium" includes a plurality of such bacteria, and a reference to "an allergen" is a reference to one or more allergens.

The term "and/or", *e.g.,* "X and/or Y" shall be understood to mean either "X and Y" or "X or Y" and shall be taken to provide explicit support for both meanings or for either meaning.

Throughout this specification, the word "comprise" or variations such as "comprises" or "comprising" will be understood to imply the inclusion of a stated element, integer or step, or group of elements, integers or steps, but not the exclusion of any other element, integer or step, or group of elements, integers or steps.

### Specific Definitions

As used herein, the term "stratify", "stratifying" or similar in the context of a method of the disclosure shall be understood to mean classification or division of subjects tested into groups or categories indicative of the likely strength of that subject's immune response. For example, a subject on which the method of the disclosure is performed may be classified or identified as an individual which is likely to elicit a strong immune response or a weak immune response *e.g.*, to an incoming treatment or antigen, relative to an immune response elicited by an individual having a reference A20 gene sequence.

As used herein, the term "subject" refers to any animal, for example, a mammalian animal, including, but not limited to humans, non-human primates, livestock (e.g. sheep, horses, cattle, pigs, donkeys), companion animals (e.g. pets such as dogs and cats), laboratory test animals (e.g. mice, rabbits, rats, guinea pigs), performance animals (e.g. racehorses, camels, greyhounds) or captive wild animals. In a preferred example, the "subject" is a human. Typically, the terms "subject" and "patient" are used interchangeably, particularly in reference to a human subject.

As used herein, the term "immune response" will be understood to refer to an alteration of an organism's immune system upon exposure to, for example, an antigen, allergen, auto-antibody, pathogen, treatment agent or a graft. The term "immune response" encompasses, but is not limited to, an immune response involving antibody production (*e.g*., humoral immunity) and/or the induction of cell-mediated immunity (*e.g*., cellular immunity including helper T cell and/or cytotoxic T cell responses). The immune response may involve an activation of a subject's immune system or, alternatively, a suppression of a subject immune system.

As used herein, the term "single nucleotide variant" or "SNV" shall be understood to mean a nucleic acid sequence (DNA or RNA) which differs at a specific nucleotide position relative to a corresponding reference sequence. An "A20 SNV" as used herein shall be understood to mean a *TNFAIP3* gene sequence encoding an A20 protein that comprises a non-synonymous SNV relative to a *TNFAIP3* gene sequence encoding a wildtype reference A20 protein. As such, an A20 SNV or "A20 variant" of the present disclosure shall differ by at least one amino acid relative to the wildtype reference A20 protein sequence. An exemplary A20 reference amino acid sequence is set forth in SEQ ID NO: 1 and/or is encoded by the cDNA sequence set forth in SEQ ID NO: 2. Exemplary A20 SNVs are set forth in Tables 1-3 herein.

As used herein, the term "A20 allelic variant", "A20 variant" or similar shall be understood to mean a variant of the A20 protein encoded by an A20 SNV sequence described herein. As described herein, an "A20 variant" of the present disclosure shall differ by at least one amino acid relative to the wildtype reference A20 protein sequence (*i.e*., as a result of the non-synonymous SNV). The "A20 variant" may be a nucleic acid comprising a SNV sequence which encodes an A20 variant protein, or may be provided as the A20 variant protein itself.

As used herein, the term "wild-type" refers to the most common polynucleotide sequence or allele for a certain gene in a population. Generally, the wild-type allele will be obtained from normal cells. Depending on the particular purpose or desire of the practitioner, the reference sequence is generally a wild-type sequence. Accordingly, a "wildtype A20 allele" shall be understood to mean a common, preferably the most common, allele of A20 in the population *e.g*., human population.

As used herein, an "autologous cell" shall be understood to related to a cell in which the donor and the recipient are the same. In contrast, an "allogeneic cell" shall be understood to mean a cell in which the donor and recipient are genetically non-identical individuals from the same species. Two or more cells are said to be allogeneic to one another when the genes at one or more loci are not identical. In some examples, allogeneic cells from individuals of the same species may be sufficiently unlike genetically to interact antigenically. In either context *i.e.,* autologous or allogeneic, the cell may be any cell types including, but not limited to, a T-cell or a cell within transplant or graft tissue.

"T-cells" belong to a group of white blood cells known as lymphocytes, and play a central role in cell-mediated immunity and, to a lesser degree the adaptive immune response. Generally, T-cells are distinguished from other lymphocytes (*e.g*., B cells and natural killer cells) by the presence of T-cell receptors (TCRs). T-cells have diverse roles, which are accomplished by differentiation of distinct populations of T-cells, recognizable by discrete gene expression profiles.

The terms "CAR-T cell", "CART cell" or similar shall be understood to mean a T-cell comprising a chimeric antigen receptor (CAR).

A "chimeric Antigen Receptor" or alternatively a "CAR", refers to a recombinant polypeptide construct comprising at least an extracellular antigen binding domain, a transmembrane domain and a cytoplasmic signaling domain (also referred to as an intracellular signaling domain) comprising a functional signalling domain derived from a stimulatory molecule and/or costimulatory molecule. As used herein, the term "antigen-binding domain" shall be understood to mean a protein or region thereof that recognizes and binds to an antigen. An exemplary antigen binding domain is one which binds to a tumor antigen or a viral antigen expressed on a cell surface.

As used herein, the terms "treating", "treat" or "treatment" and variations thereof, refer to clinical intervention designed to alter the natural course of the individual or cell being treated during the course of clinical pathology. Desirable effects of treatment include decreasing the rate of disease progression, ameliorating or palliating the disease state, and remission or improved prognosis.

As used herein, the term "cancer" refers to a disease characterized by the rapid and uncontrolled growth of aberrant cells. Cancer cells can spread locally or through the bloodstream and lymphatic system to other parts of the body. Examples of various cancers include but are not limited to, breast cancer, prostate cancer, ovarian cancer, cervical cancer, skin cancer, pancreatic cancer, colorectal cancer, renal cancer, liver cancer, brain cancer, lymphoma, blood cancers e.g., leukemia, lung cancer and the like. Further exemplary cancers are described herein. The term "cancer" includes all types of cancerous growths or oncogenic processes, metastatic tissues or malignantly transformed cells, tissues, or organs, irrespective of histopathologic type or stage of invasiveness. The terms "tumor" and "cancer" are used interchangeably herein. Both terms encompass solid and liquid, *e.g*., diffuse or circulating, tumors. As used herein, the term "cancer" or "tumor" includes premalignant, as well as malignant cancers and tumors.

A "therapeutically effective amount" is at least the minimum concentration or amount required to effect a measurable improvement in the disease or condition to be treated or prevented *e.g*., cancer, infection by a pathogen, autoimmune disease or graft rejection. The skilled artisan will be aware that such an amount will vary depending on, for example, the disease to be treated (*e.g.,* in the case of cancer, the specific type of cancer and/or stage thereof, or in the case of infection, the type of pathogen, or in the case of autoimmune disease, the type of autoimmune disease etc) and/or the particular subject and/or the type or severity of a condition being treated. Accordingly, this term is not to be construed to limit the disclosure to a specific quantity or amount of A20 variant or cell population comprising same.

### Agents and methods for modulating immune response

The present disclosure provides a method of modifying the strength of an immune response in a subject by administering to, or expressing in, the subject an A20 variant which will increase or decrease the strength of the subject's immune response relative to the subject's immune response in the absence of the A20 variant being administered or expressed.

A20 is encoded by the *TNFAIP3* gene. It is acts a as a negative regulator of NF-κB signaling: an evolutionarily ancient and central pathway for activating innate and adaptive immune responses. A20 has multiple domains with inhibitory activity against NF-κB, primarily preventing activation of the central IκB kinase (IKK) by upstream proteins RIPK1, TRAF6 and NEMO. The A20 ovarian tumor (OTU) domain is a deubiquitin (DUB) protease that cleaves activating K63-linked ubiquitin chains from RIPKI, TRAF6 or NEMO. The A20 zinc finger 7 domain (ZnF7) binds linear polyubiquitin to suppress IKK activation, whereas ZnF4 promotes ligation of K48-linked ubiquitin chains to RIPK1, triggering RIPK1 proteolysis. A20 feedback inhibition is induced at two levels: NF-κB proteins directly induce *TNFAIP3* mRNA, and the inhibitory activities of A20 protein are enhanced by IκKβ-induced serine phosphorylation near the ZnF domains, notably S381.

Surprisingly, the inventors have shown that some non-synonymous SNV variants of the A20 protein decrease a subject's immune response relative to if the subject had a reference A20 protein sequence (*e.g.,* a wild-type human A20 protein) and other non-synonymous SNV variants of the A20 protein increase a subject's immune response relative to if the subject had the reference A20 protein sequence (*e.g*., a wild-type human A20 protein). Accordingly, by controlling which A20 variant is expressed in or administered to a subject, the strength of the subject's immune response may be modified *i.e.,* tuned up by expressing in or administering to the subject an A20 variant shown to increase the immune response relative to a wild-type or reference A20 protein, or tuned down by expressing in or administering to the subject an A20 variant shown to decrease the immune response relative to a wild-type or reference A20 protein.

In one example, the method of the disclosure comprises administering to, or expressing in, the subject a SNV variant of the A20 protein which increases or decreases NF-κB and/or c-Jun amino-terminal kinase (JNK) signalling in the subject relative to if the subject was administered or expressed the reference A20 protein sequence set forth in SEQ ID NO: 1. For example, the method may comprise administering to, or expressing in, the subject an A20 variant set forth in Table 1. Mutations in Table 1 are described relative to the reference A20 protein sequence set forth in SEQ ID NO: 1, which is the wildtype amino acid sequence for human A20 protein. Administering to, or expressing in, the subject an A20 variant set forth in Table 1 increases or decreases the subject's immune response relative to if the subject was administered or expressed the reference A20 protein sequence. In accordance with this example, an increase or decrease in the subject immune response comprises a corresponding increase or decrease in NF-κB and/or JNK signalling relative to if the subject was administered or expressed the reference A20 protein sequence.

**Table 1 - A20 variants associated with modified immune response**

| A20 Variant ID | Amino acid change | bp change | rs number* | Genome Cordinates | Functional Impact** | PolyPhen2 Prediction*** |
|---|---|---|---|---|---|---|
| 1 | N102S | A/G | rs146534657 | 6:138195991 | LoF | damaging |
| 2 | 1207L † | A/C | rs141807543 | 6:138196957 | LoF | benign |
| 3 | C243Y | G/A | COSM5714695^ | 6:137876089 | LoF | damaging |
| 4 | S254N ¥ | G/A | rs61756235 | 6: 138238952 | LoF | benign |
| 5 | K301N | G/T | n.a. | 6:137877173 | LoF | damaging |
| 6 | I325N *f* | n.a. | n.a. | 6:138198380 | LoF | damaging |
| 7 | R706Q ¥¥ | G/A | rs3734553 | 6-138202200 | LoF | benign |
| 8 | R763Q | G/A | rs369621216 | 6:138202371 | LoF | damaging |
| 9 | A766T | G/A | rs5029957 | 6:138202379 | LoF | damaging |
| 10 | A766P¥ | G/C | rs5029957 | 6: 138244072 | LoF | damaging |
| 11 | V115I | G/A | rs150717698 | 6:138196029 | GoF | benign |
| 12 | R162Q | G/A | rs144788826 | 6:138196171 | GoF | damaging |
| 13 | S184N | G/A | rs147932545 | 6:138196889 | GoF | benign |

| | | | | | | |
|---|---|---|---|---|---|---|
| * rs numbers, coordinates and frequencies have been collected from gnomAD, which uses GRCh37/h19 ** Functional Impact = tested in experimental studies. GoF, Gain of function or "cold" variant; LoF Loss of Function or "hot" variant; none, no functional change *** PolyPhen2.0 gene prediction algorithm; SNV marked as 'damaging' achieved a PolyPhen2.0 score of ≥0.8 ^COSMID numbers have been collected from ensembl.org, which uses genome build GRCh38 † SNV change occurs in both Denisovan hominins and anatomically modern humans ¥ Neandertal SNV not identified in anatomically modern humans ¥¥ SNV change occurs in both Neanderthal hominins and anatomically modern humans *f* A20 variant identified through *in vivo* ENU-mutagenesis mouse screen | | | | | | |

In one example, the method of the disclosure comprises increasing the subject immune response by administering to, or expressing in, the subject an A20 variant set forth in Table 2. Administering to, or expressing in, the subject an A20 variant set forth in Table 2 increases the subject's immune response relative to if the subject was administered or expressed the reference A20 protein. Similarly, administering to, or expressing in, the subject an A20 variant set forth in Table 2 increases the subject's immune response relative to if the subject was administered or expressed any one of the A20 variants set forth in Table 3. In accordance with this example, an increase in the subject's immune response comprises an increase in NF-κB and/or JNK signalling relative to if the subject expressed the reference A20 protein or any one of the A20 variants set forth in Table 3.

**Table 2 - A20 variants associated with increased/activated immune response**

| A20 Variant ID | Amino acid change | bp change | rs number* | Genome coordinates | Functional Impact** | PolyPhen2 Prediction*** |
|---|---|---|---|---|---|---|
| 1 | N102S | A/G | rs146534657 | 6:138195991 | LoF | damaging |
| 2 | I207L † | A/C | rs141807543 | 6:138196957 | LoF | benign |
| 3 | C243Y | G/A | COSM5714695^ | 6:137876089 | LoF | damaging |
| 4 | S254N ¥ | G/A | rs61756235 | 6: 138238952 | LoF | benign |
| 5 | K301N | G/T | n.a. | 6:137877173 | LoF | damaging |
| 6 | I325N *f* | n.a. | n.a. | 6:138198380 | LoF | damaging |
| 7 | R706Q ¥¥ | G/A | rs3734553 | 6-138202200 | LoF | benign |
| 8 | R763Q | G/A | rs369621216 | 6:138202371 | LoF | damaging |
| 9 | A766T | G/A | rs5029957 | 6:138202379 | LoF | damaging |
| 10 | A766P¥ | G/C | rs5029957 | 6: 138244072 | LoF | damaging |

| | | | | | | |
|---|---|---|---|---|---|---|
| * rs numbers, coordinates and frequencies have been collected from gnomAD, which uses GRCh37/h19 ** Functional Impact = tested in experimental studies. LoF Loss of Function or "hot" variant; none, no functional change *** PolyPhen2.0 gene prediction algorithm; SNV marked as 'damaging' achieved a PolyPhen2.0 score of ≥0.8 ^COSMID numbers have been collected from ensembl.org, which uses genome build GRCh38 † SNV change occurs in both Denisovan hominins and anatomically modern humans ¥ Neandertal SNV not identified in anatomically modern humans ¥¥ SNV change occurs in both Neanderthal hominins and anatomically modern humans *f* A20 variant identified through *in vivo* ENU-mutagenesis mouse screen | | | | | | |

In one example, the method of the disclosure comprises increasing the subject immune response by administering to, or expressing in, the subject A20 variant 1. In one example, the method of the disclosure comprises increasing the subject immune response by administering to, or expressing in, the subject A20 variant 2. In one example, the method of the disclosure comprises increasing the subject immune response by administering to, or expressing in, the subject A20 variant 3. In one example, the method of the disclosure comprises increasing the subject immune response by administering to, or expressing in, the subject A20 variant 4. In one example, the method of the disclosure comprises increasing the subject immune response by administering to, or expressing in, the subject A20 variant 5. In one example, the method of the disclosure comprises increasing the subject immune response by administering to, or expressing in, the subject A20 variant 6. In one example, the method of the disclosure comprises increasing the subject immune response by administering to, or expressing in, the subject A20 variant 7. In one example, the method of the disclosure comprises increasing the subject immune response by administering to, or expressing in, the subject A20 variant 8. In one example, the method of the disclosure comprises increasing the subject immune response by administering to, or expressing in, the subject A20 variant 9. In one example, the method of the disclosure comprises increasing the subject immune response by administering to, or expressing in, the subject A20 variant 10.

In another example, the method of the disclosure comprises decreasing the subject immune response by administering to, or expressing in, the subject an A20 variant set forth in Table 3. Administering to, or expressing in, the subject an A20 variant set forth in Table 3 decreases the subject's immune response relative to if the subject was administered or expressed the reference A20 protein. Similarly, administering to, or expressing in, the subject an A20 variant set forth in Table 3 decreases the subject's immune response relative to if the subject was administered or expressed any one of the A20 variants set forth in Table 2. In accordance with this example, a decrease in the subject's immune response comprises an decrease in NF-κB and/or JNK signalling relative to if the subject expressed the reference A20 protein or any one of the A20 variants set forth in Table 2.

**Table 3- A20 variants associated with decreased/repressed immune response**

| A20 Variant ID | Amino acid change | bp change | rs number* | Genome coordinates | Functional Impact** | PolyPhen2 Prediction*** |
|---|---|---|---|---|---|---|
| 11 | V115I | G/A | rs150717698 | 6:138196029 | GoF | benign |
| 12 | R162Q | G/A | rs144788826 | 6:138196171 | GoF | damaging |
| 13 | S184N | G/A | rs147932545 | 6:138196889 | GoF | benign |

| | | | | | | |
|---|---|---|---|---|---|---|
| * rs numbers, coordinates and frequencies have been collected from gnomAD, which uses GRCh37/h19 ** Functional Impact = tested in experimental studies. LoF Loss of Function or "hot" variant; none, no functional change *** PolyPhen2.0 gene prediction algorithm; SNV marked as 'damaging' achieved a PolyPhen2.0 score of ≥0.8 | | | | | | |

In one example, the method of the disclosure comprises increasing the subject immune response by administering to, or expressing in, the subject A20 variant 11. In one example, the method of the disclosure comprises increasing the subject immune response by administering to, or expressing in, the subject A20 variant 12. In one example, the method of the disclosure comprises increasing the subject immune response by administering to, or expressing in, the subject A20 variant 13.

### A20 protein, fragments and variants

In one example, the A20 SNV variant for modifying the strength of an immune response in a subject as described herein is provided in the form of an A20 variant protein or a functional fragment or variant thereof which retains its NF-κB and/or JNK signalling function. In one example, the A20 variant protein is any variant protein which increases or decreases NF-κB and/or JNK signalling in the subject relative to if the subject was administered or expressed the reference A20 protein sequence set forth in SEQ ID NO: 1. Exemplary A20 variant proteins for modifying an immune response are set forth in Table 1. As described hereinabove, the variants presented in Table 1 are described relative to the reference A20 protein sequence set forth in SEQ ID NO: 1, which is the wildtype amino acid sequence for human A20 protein. In accordance with an example in which the A20 variant protein increases NF-κB and/or JNK signalling in the subject and thereby increases the immune response in a subject, the A20 variant may be an A20 variant protein set forth in Table 2 (*i.e.,* a variant selected from A20 variants 1-10). In accordance with an example in which the A20 variant protein decreases NF-κB and/or JNK signalling in the subject and thereby decreases the immune response in a subject, the A20 variant may be an A20 variant protein set forth in Table 3 (*i.e.,* a variant selected from A20 variants 11-13). Alternative splice variants of the A20 variant proteins described herein which retain A20 activity *i.e.,* NF-κB and/or JNK signalling activity, are also contemplated for use in the methods of the disclosure.

In another example, an A20 variant protein of the disclosure comprises a sequence at least about 75% or 80% or 85% or 90% or 95% or 97% or 98% or 99% identical to the sequence set forth in SEQ ID NO: 1, provided that the A20 variant protein comprises the mutation of the corresponding variant set forth in Tables 1-3 and exhibits modified NF-κB and/or JNK signalling activity relative the sequence set forth in SEQ ID NO: 1. For example, an A20 variant protein of the disclosure which increases an immune response in a subject may comprise a sequence at least about 75% or 80% or 85% or 90% or 95% or 97% or 98% or 99% identical to the sequence set forth in SEQ ID NO: 1, provided that the A20 variant comprises one of the mutations set forth in Table 2 and exhibits increased NF-κB and/or JNK signalling activity relative the sequence set forth in SEQ ID NO: 1. For example, an A20 variant protein of the disclosure which decreases an immune response in a subject may comprise a sequence at least about 75% or 80% or 85% or 90% or 95% or 97% or 98% or 99% identical to the sequence set forth in SEQ ID NO: 1, provided that the A20 variant comprises one of the mutations set forth in Table 3 and exhibits decreased NF-κB and/or JNK signalling activity relative the sequence set forth in SEQ ID NO: 1.

Functional fragments, analogs and/or derivatives of A20 variant protein set forth in Tables 1-3 are also contemplated for use in the methods of the present disclosure, provided that the fragments, analogs and/or derivatives retain the NF-κB and/or JNK signalling activity of the corresponding A20 variant protein set forth in Tables 1-3.

For example, the present disclosure contemplates the use of A20 variants set forth in Tables 1-3 comprising conservative substitutions or mutations in a small number of residues of the protein, provided that the variant retains the NF-κB and/or JNK signalling activity of the corresponding A20 variant set forth in Tables 1-3. A "conservative amino acid substitution" is one in which the amino acid residue is replaced with an amino acid residue having a similar side chain and/or hydropathicity and/or hydrophilicity.

Families of amino acid residues having similar side chains have been defined in the art, including basic side chains (*e.g.,* lysine, arginine, histidine), acidic side chains (*e.g.,* aspartic acid, glutamic acid), uncharged polar side chains (*e.g*., glycine, asparagine, glutamine, serine, threonine, tyrosine, cysteine), nonpolar side chains (*e.g*., alanine, valine, leucine, isoleucine, proline, phenylalanine, methionine, tryptophan), β-branched side chains (*e.g*., threonine, valine, isoleucine) and aromatic side chains (*e.g*., tyrosine, phenylalanine, tryptophan, histidine). Hydropathic indices are described, for example in Kyte and Doolittle J. Mol. Biol., 157: 105-132, 1982 and hydrophylic indices are described in, *e.g.,* US4554101.

The present disclosure also contemplates the use of variants of the A20 proteins set forth in Tables 1-3 comprising non-conservative substitutions or mutations in a small number of residues of the protein, provided that the A20 variant proteins retain the NF-κB and/or JNK signalling activity of the corresponding A20 variant set forth in Tables 1-3. For example, of particular interest are substitutions of charged amino acids with another charged amino acid and with neutral or positively charged amino acids. In some examples, the A20 variant protein of the disclosure comprises 10 or fewer, *e.g.,* 9 or 8 or 7 or 6 or 5 or 4 or 3 or 2 or 1 non-conservative amino acid substitutions.

The present disclosure also contemplates the use of variants of the A20 protein set forth in Tables 1-3 comprising one or more non-naturally occurring amino acids or amino acid analogues. For example, an A20 variant protein may comprise one or more naturally occurring non-genetically encoded L-amino acids, synthetic L-amino acids or D-enantiomers of an amino acid. For example, the A20 variant protein may comprise only D-amino acids. Exemplary non-coded amino acids include: hydroxyproline, β-alanine, 2,3-diaminopropionic acid, α-aminoisobutyric acid, N-methylglycine (sarcosine), ornithine, citrulline, t-butylalanine, t-butylglycine, N-methylisoleucine, phenylglycine, cyclohexylalanine, norleucine, naphthylalanine, pyridylananine 3-benzothienyl alanine 4-chlorophenylalanine, 2-fluorophenylalanine, 3-fluorophenylalanine, 4-fluorophenylalanine, penicillamine, 1,2,3,4-tetrahydro-tic isoquinoline-3-carboxylic acid β-2-thienylalanine, methionine sulfoxide, homoarginine, N-acetyl lysine, 2,4-diamino butyric acid, ρ-aminophenylalanine , N-methylvaline, homocysteine, homoserine, ε-amino hexanoic acid, δ-amino valeric acid, 2,3-diaminobutyric acid.

The A20 variant protein or fragment, analog or derivative thereof may be provided as a conjugate. For example, the A20 variant protein, fragment, analog or derivative may be conjugated to a nonproteinaceous moiety known in the art for improving one or more pharmacokinetic properties of protein-based agents. Preferably, the moiety suitable for derivatization of the protein, fragment, analog or derivative is a physiologically acceptable polymer, preferably a water soluble polymer. Such polymers are useful for increasing stability and/or reducing clearance (*e.g*., by the target tissue or organ). Non-limiting examples of water soluble polymers include, but are not limited to, polyethylene glycol (PEG), polyvinyl alcohol (PVA), or propropylene glycol (PPG).

Alternatively, or in addition, the A20 variant protein, fragment, analog or derivative described herein may be conjugated or linked to another protein, including another protein of the disclosure or an analog or derivative derived therefrom *e.g*., described herein. Other proteins are not excluded. Additional proteins will be apparent to the skilled artisan and include, for example, an immunomodulator or a half-life extending protein or a peptide or other protein that binds to serum albumin amongst others. When linked to another protein, the A20 variant protein, fragment, analog or derivative described herein may be a fusion protein or chimeric protein. Methods for producing fusion proteins and chimeric proteins are known in the art.

Conjugates of the A20 variant protein, fragment, analog or derivative and additional moieties can be made using a variety of bifunctional protein-coupling agents such as, but not limited to, 4-(4'acetylphenoxy)butanoic acid (AcBut), 3-acetylphenyl acidic acid (AcPac), 4-mercapto-4-methyl-pentanoic acid (Amide), N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene), and derivatives thereof.

The A20 variant protein, fragment, analog or derivative can be produced by recombinant means or synthetic means.

Recombinant means generally comprise operably linking a nucleic acid encoding the A20 variant protein to be expressed to a promoter to thereby form an expression construct, which can be an expression vector (*e.g.,* a plasmid or phagemid). The present disclosure contemplates such an expression construct. The nucleic acid can be produced and/or isolated and cloned into an appropriate construct using methods known in the art and/or described in Ausubel et al (In: Current Protocols in Molecular Biology. Wiley Interscience, ISBN 047 150338, 1987) and/or (Sambrook et al (In: Molecular Cloning: Molecular Cloning: A Laboratory Manual, Cold Spring Harbor Laboratories, New York, Third Edition 2001).

Suitable promoters and/or expression vectors will be apparent to the skilled person based on the cell/expression system to be used. For example, typical promoters suitable for expression in a mammalian cell include, for example a promoter selected from the group consisting of, retroviral LTR elements, the SV40 early promoter, the SV40 late promoter, the CMV IE (cytomegalovirus immediate early) promoter, the EF_{1α} promoter (from human elongation factor 1α), the EM7 promoter, the UbC promoter (from human ubiquitin C). Examples of useful mammalian host cell lines include monkey kidney CVl line transformed by SV40 (COS-7); human embryonic kidney line (293 or 293 cells) ; baby hamster kidney cells (BHK,); Chinese hamster ovary cells (CHO); African green monkey kidney cells (VERO-76); or myeloma cells (*e.g.,* NS/0 cells). For example, the cells are CHO cells.

Other elements of expression constructs/vectors are known in the art and include, for example, enhancers, transcriptional terminators, polyadenylation sequences, nucleic acids encoding selectable or detectable markers and origins of replication.

Of course, the present disclosure contemplates expression of the A20 variant proteins, fragments, analogs or derivatives thereof in any cell, including mammalian cells, bacterial cells, fungal cells, insect cells or plant cells.

Following production of a suitable expression construct, it is introduced into a suitable cell using any method known in the art. Exemplary methods include microinjection, transfection mediated by DEAE-dextran, transfection mediated by liposomes such as by using lipofectamine (Gibco, MD, USA) and/or cellfectin (Gibco, MD, USA), PEG-mediated DNA uptake, electroporation and microparticle bombardment such as by using DNA-coated tungsten or gold particles (Agracetus Inc., WI, USA) amongst others.

The present disclosure also encompasses recombinant cells expressing a A20 variant protein, fragment, analog or derivative. The cells are then cultured under conditions known in the art to produce the A20 variant protein, fragment, analog or derivative.

In one example, the A20 variant protein, fragment, analog or derivative is synthetic, and may therefore be produced using chemical methods known to a person skilled in the art.

For example, synthetic A20 variant proteins, fragments, analogs or derivatives may be prepared using known techniques of solid phase, liquid phase, or peptide condensation, or any combination thereof, and can include natural and/or unnatural amino acids. Amino acids used for polypeptide synthesis may be standard Boc (Nα-amino protected Nα-t-butyloxycarbonyl) amino acid resin with the deprotecting, neutralization, coupling and wash protocols of the original solid phase procedure of Merrifield, J. Am. Chem. Soc., 85:2149-2154, 1963, or the base-labile Nα-amino protected 9-fluorenylmethoxycarbonyl (Fmoc) amino acids described by Carpino and Han, J. Org. Chem., 37:3403-3409, 1972.

As the full length human A20 protein is 790 amino acids in length, convergent or stepwise synthesis techniques are also contemplated for synthesizing the A20 variant proteins, fragments, analogs or derivatives of the disclosure *e.g.,* as described in Muir et al. (1993) Curr. Opin. Biotech., 4:420 and Miller et al., (1989) Science, 245:1149.

Following production/expression/synthesis, the A20 variant protein, fragment, analog or derivative may be purified using a method known in the art. Such purification provides the protein, fragment, analog or derivative substantially free of conspecific protein, nucleic acids, lipids, carbohydrates, and the like. For example, the protein, fragment, analog or derivative will be in a preparation wherein more than about 90% (e.g. 95%, 98% or 99%) of the protein in the preparation is the A20 variant protein, fragment, analog or derivative.

Standard methods of purification may be employed to obtain an isolated A20 variant protein or functional fragment, analog or derivative thereof, including but not limited to various high-pressure (or performance) liquid chromatography (HPLC) and non-HPLC peptide isolation protocols, such as size exclusion chromatography, ion exchange chromatography, phase separation methods, electrophoretic separations, precipitation methods, salting in/out methods, immunochromatography, and/or other methods.

### Nucleic acids, expression constructs/vectors

Also provided herein is an isolated nucleic acid comprising a nucleotide sequence encoding an A20 variant protein or the functional fragment thereof, wherein the nucleotide sequence comprises an A20 SNV variant for modifying the strength of an immune response in a subject as described herein. Exemplary A20 SNV variants are set forth in Table 1-3 hereof. In one example, an isolated nucleic acid of the discloses comprises a nucleotide sequence encoding A20 variant 1 as described herein. In one example, an isolated nucleic acid of the discloses comprises a nucleotide sequence encoding A20 variant 2 as described herein. In one example, an isolated nucleic acid of the discloses comprises a nucleotide sequence encoding A20 variant 3 as described herein. In one example, an isolated nucleic acid of the discloses comprises a nucleotide sequence encoding A20 variant 4 as described herein. In one example, an isolated nucleic acid of the discloses comprises a nucleotide sequence encoding A20 variant 5 as described herein. In one example, an isolated nucleic acid of the discloses comprises a nucleotide sequence encoding A20 variant 6 as described herein. In one example, an isolated nucleic acid of the discloses comprises a nucleotide sequence encoding A20 variant 7 as described herein. In one example, an isolated nucleic acid of the discloses comprises a nucleotide sequence encoding A20 variant 7 as described herein. In one example, an isolated nucleic acid of the discloses comprises a nucleotide sequence encoding A20 variant 8 as described herein. In one example, an isolated nucleic acid of the discloses comprises a nucleotide sequence encoding A20 variant 9 as described herein. In one example, an isolated nucleic acid of the discloses comprises a nucleotide sequence encoding A20 variant 10 as described herein. In one example, an isolated nucleic acid of the discloses comprises a nucleotide sequence encoding A20 variant 11 as described herein. In one example, an isolated nucleic acid of the discloses comprises a nucleotide sequence encoding A20 variant 12 as described herein. In one example, an isolated nucleic acid of the discloses comprises a nucleotide sequence encoding A20 variant 13 as described herein.

Methods of producing isolated nucleic acid molecules are known in the art and described hereinabove, and shall be taken to apply *mutatis mutandis* to the present example of the disclosure.

In one example, the A20 SNV variant for modifying the strength of an immune response in a subject as described herein is provided in the form of an expression construct or vector comprising a nucleic acid encoding an A20 variant protein or functional fragment thereof (*i.e.,* a functional fragment which retains the NF-κB and/or JNK signalling function of the full length variant), wherein the expression construct or vector is capable of expressing the A20 variant protein or the functional fragment thereof in a cell *e.g.,* a mammalian cell. For example, the expression construct may comprise an isolated nucleic acid as described herein.

In one example, the expression construct or vector comprises a nucleic acid encoding an A20 variant protein which increases or decreases NF-κB and/or JNK signalling in a subject relative to if the subject was administered or expressed the reference A20 protein sequence set forth in SEQ ID NO: 1. Exemplary expression constructs or vectors comprise a nucleic acid encoding an A20 variant set forth in Table 1 (e.g., A20 variants 1-13). In accordance with an example in which the A20 variant increases NF-κB and/or JNK signalling in the subject and thereby increases the immune response in a subject, the expression construct or vector may comprise a nucleic acid encoding an A20 variant set forth in Table 2 (e.g., A20 variants 1-10). In accordance with an example in which the A20 variant decreases NF-κB and/or JNK signalling in the subject and thereby decreases the immune response in a subject, the expression construct or vector may comprise a nucleic acid encoding an A20 variant set forth in Table 3 (e.g., A20 variants 11-13).

In another example, the expression construct or vector comprises a nucleic acid encoding an A20 variant protein comprising a sequence at least about 75% or 80% or 85% or 90% or 95% or 97% or 98% or 99% identical to the sequence set forth in SEQ ID NO: 1, provided that the A20 variant protein comprises one of the mutations set forth in Tables 1-3 and exhibits modified NF-κB and/or JNK signalling activity relative the sequence set forth in SEQ ID NO: 1.

For example, an expression construct or vector of the disclosure may comprise a nucleic acid encoding an A20 variant protein comprising a sequence at least about 75% or 80% or 85% or 90% or 95% or 97% or 98% or 99% identical to the sequence set forth in SEQ ID NO: 1, provided that the A20 variant comprises one of the mutations set forth in Table 2 and exhibits increased NF-κB and/or JNK signalling activity relative the sequence set forth in SEQ ID NO: 1. In one example, the expression construct or vector of the disclosure comprise a nucleic acid encoding an A20 variant protein comprising a sequence at least about 75% identical to the sequence set forth in SEQ ID NO: 1, provided that the A20 variant comprises one of the mutations set forth in Table 2 and exhibits increased NF-κB and/or JNK signalling activity relative the sequence set forth in SEQ ID NO: 1. In one example, the expression construct or vector of the disclosure comprise a nucleic acid encoding an A20 variant protein comprising a sequence at least about 80% identical to the sequence set forth in SEQ ID NO: 1, provided that the A20 variant comprises one of the mutations set forth in Table 2 and exhibits increased NF-κB and/or JNK signalling activity relative the sequence set forth in SEQ ID NO: 1. In one example, the expression construct or vector of the disclosure comprise a nucleic acid encoding an A20 variant protein comprising a sequence at least about 85% identical to the sequence set forth in SEQ ID NO: 1, provided that the A20 variant comprises one of the mutations set forth in Table 2 and exhibits increased NF-κB and/or JNK signalling activity relative the sequence set forth in SEQ ID NO: 1. In one example, the expression construct or vector of the disclosure comprise a nucleic acid encoding an A20 variant protein comprising a sequence at least about 90% identical to the sequence set forth in SEQ ID NO: 1, provided that the A20 variant comprises one of the mutations set forth in Table 2 and exhibits increased NF-κB and/or JNK signalling activity relative the sequence set forth in SEQ ID NO: 1. In one example, the expression construct or vector of the disclosure comprise a nucleic acid encoding an A20 variant protein comprising a sequence at least about 95% identical to the sequence set forth in SEQ ID NO: 1, provided that the A20 variant comprises one of the mutations set forth in Table 2 and exhibits increased NF-κB and/or JNK signalling activity relative the sequence set forth in SEQ ID NO: 1. In one example, the expression construct or vector of the disclosure comprise a nucleic acid encoding an A20 variant protein comprising a sequence at least about 97% identical to the sequence set forth in SEQ ID NO: 1, provided that the A20 variant comprises one of the mutations set forth in Table 2 and exhibits increased NF-κB and/or JNK signalling activity relative the sequence set forth in SEQ ID NO: 1. In one example, the expression construct or vector of the disclosure comprise a nucleic acid encoding an A20 variant protein comprising a sequence at least about 98% identical to the sequence set forth in SEQ ID NO: 1, provided that the A20 variant comprises one of the mutations set forth in Table 2 and exhibits increased NF-κB and/or JNK signalling activity relative the sequence set forth in SEQ ID NO: 1. In one example, the expression construct or vector of the disclosure comprise a nucleic acid encoding an A20 variant protein comprising a sequence at least about 99% identical to the sequence set forth in SEQ ID NO: 1, provided that the A20 variant comprises one of the mutations set forth in Table 2 and exhibits increased NF-κB and/or JNK signalling activity relative the sequence set forth in SEQ ID NO: 1. In one example, the expression construct or vector of the disclosure comprise a nucleic acid encoding an A20 variant protein comprising the sequence set forth in SEQ ID NO: 1 with the exception of one of the mutations set forth in Table 2, and exhibits increased NF-κB and/or JNK signalling activity relative the sequence set forth in SEQ ID NO: 1.

For example, an expression construct or vector of the disclosure may comprise a nucleic acid encoding an A20 variant protein comprising a sequence at least about 75% or 80% or 85% or 90% or 95% or 97% or 98% or 99% identical to the sequence set forth in SEQ ID NO: 1, provided that the A20 variant comprises one of the mutations set forth in Table 3 and exhibits decreased NF-κB and/or JNK signalling activity relative the sequence set forth in SEQ ID NO: 1. In one example, the expression construct or vector of the disclosure comprise a nucleic acid encoding an A20 variant protein comprising a sequence at least about 75% identical to the sequence set forth in SEQ ID NO: 1, provided that the A20 variant comprises one of the mutations set forth in Table 3 and exhibits decreased NF-κB and/or JNK signalling activity relative the sequence set forth in SEQ ID NO: 1. In one example, the expression construct or vector of the disclosure comprise a nucleic acid encoding an A20 variant protein comprising a sequence at least about 80% identical to the sequence set forth in SEQ ID NO: 1, provided that the A20 variant comprises one of the mutations set forth in Table 3 and exhibits decreased NF-κB and/or JNK signalling activity relative the sequence set forth in SEQ ID NO: 1. In one example, the expression construct or vector of the disclosure comprise a nucleic acid encoding an A20 variant protein comprising a sequence at least about 85% identical to the sequence set forth in SEQ ID NO: 1, provided that the A20 variant comprises one of the mutations set forth in Table 3 and exhibits decreased NF-κB and/or JNK signalling activity relative the sequence set forth in SEQ ID NO: 1. In one example, the expression construct or vector of the disclosure comprise a nucleic acid encoding an A20 variant protein comprising a sequence at least about 90% identical to the sequence set forth in SEQ ID NO: 1, provided that the A20 variant comprises one of the mutations set forth in Table 3 and exhibits decreased NF-κB and/or JNK signalling activity relative the sequence set forth in SEQ ID NO: 1. In one example, the expression construct or vector of the disclosure comprise a nucleic acid encoding an A20 variant protein comprising a sequence at least about 95% identical to the sequence set forth in SEQ ID NO: 1, provided that the A20 variant comprises one of the mutations set forth in Table 3 and exhibits decreased NF-κB and/or JNK signalling activity relative the sequence set forth in SEQ ID NO: 1. In one example, the expression construct or vector of the disclosure comprise a nucleic acid encoding an A20 variant protein comprising a sequence at least about 97% identical to the sequence set forth in SEQ ID NO: 1, provided that the A20 variant comprises one of the mutations set forth in Table 3 and exhibits decreased NF-κB and/or JNK signalling activity relative the sequence set forth in SEQ ID NO: 1. In one example, the expression construct or vector of the disclosure comprise a nucleic acid encoding an A20 variant protein comprising a sequence at least about 98% identical to the sequence set forth in SEQ ID NO: 1, provided that the A20 variant comprises one of the mutations set forth in Table 3 and exhibits decreased NF-κB and/or JNK signalling activity relative the sequence set forth in SEQ ID NO: 1. In one example, the expression construct or vector of the disclosure comprise a nucleic acid encoding an A20 variant protein comprising a sequence at least about 99% identical to the sequence set forth in SEQ ID NO: 1, provided that the A20 variant comprises one of the mutations set forth in Table 3 and exhibits decreased NF-κB and/or JNK signalling activity relative the sequence set forth in SEQ ID NO: 1. In one example, the expression construct or vector of the disclosure comprise a nucleic acid encoding an A20 variant protein comprising the sequence set forth in SEQ ID NO: 1 with the exception of one of the mutations set forth in Table 3, and exhibits decreased NF-κB and/or JNK signalling activity relative the sequence set forth in SEQ ID NO: 1.

Methods for producing expression constructs for expressing a protein of interest (such as an A20 variant protein of the disclosure) in a cell will be apparent to the skilled artisan. For example, the nucleic acid to be expressed in the cell *i.e.,* a nucleic acid encoding the A20 variant protein described herein, will be operably-linked to a promoter for inducing expression of the A20 variant protein in the cell. For example, the nucleic acid will be linked to a promoter operable in a variety of cells of a subject, such as, for example, a viral promoter, *e.g.,* a CMV promoter (*e.g.,* a CMV-IE promoter) or a SV-40 promoter. Additional suitable promoters are known in the art and shall be taken to apply mutatis mutandis to the present example of the disclosure.

In one example, the nucleic acid will be prepared in the form of an expression construct. As used herein, the term "expression construct" refers to a nucleic acid that has the ability to confer expression on a nucleic acid to which it is operably connected, in a cell. Within the context of the present disclosure, it is to be understood that an expression construct may comprise or be a plasmid, minicircle, bacteriophage, phagemid, cosmid, virus sub-genomic or genomic fragment, or other nucleic acid capable of maintaining and/or replicating heterologous DNA in an expressible format. Accordingly, a plasmid, minicircle, bacteriophage, phagemid, cosmid, virus sub-genomic or genomic fragment vector may be used to deliver an A20 variant of the disclosure to a subject.

Methods for the construction of a suitable expression constructs of the disclosure will be apparent to the skilled artisan and are described, for example, in Ausubel et al., (In: Current Protocols in Molecular Biology. Wiley Interscience, ISBN 047 150338, 1987) or Sambrook et al (In: Molecular Cloning: Molecular Cloning: A Laboratory Manual, Cold Spring Harbor Laboratories, New York, Third Edition 2001). For example, each of the components of the expression construct is amplified from a suitable template nucleic acid using, for example, PCR and subsequently cloned into a suitable expression construct, such as for example, a plasmid or a phagemid.

Plasmid vectors suitable for use with such an expression construct are known in the art and/or described herein. For example, an expression vector suitable for the method of the present disclosure in a mammalian cell is, for example, a vector of the pcDNA vector suite supplied by Invitrogen, a vector of the pCI vector suite (Promega), a vector of the pCMV vector suite (Clontech), a pM vector (Clontech), a pSI vector (Promega), a VP 16 vector (Clontech) or a vector of the pcDNA vector suite (Invitrogen). The skilled artisan will be aware of additional plasmid vectors and sources of such vectors, such as, for example, Life Technologies Corporation, Clontech or Promega.

Means for introducing expression constructs and vectors into a cell for expression of a desired protein are also known to those skilled in the art. The technique used for a given organism depends on the known successful techniques. Means for introducing recombinant DNA into cells include microinjection, transfection mediated by DEAE-dextran, transfection mediated by liposomes such as by using lipofectamine (Gibco, MD, USA) and/or cellfectin (Gibco, MD, USA), PEG-mediated DNA uptake, electroporation and microparticle bombardment such as by using DNA- coated tungsten or gold particles (Agracetus Inc., WI, USA) amongst others.

In another example, the vector is a viral vector. Accordingly, a viral vector based on any appropriate virus may be used to deliver an A20 variant of the disclosure to a subject. In addition, hybrid viral systems may be of use. The choice of viral delivery system will depend on various parameters, such as the tissue targeted for delivery, transduction efficiency of the system, pathogenicity, immunological and toxicity concerns, and the like.

Commonly used classes of viral systems used in gene therapy can be categorized into two groups according to whether their genomes integrate into host cellular chromatin (oncoretroviruses and lentiviruses) or persist in the cell nucleus predominantly as extrachromosomal episomes (adeno-associated virus, adenoviruses and herpesviruses). In one example, a viral vector of the disclosure integrates into a host cell's chromatin. In another example, a viral vector of the disclosure persists in a host cell's nucleus as an extrachomosomal episome.

In one example, a viral vector is an adenoviral (AdV) vector. Adenoviruses are medium-sized double-stranded, non-enveloped DNA viruses with linear genomes that is between 26-48 Kbp. Adenoviruses gain entry to a target cell by receptor-mediated binding and internalization, penetrating the nucleus in both non-dividing and dividing cells. Adenoviruses are heavily reliant on the host cell for survival and replication and are able to replicate in the nucleus of vertebrate cells using the host's replication machinery.

In one example, a viral vector is from the Parvoviridae family. The Parvoviridae is a family of small single-stranded, non-enveloped DNA viruses with genomes approximately 5000 nucleotides long. Included among the family members is adeno-associated virus (AAV). In one example, a viral vector used to deliver an A20 variant of the disclosure to a subject is an AAV. The AAV may be from any serotype known to infect humans, for example, serotype 2, 3, 4, 5, 6, 7, 8, 9, 10 ,11 or 12. AAV is a dependent parvovirus that generally requires co-infection with another virus (typically an adenovirus or herpesvirus) to initiate and sustain a productive infectious cycle. In the absence of such a helper virus, AAV is still competent to infect or transduce a target cell by receptor-mediated binding and internalization, penetrating the nucleus in both non-dividing and dividing cells. Because progeny virus is not produced from AAV infection in the absence of helper virus, the extent of transduction is restricted only to the initial cells that are infected with the virus. It is this feature which makes AAV a desirable vector for the present disclosure. Furthermore, unlike retrovirus, adenovirus, and herpes simplex virus, AAV appears to lack human pathogenicity and toxicity (Kay, et al., Nature. 424: 251 (2003)). Since the genome normally encodes only two genes it is not surprising that, as a delivery vehicle, AAV is limited by a packaging capacity of 4.5 single stranded kilobases (kb).

Another viral delivery system useful for delivery of an A20 variant of the disclosure to a subject is a system based on viruses from the family Retroviridae. Retroviruses comprise single-stranded RNA animal viruses that are characterized by two unique features. First, the genome of a retrovirus is diploid, consisting of two copies of the RNA. Second, this RNA is transcribed by the virion-associated enzyme reverse transcriptase into double-stranded DNA. This double-stranded DNA or provirus can then integrate into the host genome and be passed from parent cell to progeny cells as a stably-integrated component of the host genome.

In some examples, the viral vector used to deliver an A20 variant of the disclosure to a subject is a lentivirus. Lentivirus vectors are often pseudotyped with vesicular somatitis virus glycoprotein (VSV-G), and have been derived from the human immunodeficiency virus (HIV); visan-maedi, which causes encephalitis (visna) or pneumonia in sheep; equine infectious anemia virus (EIAV), which causes autoimmune hemolytic anemia and encephalopathy in horses; feline immunodeficiency virus (FIV), which causes immune deficiency in cats; bovine immunodeficiency virus (BIV) which causes lymphadenopathy and lymphocytosis in cattle; and simian immunodeficiency virus (SIV), which causes immune deficiency and encephalopathy in non-human primates. Vectors that are based on HIV generally retain <5% of the parental genome, and <25% of the genome is incorporated into packaging constructs, which minimizes the possibility of the generation of reverting replication-competent HIV. Biosafety has been further increased by the development of self-inactivating vectors that contain deletions of the regulatory elements in the downstream long-terminal-repeat sequence, eliminating transcription of the packaging signal that is required for vector mobilization. One of the main advantages to the use of lentiviral vectors is that gene transfer is persistent in most tissues or cell types, even following cell division of the transduced cell.

Other viral or non-viral systems known to those skilled in the art may be used to deliver an A20 variant of the disclosure to a subject, including but not limited to gene-deleted adenovirus-transposon vectors (see Yant, et al., Nature Biotech. 20:999-1004 (2002)); systems derived from Sindbis virus or Semliki forest virus (see Perri, et al, J. Virol. 74(20):9802-07 (2002)); systems derived from Newcastle disease virus or Sendai virus.

### T-cells

In one example, the present disclosure provides a cell *e.g.,* a T-cell, which has been modified to express an A20 variant described herein. It is contemplated that such cell may be used to modulate a subject's immune response in a method of the disclosure. For example, a subject in need of an increased immune response may be provided a T-cell expressing an A20 variant protein of the disclosure e.g., an A20 variant set forth in Table 2, which will increase the strength of the subject's immune response relative to the subject's immune response in the absence of the A20 variant protein being expressed by the T-cell. The cell may be modified to express the A20 variant protein according to any suitable means known in the art. For example, the cell may be modified to express an A20 variant protein of the disclosure using a meganuclease, a zinc finger nuclease (ZFN), a transcription activator-like effector-based nuclease (TALEN) or a clustered regularly interspaced short palindromic repeats (CRISPR/Cas) system.

In one example, the cell is an autologous cell *e.g.,* an autologous T-cell. In another example, the cell is an allogeneic cell *e.g.,* an allogeneic T-cell lacking expression of a functional T-cell receptor (TCR).

In accordance with an example in which the modified cell is a T-cell, the T-cell may comprise or express a chimeric antigen receptor (CAR). Accordingly, a T-cell which is modified to express an A20 variant protein may be a CAR-T cell.

In one example, the CAR comprises an antigen binding domain in the extracellular region.

In one example, the antigen binding domain is a murine antibody or antibody fragment comprising an antigen binding domain. In one example, the antigen binding domain is a humanized antibody or antibody fragment comprising an antigen binding domain. In one example, the antigen binding domain is a human antibody or antibody fragment comprising an antigen binding domain.

The choice of an antigen binding domain can depend upon the type and number of ligands or receptors that define the surface of a target cell. For example, the antigen binding domain may be chosen to recognize an antigen that acts as a cell surface marker on target cells associated with a particular disease state. Examples of cell surface markers that may act as ligands or receptors include a cell surface marker associated with a particular disease state, *e.g.,* cell surface makers for viral diseases, bacterial diseases parasitic infections, autoimmune diseases and disorders associated with unwanted cell proliferation, *e.g.,* a cancer, *e.g.*, a cancer described herein.

In certain examples, the antigen binding domain recognizes an antigen of a proliferative disorder e.g., cancer, including but not limited to primary or metastatic melanoma, thymoma, lymphoma, sarcoma, lung cancer (*e.g.,* NSCLC or SCLC), liver cancer, non-Hodgkin's lymphoma, Hodgkin' s lymphoma, leukemias, multiple myeloma, glioblastoma, neuroblastoma, uterine cancer, cervical cancer, renal cancer, thyroid cancer, bladder cancer, kidney cancer and adenocarcinomas such as breast cancer, prostate cancer, ovarian cancer, pancreatic cancer, colon cancer and the like. In some embodiments, the cancer is B-cell acute lymphoid leukemia ("BALL"), T-cell acute lymphoid leukemia ("TALL"), acute lymphoid leukemia (ALL), acute myelogenous leukemia (AML); one or more chronic leukemias including but not limited to chronic myelogenous leukemia (CML), chronic lymphocytic leukemia (CLL); additional hematologic cancers or hematologic conditions including, but not limited to B cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative conditions, MALT lymphoma, mantle cell lymphoma, Marginal zone lymphoma, multiple myeloma, myelodysplasia and myelodysplasia syndrome, non-Hodgkin's lymphoma, plasmablastic lymphoma, plasmacytoid dendritic cell neoplasm, Waldenstrom macroglobulinemia.

In one example, the antigen binding domain binds specifically to a tumor antigen which comprises one or more antigenic cancer epitopes immunologically recognized by tumor infiltrating lymphocytes (TIL) derived from a cancer tumor of a mammal.

Tumor antigens can be proteins that are produced by tumor cells that elicit an immune response, particularly T-cell mediated immune responses. The selection of the antigen binding domain of the dsiclosure will depend on the particular type of cancer to be treated. Tumor antigens are well known in the art and include, for example, a glioma- associated antigen, carcinoembryonic antigen (CEA), EGFRvIII, IL-l lRa, IL-13Ra, EGFR, FAP, B7H3, Kit, CA-IX, CS-1, MUC1, BCMA, bcr-abl, HER2, β-human chorionic gonadotropin, alphafetoprotein (AFP), ALK, CD19, CD123, cyclin Bl, lectin-reactive AFP, Fos-related antigen 1, ADRB3, thyroglobulin, EphA2, RAGE-1, RUl, RU2, SSX2, AKAP-4, LCK, OY-TESl, PAX5, SART3, CLL-1, fucosyl GM1, GloboH, MN-CA IX, EPCAM, EVT6-AML, TGS5, human telomerase reverse transcriptase, plysialic acid, PLAC1, RUl, RU2 (AS), intestinal carboxyl esterase, lewisY, sLe, LY6K, mut hsp70-2, M-CSF, MYCN, RhoC, TRP-2, CYP1B1, BORIS, prostase, prostate-specific antigen (PSA), PAX3, PAP, NY-ESO-1, LAGE-la, LMP2, NCAM, p53, p53 mutant, Ras mutant, gplOO, prostein, OR51E2, PANX3, PSMA, PSCA, Her2/neu, hTERT, HMWMAA, HAVCR1, VEGFR2, PDGFR-beta, survivin and telomerase, legumain, HPV E6,E7, sperm protein 17, SSEA-4, tyrosinase, TARP, WT1, prostate-carcinoma tumor antigen-1 (PCTA-1), ML-IAP, MAGE, MAGE- A 1,MAD-CT- 1, MAD-CT-2, Melan A/MART 1, XAGE1, ELF2M, ERG (TMPRSS2 ETS fusion gene), NA17, neutrophil elastase, sarcoma translocation breakpoints, NY-BR-1, ephrinB2, CD20, CD22, CD24, CD30, CD33, CD38, CD44v6, CD97, CD171, CD179a, androgen receptor, FAP, insulin growth factor (IGF)-I, IGF-II, IGF-I receptor, GD2, o-acetyl-GD2, GD3, GM3, GPRC5D, GPR20, CXORF61, folate receptor (FRa), folate receptor beta, ROR1, Flt3, TAG72, TN Ag, Tie 2, TEM1, TEM7R, CLDN6, TSHR, UPK2, and mesothelin. In one example, the tumor antigen is selected from the group consisting of folate receptor (FRa), mesothelin, EGFRvIII, IL-13Ra, CD123, CD19, CD33, BCMA, GD2, CLL-1, CA-IX, MUC1, HER2, and any combination thereof. In one example, the tumor antigen is CD19.

In one example, the tumor antigen comprises one or more antigenic cancer epitopes associated with a malignant tumor. Malignant tumors express a number of proteins that can serve as target antigens for an immune attack. These molecules include but are not limited to tissue-specific antigens such as MART-1, tyrosinase and GP 100 in melanoma and prostatic acid phosphatase (PAP) and prostate-specific antigen (PSA) in prostate cancer. Other target antigens include transformation-related molecules such as the oncogene HER-2/Neu/ErbB-2. Yet another group of target antigens are onco-fetal antigens such as carcinoembryonic antigen (CEA). In B-cell lymphoma the tumor- specific idiotype immunoglobulin constitutes a truly tumor- specific immunoglobulin antigen that is unique to the individual tumor. B-cell differentiation antigens such as CD 19, CD20 and CD37 are other candidates for target antigens in B-cell lymphoma.

In some examples, the tumor antigen is a tumor antigen described in WO2015/120096, WO2015/142675, WO2016/019300, WO2016/011210, WO2016/109410 and WO2016/069283, the contents of which are incorporated by reference in their entirety.

Depending on the desired antigen to be targeted, the sequence encoding the CAR can be engineered to include the appropriate antigen binding domain that is specific to the desired antigen target.

The antigen binding domain of the CAR-T cell can be derived from an antibody molecule, *e.g.,* one or more of monoclonal antibodies, polyclonal antibodies, recombinant antibodies, human antibodies, humanized antibodies, single-domain antibodies *e.g*., a heavy chain variable domain (VH), a light chain variable domain (VL) from *e.g*., human, and a variable domain (VHH). In some examples, it is beneficial for the antigen binding domain to be derived from the same species in which the CAR will ultimately be used in, *e.g.,* for use in humans, it may be beneficial for the antigen binding domain of the CAR, described herein, to comprise a human or a humanized antigen binding domain.

In some examples, the antigen binding domain comprises a fragment of an antibody that is sufficient to confer recognition and specific binding to the target antigen. Examples of an antibody fragment include, but are not limited to, an Fab, Fab', F(ab')2, or Fv fragment, an scFv antibody fragment, a linear antibody, single domain antibody such as an sdAb (either VL or VH), a camelid VHH domain, and multi- specific antibodies formed from antibody fragments.

In other examples, the CAR-T cell comprises a bispecific CAR. Exemplary bispecific CARs which are contemplated are described in WO2018/049471, the full contents of which is incorporated herein.

T-cells, including CAR-T cells, may be produced according to any methods known in the art. In one example, a T-cell *e.g.,* a CAR-T cell, which expresses an A20 variant protein of the disclosure may be produced by introducing into the cell an expression construct or vector encoding an A20 variant protein as described herein. Methods of producing CAR-T cells which express genes of interest are described in, for example, n WO2018/049471, the full contents of which is incorporated herein.

In one example, the T-cell may be present in a subpopulation of T-cells which have been selected for particular properties *e.g*., based on HLA typing and/ resistance to an immunosuppressant.

T-cells of the disclosure may be formulated for administration in adoptive T-cell therapy.

Formulation of the composition to be administered will vary according to the route of administration and formulation (*e.g*., solution, emulsion) selected. An appropriate pharmaceutical composition comprising the composition of the present disclosure to be administered can be prepared in a physiologically acceptable carrier. A mixture of compositions can also be used. For solutions or emulsions, suitable carriers include, for example, aqueous or alcoholic/aqueous solutions, emulsions or suspensions, including saline and buffered media. Parenteral vehicles can include sodium chloride solution, Ringer's dextrose, dextrose and sodium chloride, lactated Ringer's or fixed oils. A variety of appropriate aqueous carriers are known to the skilled artisan, including water, buffered water, buffered saline, polyols (*e.g*., glycerol, propylene glycol, liquid polyethylene glycol), dextrose solution and glycine. Intravenous vehicles can include various additives, preservatives, or fluid, nutrient or electrolyte replenishers (See, generally, Remington's Pharmaceutical Science, 16th Edition, Mack, Ed. 1980). The compositions can optionally contain pharmaceutically acceptable auxiliary substances as required to approximate physiological conditions such as pH adjusting and buffering agents and toxicity adjusting agents, for example, sodium acetate, sodium chloride, potassium chloride, calcium chloride and sodium lactate.

The optimum concentration of cell populations in the chosen medium can be determined empirically, according to procedures well known to the skilled artisan, and will depend on the ultimate pharmaceutical formulation desired.

In one example, a plurality of T-cells described herein, or compositions comprising same, are provided in a bank. In one example, the T-cells in the bank have been modified to express A20 variant proteins of the disclosure. Different T-cells within the bank may be modified to express different A20 variant proteins as described herein, depending on the extent to which the immune system of a subject is to be increased. In addition, the T-cells in the bank may be CAR-T cells of the disclosure.

In accordance with this example, the T-cells of the disclosure may be "banked" for future use, at a cell bank or depository or storage facility, or any place where such as cells are kept cryopreserved, *e.g*., in liquid nitrogen, for safekeeping. Furthermore, appropriate computer systems can be used for data processing, to maintain records relating to donor information and to ensure rapid and efficient retrieval of cells from the storage repositories.

In one example, each of the storage containers (*e.g*., bags or tubes) can be tagged with positive identification based on a distinctive property associated with the donor, lines or cell type, prior to storing in a bank according to the disclosure. For example, DNA genetic fingerprint and HLA typing may be used with secured identification mechanism such as acceptable methods using microchips, magnetic strip, and/or bar code labels. This identification step may be included in the banking process.

In one example, at least one of the HLA alleles in the T-cells in each composition in the bank has been identified. In one example, the HLA is a HLA-DR allele.

At the time of use, only the required storage unit is retrieved, the number of units necessary to fulfil a desired dosage being selectable. Certain diseases may require cell therapy that includes a series of repeated treatments. The population of cells may be extracted from the bank and increased by cellular expansion before preparation of the pharmaceutical composition and administration to the subject.

T-cells of the disclosure which have been modified to express A20 variant proteins may be cryopreserved for adoptive T cell transfer.

### Functional assays

The ability of an A20 variant to modify the strength of an immune response in a subject *i.e.,* , increase the strength of the immune response or decrease the strength of the immune response, may be determined using any suitable method or assay known in the art. For example, the effect of an A20 variant of the disclosure on the strength of the immune response may be determined by performing an NF-κB-luciferase reporter assay or by measuring JNK activation upon hTNFα stimulation as described in Examples 3 and 4. Alternatively, or in addition, the effect of an A20 variant of the disclosure on the strength of the immune response may be determined *in vivo* by performing experiments similar to those described in Example 6.

### Pharmaceutical compositions

The A20 variant protein, expression construct or vector encoding same, or T cell as described herein may be provided in a pharmaceutical composition. The pharmaceutical composition (syn. active ingredient) may be administered parenterally, topically, orally, or locally or by aerosol administration, or transdermal administration, for prophylactic or for therapeutic treatment Formulation of the active ingredient to be administered will vary according to the agent, route of administration and formulation (*e.g*., solution, emulsion, capsule) selected. An appropriate pharmaceutical composition comprising the active ingredient to be administered can be prepared in a physiologically acceptable carrier. A mixture of active ingredients which increase A20 activity can also be used. For solutions or emulsions, suitable carriers include, for example, aqueous or alcoholic/aqueous solutions, emulsions or suspensions, including saline and buffered media. Parenteral vehicles can include sodium chloride solution, Ringer's dextrose, dextrose and sodium chloride, lactated Ringer's or fixed oils. A variety of appropriate aqueous carriers are known to the skilled artisan, including water, buffered water, buffered saline, polyols (*e.g*., glycerol, propylene glycol, liquid polyethylene glycol), dextrose solution and glycine. Intravenous vehicles can include various additives, preservatives, or fluid, nutrient or electrolyte replenishers (See, generally, Remington's Pharmaceutical Science, 16th Edition, Mack, Ed. 1980). The compositions can optionally contain pharmaceutically acceptable auxiliary substances as required to approximate physiological conditions such as pH adjusting and buffering agents and toxicity adjusting agents, for example, sodium acetate, sodium chloride, potassium chloride, calcium chloride and sodium lactate. The active ingredient(s) of the disclosure can be lyophilized for storage and reconstituted in a suitable carrier prior to use according to art-known lyophilization and reconstitution techniques.

The optimum concentration of the active ingredient(s) in the chosen medium can be determined empirically, according to procedures known to the skilled artisan, and will depend on the ultimate pharmaceutical formulation desired.

The dosage ranges for the administration of the active ingredient are those large enough to produce the desired effect *i.e.,* increase the strength of the immune response or decrease the strength of the immune response in a subject.

The dosage should not be so large as to cause adverse side effects, such as hyper viscosity syndromes, pulmonary edema, congestive heart failure, and the like. Generally, the dosage will vary with the age, condition, sex and extent of the disease in the patient and can be determined by one of skill in the art. The dosage can be adjusted by the individual physician in the event of any complication. Dosage can vary from about 0.1 mg/kg to about 300 mg/kg, such as from about 0.2 mg/kg to about 200 mg/kg, for example from about 0.5 mg/kg to about 20 mg/kg, in one or more dose administrations daily, for one or several days.

One or more active ingredients of the present disclosure can be administered to an individual by an appropriate route, either alone or in combination with (before, simultaneous with, or after) another drug or agent.

It will be appreciated by those skilled in the art that some active ingredients (*e.g*., A20 protein) of the present disclosure may be introduced into a subject by administering an expression construct of the disclosure or a cell expressing the active ingredient. A variety of methods can be used for introducing a nucleic acid encoding the active ingredient into a target cell *in vivo.* For example, the naked nucleic acid may be injected at the target site, may be encapsulated into liposomes, or may be introduced by way of a viral vector.

In one example, the pharmaceutical composition of the disclosure is a vaccine. In accordance with this example, the vaccine may comprise an A20 variant protein or an expression construct or vector encoding same which will increase the immune response in the subject to which it is administered. For example, the vaccine may comprise an A20 variant set forth in Table 2 or variant thereof as described herein. The vaccine will also comprise one or more antigens. In accordance with this example, the presence of the A20 variant in the vaccine will increase the immune response elicited against the antigen. Examples of suitable antigens include, but are not limited to those from ebola virus, influenza virus, pneumococcal, meningococcal, rotavirus, polio, hepatitis (e.g., HCV), chickenpox, small pox, measles, mumps, rubella, whooping cough, HPV, , HIV, DENV, plasmodium sp., tetanus and diphtheria. However, it is contemplated that the A20 variant may be formulated with any antigen. In some examples, the vaccine may comprise an adjuvant. The adjuvant may be any adjuvant known in the art. Examples of suitable adjuvants include but are not limited to alum, pertussis toxin, lacto fucopentaose III, phosphopolymer, complete Freund's adjuvant, monophosphoryl lipid A, 3-de-O-acylated monophosphoryl lipid A (3D-MPL), aluminium salt, CpG-containing oligonucleotides, immunostimulatory DNA sequences, saponin, Montanide ISA 720, SAF, ISCOMS, MF-59, SBAS-3, SBAS-4, Detox, RC-529, aminoalkyl glucosaminide 4-phosphate, and LbeIF4A or combinations thereof

In some examples, the pharmaceutical composition of the disclosure *e.g*., vaccine, comprises a surfactant. The surfactant may be any surfactant known in the art. Examples of suitable surfactants include, but are not limited to polysorbate 80 (Tween 80^{®}), phospholipids, oleic acid (omega-9 fatty acid), Triton X-100 and Nonoxynol-9 In some examples, the pharmaceutical composition of the disclosure *e.g*., vaccine, comprises a stabiliser. The stabiliser may be any stabiliser known in the art. Examples of suitable stabilisers include, but are not limited to sugars such as lactose and sucrose, amino acids such as glycine and monosodiumglutamate (salts of amino acids), proteins such as recombinant human albumin (Recombumin^{®}) derived from baker's yeast or fetal bovine (cow) serum and gelatin and partially hydrolysed collagen

The A20 variant may be formulated or administered in conjunction with one or more therapeutic, prophylactic or immunomodulatory agents.

In one example, an A20 variant of the disclosure *e.g*., as set forth in Table 2, may be formulated or administered in conjunction with a chemotherapeutic agent *e.g.,* for cancer treatment. Suitable chemotherapeutic agents are known in the art. Examples of chemotherapeutic agents include, but are not limited to actinomycin, azacitidine, azathioprine, bleomycin, bortezomib, carboplatin, capecitabine, cisplatin, chlorambucil, cyclophosphamide, cytarabine, Daunorubicin, Docetaxel, Doxifluridine, Doxorubicin, Epirubicin, Epothilone, Etoposide, Fluorouracil, Gemcitabine, Hydroxyurea, Idarubicin, Imatinib, Irinotecan, Mechlorethamine, Mercaptopurine, Methotrexate, Mitoxantrone, Oxaliplatin, Paclitaxel, Pemetrexed, Teniposide, Tioguanine, Topotecan, Valrubicin, Vemurafenib, Vinblastine, Vincristine, Vindesine and Vinorelbine.

In another example, an A20 variant of the disclosure *e.g*., as set forth in Table 3, may be formulated or administered in conjunction with an agent for treating or managing autoimmune disease. Suitable agents for treating or managing autoimmune disease are known in the art. Examples of such agents include, but are not limited to prednisone, prednisolone, hydrocortisone, dexamethasone, cyclosporine, budesonide, tacrolimus, sirolimus, everolimus, azathioprine, leflunomide, mycophenolate, abatacept, adalimumab, anakinra, certolizumab etanercept golimumab, infliximab, ixekizumab, natalizumab, rituximab, secukinumab tocilizumab, ustekinumab, vedolizumab, basiliximab, daclizumab and muromonab.

In another example, an A20 variant of the disclosure *e.g*., as set forth in Table 3, may be formulated or administered in conjunction with an agent for treating or managing transplantation tolerance and/or rejection. Suitable agents for treating or managing transplantation tolerance and/or rejection are known in the art. Examples of agents for treating or managing transplantation tolerance and/or rejection include, but are not limited to tacrolimus, cyclosporine, Mycophenolate Mofetil, mycophenolate sodium, azathioprine, sirolimus, prednisone, prednisolone, dexamethasone and leflunomide.

### Methods of treatment

The inventors have shown *e.g.,* in *in vitro* and *in vivo* models, that expression of A20 SNV variants described herein can be used to modulate NF-κB and/or JNK signalling. In doing so, the inventors have shown that some SNV variants of the A20 protein decrease a subject's immune response relative to if the subject expressed a reference A20 protein sequence (*e.g.,* a wild-type A20 variant) and other variants of the A20 protein increase a subject's immune response relative to if the subject had the reference A20 protein sequence (*e.g.,* a wild-type A20 variant). By controlling which A20 variant is expressed in or administered to a subject, the strength of the subject's immune response may be modified *i.e.,* "tuned up" by expressing in or administering to the subject an A20 variant shown to increase the immune response relative to a wild-type or reference A20 protein, or "tuned down" by expressing in or administering to the subject an A20 variant shown to decrease the immune response relative to a wild-type or reference A20 protein. Exemplary A20 variants of the disclosure *e.g*., variants set forth in Tables 1-3 and functional fragments, analogs or derivatives thereof, have been described herein and shall be taken to apply *mutatis mutandis* to each and every examples describing a methods of treatment.

Accordingly, the present disclosure provides a method of modifying the strength of an immune response in a subject by administering to, or expressing in, the subject an A20 variant which will increase or decrease the strength of the subject's immune response relative to the subject's immune response in the absence of the A20 variant being administered or expressed.

In one example, the method of the disclosure comprises increasing the subject immune response by administering to, or expressing in, the subject an A20 variant set forth in Table 2 or a functional fragments, analogs or derivatives thereof as described herein.

In another example, the method of the disclosure comprises decreasing the subject immune response by administering to, or expressing in, the subject an A20 variant set forth in Table 3 or a functional fragments, analogs or derivatives thereof as described herein.

In one example, the A20 variant is provided in the form of a protein, as described herein. In one example, the A20 variant is provided in the form of an expression construct or vector encoding the A20 variant protein, as described herein. In one example, the method comprises administering to the subject a cell *e.g.,* a T-cell, which has been modified to express the A20 variant, as described herein. The T-cell may be an autologous T-cell or an allogeneic T-cell. In one example, the T-cell is a CAR-T cell, as described herein.

In one example, the subject in whom the strength of the immune response is to be modified is suffering from cancer and the A20 variant administered to or expressed in the subject is an A20 variant set forth in Table 2 or a functional fragment, analog or derivative thereof as described herein. The cancer to be treated may be any cancer, including, but not limited to, lymphoblastic leukemia (ALL), acute myeloid leukemia, adrenocortical carcinoma, basal-cell carcinoma, bile duct cancer, bladder cancer, bone tumor, osteosarcoma/malignant fibrous histiocytoma, brainstem glioma, brain tumor, cerebellar astrocytoma, cerebral astrocytoma/malignant glioma, ependymoma, medulloblastoma, supratentorial primitive neuroectodermal tumors, breast cancer, bronchial adenomas/carcinoids, Burkitt's lymphoma, carcinoid tumor, cervical cancer, chronic lymphocytic leukemia, chronic myelogenous leukemia, chronic myeloproliferative disorders, colon cancer, cutaneous T-cell lymphoma, endometrial cancer, ependymoma, esophageal cancer, Ewing's sarcoma, intraocular melanoma, retinoblastoma, gallbladder cancer, gastric cancer, gastrointestinal carcinoid tumor, gastrointestinal stromal tumor (GIST), germ cell tumor, glioma, childhood visual pathway and hypothalamic, Hodgkin lymphoma, melanoma, islet cell carcinoma, Kaposi sarcoma, renal cell cancer, laryngeal cancer, leukaemias, lymphomas, mesothelioma, neuroblastoma, non-Hodgkin lymphoma, oropharyngeal cancer, osteosarcoma, ovarian cancer, pancreatic cancer, parathyroid cancer, pharyngeal cancer, pituitary adenoma, plasma cell neoplasia, prostate cancer, renal cell carcinoma, retinoblastoma, sarcoma, testicular cancer, thyroid cancer, or uterine cancer. =

In accordance with an example in which the subject is suffering from cancer, the subject may be receiving treatment by immunotherapy or has been prescribed a treatment by immunotherapy. In this respect, it is contemplated that the A20 variant of the disclosure may be administered in conjunction with one or more other agents for treatment of cancer. Examples of anticancer agents include, but are not limited to, immune checkpoint inhibitor, a targeted antibody immunotherapy, a CAR-T cell therapy, an oncolytic virus, or a cytostatic drug. In one example, the methods of the present disclosure provide and A20 variant with an anti-cancer agent selected from the group consisting of: Yervoy (ipilimumab, BMS); Keytruda (pembrolizumab, Merck); Opdivo (nivolumab, BMS); MEDI4736 (AZ/MedImmune); MPDL3280A (Roche/Genentech); Tremelimumab (AZ/MedImmune); CT-011 (pidilizumab, CureTech); BMS-986015 (lirilumab, BMS); MEDI0680 (AZ/MedImmune); MSB-0010718C (Merck); PF-05082566 (Pfizer); MEDI6469 (AZ/MedImmune); BMS-986016 (BMS); BMS-663513 (urelumab, BMS); IMP321 (Prima Biomed); LAG525 (Novartis); ARGX-110 (arGEN-X); PF-05082466 (Pfizer); CDX-1127 (varlilumab; CellDex Therapeutics); TRX-518 (GITR Inc.); MK-4166 (Merck); JTX-2011 (Jounce Therapeutics); ARGX-115 (arGEN-X); NLG-9189 (indoximod, NewLink Genetics); INCB024360 (Incyte); IPH2201 (Innate Immotherapeutics/AZ); NLG-919 (NewLink Genetics); anti-VISTA (JnJ); Epacadostat (INCB24360, Incyte); F001287 (Flexus/BMS); CP 870893 (University of Pennsylvania); MGA271 (Macrogenix); Emactuzumab (Roche/Genentech); Galunisertib (Eli Lilly); Ulocuplumab (BMS); BKT140/BL8040 (Biokine Therapeutics); Bavituximab (Peregrine Pharmaceuticals); CC 90002 (Celgene); 852A (Pfizer); VTX-2337 (VentiRx Pharmaceuticals); IMO-2055 (Hybridon, Idera Pharmaceuticals); LY2157299 (Eli Lilly); EW-7197 (Ewha Women's University, Korea); Vemurafenib (Plexxikon); Dabrafenib (Genentech/GSK); BMS-777607 (BMS); BLZ945 (Memorial Sloan-Kettering Cancer Centre); Unituxin (dinutuximab, United Therapeutics Corporation); Blincyto (blinatumomab, Amgen); Cyramza (ramucirumab, Eli Lilly); Gazyva (obinutuzumab, Roche/Biogen); Kadcyla (ado-trastuzumab emtansine, Roche/Genentech); Perjeta (pertuzumab, Roche/Genentech); Adcetris (brentuximab vedotin, Takeda/Millennium); Arzerra (ofatumumab, GSK); Vectibix (panitumumab, Amgen); Avastin (bevacizumab, Roche/Genentech); Erbitux (cetuximab, BMS/Merck); Bexxar (tositumomab-I131, GSK); Zevalin (ibritumomab tiuxetan, Biogen); Campath (alemtuzumab, Bayer); Mylotarg (gemtuzumab ozogamicin, Pfizer); Herceptin (trastuzumab, Roche/Genentech); Rituxan (rituximab, Genentech/Biogen); volociximab (Abbvie); Enavatuzumab (Abbvie); ABT-414 (Abbvie); Elotuzumab (Abbvie/BMS); ALX-0141 (Ablynx); Ozaralizumab (Ablynx); Actimab-C (Actinium); Actimab-P (Actinium); Milatuzumab-dox (Actinium); Emab-SN-38 (Actinium); Naptumonmab estafenatox (Active Biotech); AFM13 (Affimed); AFM11 (Affimed); AGS-16C3F (Agensys); AGS-16M8F (Agensys); AGS-22ME (Agensys); AGS-15ME (Agensys); GS-67E (Agensys); ALXN6000 (samalizumab, Alexion); ALT-836 (Altor Bioscience); ALT-801 (Altor Bioscience); ALT-803 (Altor Bioscience); AMG780 (Amgen); AMG 228 (Amgen); AMG820 (Amgen); AMG172 (Amgen); AMG595 (Amgen); AMG110 (Amgen); AMG232 (adecatumumab, Amgen); AMG211 (Amgen/MedImmune); BAY20-10112 (Amgen/Bayer); Rilotumumab (Amgen); Denosumab (Amgen); AMP-514 (Amgen); MEDI575 (AZ/MedImmune); MEDI3617 (AZ/MedImmune); MEDI6383 (AZ/MedImmune); MEDI551 (AZ/MedImmune); Moxetumomab pasudotox (AZ/MedImmune); MEDI565 (AZ/MedImmune); MEDI0639 (AZ/MedImmune); MEDI0680 (AZ/MedImmune); MEDI562 (AZ/MedImmune); AV-380 (AVEO); AV203 (AVEO); AV299 (AVEO); BAY79-4620 (Bayer); Anetumab ravtansine (Bayer); vantictumab (Bayer); BAY94-9343 (Bayer); Sibrotuzumab (Boehringer Ingleheim); BI-836845 (Boehringer Ingleheim); B-701 (BioClin); BIIB015 (Biogen); Obinutuzumab (Biogen/Genentech); BI-505 (Bioinvent); BI-1206 (Bioinvent); TB-403 (Bioinvent); BT-062 (Biotest) BIL-010t (Biosceptre); MDX-1203 (BMS); MDX-1204 (BMS); Necitumumab (BMS); CAN-4 (Cantargia AB); CDX-011 (Celldex); CDX1401 (Celldex); CDX301 (Celldex); U3-1565 (Daiichi Sankyo); patritumab (Daiichi Sankyo); tigatuzumab (Daiichi Sankyo); nimotuzumab (Daiichi Sankyo); DS-8895 (Daiichi Sankyo); DS-8873 (Daiichi Sankyo); DS-5573 (Daiichi Sankyo); MORab-004 (Eisai); MORab-009 (Eisai); MORab-003 (Eisai); MORab-066 (Eisai); LY3012207 (Eli Lilly); LY2875358 (Eli Lilly); LY2812176 (Eli Lilly); LY3012217(Eli Lilly); LY2495655 (Eli Lilly); LY3012212 (Eli Lilly); LY3012211 (Eli Lilly); LY3009806 (Eli Lilly); cixutumumab (Eli Lilly); Flanvotumab (Eli Lilly); IMC-TR1 (Eli Lilly); Ramucirumab (Eli Lilly); Tabalumab (Eli Lilly); Zanolimumab (Emergent Biosolution); FG-3019 (FibroGen); FPA008 (Five Prime Therapeutics); FP-1039 (Five Prime Therapeutics); FPA144 (Five Prime Therapeutics); catumaxomab (Fresenius Biotech); IMAB362 (Ganymed); IMAB027 (Ganymed); HuMax-CD74 (Genmab); HuMax-TFADC (Genmab); GS-5745 (Gilead); GS-6624 (Gilead); OMP-21M18 (demcizumab, GSK); mapatumumab (GSK); IMGN289 (ImmunoGen); IMGN901 (ImmunoGen); IMGN853 (ImmunoGen); IMGN529 (ImmunoGen); IMMU-130 (Immunomedics); milatuzumab-dox (Immunomedics); IMMU-115 (Immunomedics); IMMU-132 (Immunomedics); IMMU-106 (Immunomedics); IMMU-102 (Immunomedics); Epratuzumab (Immunomedics); Clivatuzumab (Immunomedics); IPH41 (Innate Immunotherapeutics); Daratumumab (Janssen/Genmab); CNTO-95 (Intetumumab, Janssen); CNTO-328 (siltuximab, Janssen); KB004 (KaloBios); mogamulizumab (Kyowa Hakko Kirrin); KW-2871 (ecromeximab, Life Science); Sonepcizumab (Lpath); Margetuximab (Macrogenics); Enoblituzumab (Macrogenics); MGD006 (Macrogenics); MGF007 (Macrogenics); MK-0646 (dalotuzumab, Merck); MK-3475 (Merck); Sym004 (Symphogen/Merck Serono); DI17E6 (Merck Serono); MOR208 (Morphosys); MOR202 (Morphosys); Xmab5574 (Morphosys); BPC-1C (ensituximab, Precision Biologics); TAS266 (Novartis); LFA102 (Novartis); BHQ880 (Novartis/Morphosys); QGE031 (Novartis); HCD122 (lucatumumab, Novartis); LJM716 (Novartis); AT355 (Novartis); OMP-21M18 (Demcizumab, OncoMed); OMP52M51 (Oncomed/GSK); OMP-59R5 (Oncomed/GSK); vantictumab (Oncomed/Bayer); CMC-544 (inotuzumab ozogamicin, Pfizer); PF-03446962 (Pfizer); PF-04856884 (Pfizer); PSMA-ADC (Progenics); REGN1400 (Regeneron); REGN910 (nesvacumab, Regeneron/Sanofi); REGN421 (enoticumab, Regeneron/Sanofi); RG7221, RG7356, RG7155, RG7444, RG7116, RG7458, RG7598, RG7599, RG7600, RG7636, RG7450, RG7593, RG7596, DCDS3410A, RG7414 (parsatuzumab), RG7160 (imgatuzumab), RG7159 (obintuzumab), RG7686, RG3638 (onartuzumab), RG7597 (Roche/Genentech); SAR307746 (Sanofi); SAR566658 (Sanofi); SAR650984 (Sanofi); SAR153192 (Sanofi); SAR3419 (Sanofi); SAR256212 (Sanofi), SGN-LIV1A (lintuzumab, Seattle Genetics); SGN-CD33A (Seattle Genetics); SGN-75 (vorsetuzumab mafodotin, Seattle Genetics); SGN-19A (Seattle Genetics) SGN-CD70A (Seattle Genetics); SEA-CD40 (Seattle Genetics); ibritumomab tiuxetan (Spectrum); MLN0264 (Takeda); ganitumab (Takeda/Amgen); CEP-37250 (Teva); TB-403 (Thrombogenic); VB4-845 (Viventia); Xmab2512 (Xencor); Xmab5574 (Xencor); nimotuzumab (YM Biosciences); Carlumab (Janssen); NY-ESO TCR (Adaptimmune); MAGE-A-10 TCR (Adaptimmune); CTL019 (Novartis); JCAR015 (Juno Therapeutics); KTE-C19 CAR (Kite Pharma); UCART19 (Cellectis); BPX-401 (Bellicum Pharmaceuticals); BPX-601 (Bellicum Pharmaceuticals); ATTCK20 (Unum Therapeutics); CAR-NKG2D (Celyad); Onyx-015 (Onyx Pharmaceuticals); H101 (Shanghai Sunwaybio); DNX-2401 (DNAtrix); VCN-01 (VCN Biosciences); Colo-Ad1 (PsiOxus Therapeutics); ProstAtak (Advantagene); Oncos-102 (Oncos Therapeutics); CG0070 (Cold Genesys); Pexa-vac (JX-594, Jennerex Biotherapeutics); GL-ONC1 (Genelux); T-VEC (Amgen); G207 (Medigene); HF10 (Takara Bio); SEPREHVIR (HSV1716, Virttu Biologics); OrienX010 (OrienGene Biotechnology); Reolysin (Oncolytics Biotech); SVV-001 (Neotropix); Cacatak (CVA21, Viralytics); Alimta (Eli Lilly), cisplatin, oxaliplatin, irinotecan, folinic acid, methotrexate, cyclophosphamide, 5-fluorouracil, Zykadia (Novartis), Tafinlar (GSK), Xalkori (Pfizer), Iressa (AZ), Gilotrif (Boehringer Ingelheim), Tarceva (Astellas Pharma), Halaven (Eisai Pharma), Veliparib (Abbvie), AZD9291 (AZ), Alectinib (Chugai), LDK378 (Novartis), Genetespib (Synta Pharma), Tergenpumatucel-L (NewLink Genetics), GV1001 (Kael-GemVax), Tivantinib (ArQule); Cytoxan (BMS); Oncovin (Eli Lilly); Adriamycin (Pfizer); Gemzar (Eli Lilly); Xeloda (Roche); Ixempra (BMS); Abraxane (Celgene); Trelstar (Debiopharm); Taxotere (Sanofi); Nexavar (Bayer); IMMU-132 (Immunomedics); E7449 (Eisai); Thermodox (Celsion); Cometriq (Exellxis); Lonsurf (Taiho Pharmaceuticals); Camptosar (Pfizer); UFT (Taiho Pharmaceuticals); and TS-1 (Taiho Pharmaceuticals).

In one example, the subject in whom the strength of the immune response is to be modified is suffering from complement deficiency and the A20 variant administered to or expressed in the subject is an A20 variant set forth in Table 2 or a functional fragment, analog or derivative thereof as described herein.

In another example, the subject in whom the strength of the immune response is to be modified is suffering from an autoimmune disease and the A20 variant administered to or expressed in the subject is an A20 variant set forth in Table 3 or a functional fragment, analog or derivative thereof as described herein. Examples of autoimmune disease include, but are not limited to uveitis, multiple sclerosis; arthritis, such as rheumatoid arthritis, osteoarthritis, psoriatic arthritis, or juvenile idiopathic arthritis; neuromyelitis optica (Devic's disease); ankylosing spondylitis; spondyloarthritis; psoriasis; systemic lupus erythematosus; inflammatory bowel disease, such as Crohn's disease or ulcerative colitis; celiac disease; asthma, such as allergic asthma or neutrophilic asthma; chronic obstructive pulmonary disease (COPD); scleritis; vasculitis; Behcet's disease; atherosclerosis; atopic dermatitis; emphysema; periodontitis; allergic rhinitis; and allograft rejection.

In another example, the subject in whom the strength of the immune response is to be modified is suffering from a pathogenic infection or is in need or protection against pathogenic infection, and the A20 variant administered to or expressed in the subject is an A20 variant set forth in Table 2 or a functional fragment, analog or derivative thereof as described herein.. The infection may be caused by any pathogen, including bacteria, virus, fungus. Examples of bacterial, viral and fungal infectious agents include but are not limited to *Mycobacterium tuberculosis, Bordetella pertussis, Campylobacter jejuni, Chlamydia, Clostridium, Haemophilus, Neisseria, Pseudomonas, Salmonella, Shigella Staphylococcus, Streptococcus, Yersinia,* varicella-roster virus, measles virus, rotavirus, norovirus, Hepatitis virus, Herpes, HIV, Rubella virus, *Aspergillus, Candida, Coccidioides, Cryptococcus, Histoplasma, Pneumocystis,* and *Stachybotrys*

In another example, the subject in whom the strength of the immune response is to be modified is a transplant patient, such as for example, a subject who has undergone tissue or organ transplantation or is about to undergo tissue or organ transplantation. In accordance with this example, the A20 variant administered to or expressed in the subject is an A20 variant set forth in Table 3 or a functional fragment, analog or derivative thereof as described herein.

In accordance with any example in which an A20 variant, or functional fragment, analog or derivative thereof, as described herein is to be expressed in or administered to a subject as part of a combination therapy *i.e.,* in conjunction with one or more other therapeutic agents or treatments as described herein, the A20 variant, or functional fragment, analog or derivative thereof, may be provided to the subject simultaneously or consecutive to the one or more other therapeutic agents or treatments.

### Diagnostic/Prognostic methods

Based on the finding that different A20 variants of the disclosure correlate with differing NF-κB and/or JNK signalling activity, the inventors also contemplate that the A20 SNV variants could be useful as biomarkers to stratify patients according to the likely strength of their immune response before undergoing treatment or during treatment. Such information may also be useful in selecting the type of and/or duration of treatment, and for ongoing patient management.

Accordingly, in one example, the present disclosure provides a method of stratifying a subject according to the strength of their immune response. In one example, the method of stratifying the subject comprises: (i) determining the nucleotide sequence of the A20 gene in the subject; (ii) comparing the nucleotide sequence of the A20 gene determined at (i) with a reference A20 gene nucleotide sequence; and (iii) predicting the strength of the subject's immune response on the basis of the subject's A20 gene nucleotide sequence being identical to the reference sequence at one or more nucleotide positions or on the basis of one or more differences in the nucleotide sequence of the A20 gene in the subject relative to the reference sequence. For example, the presence of an A20 SNV in the subject relative to the reference A20 nucleotide sequence e.g., an SNV encoding an A20 variant set forth in Table 1, will be indicative that the subject's immune response will be increased or decreased relative to if the subject had the reference A20 nucleotide sequence. In one example, the presence of an A20 SNV corresponding to an A20 variant set forth in Table 2 in the subject is indicative that the subject's immune response will be increased relative to if the subject had the reference A20 nucleotide sequence. Conversely, the presence of an A20 SNV corresponding to an A20 variant set forth in Table 3 in the subject is indicative that the subject's immune response will be decreased relative to if that subject had the reference A20 nucleotide sequence. As described herein, the reference A20 nucleotide sequence against which the subject's A20 nucleotide sequence is compared may be a sequence encoding a wild-type A20 protein *e.g.,* a wild-type human A20 protein. The nucleotide sequence of the A20 gene, *TNFAIP3,* in the subject may be determined by any suitable sequencing methods known in the art. For example, the A20 nucleotide sequence may be determined by Sanger sequencing or high-throughput sequencing methods such as next-gen sequencing. The subject's A20 nucleotide sequence may be compared to the reference A20 nucleotide sequence by any suitable method known in the art. For example, the subject's A20 nucleotide sequence may be aligned (using any suitable sequence alignment or bioinformatics software) with the reference A20 gene sequence to identify differences between the subject's A20 nucleotide sequence and the reference sequence. It may also be readily determined whether any differences in nucleotide sequence translate into differences at the amino acid level *i.e.,* whether SNVs are synonymous or non-synonymous. The method of stratifying a subject according to the strength of their immune response may be performed prior to administering to, or expressing in, the subject an A20 variant which modifies the immune response e.g., prior to performing a method of modifying the strength of an immune response in a subject as described herein, in order to determine whether the strength of the subject's immune response needs to be increased or decreased.

It will be appreciated by the skilled artisan that the methods of the present disclosure allows the stratification of a subject in order to be treated by the methods of treatment of the present disclosure.

### Examples

### Example 1 - The A20 locus contains benign and deleterious variants

Using available human genome sequence data from the ExAC database, the inventors utilised PolyPhen2 to determine the distribution of non-synonymous single nucleotide variations (nsSNVs) classified as benign or deleterious within the A20 locus. The results from this analyses are expressed in violin plot format (Figure 1a). For comparison the inventors conducted the same analysis for the mouse and rat A20 locus and also for a hypothetical human A20 locus (build GRCh38) in which every single possible base pair was altered by a computer algorithm and scored according to the PolyPhen2. When compared to the A20 mouse-rat locus, the human genome has maintained a large burden of A20 variants classified as deleterious (top of the violin plots).

The inventors applied the same kind of analysis on nsSNVs across the entire A20 locus, but now focusing on human A20 protein coding domains (functional and structurally connecting). Three main groupings emerged as is illustrated in Figure 1B. The three groups segregated depending on the enrichment of functional consequences spectrum of nsSNVs: one group constituted by almost all benign variants, a second group with almost all deleterious variants and a group of more evenly distributed nsSNVs consequences. Interestingly, functional domains (e.g. OTU and Zinc Fingers domains) were mostly found within the group with deleterious variants, while the structurally connecting domains between the OTU and Zinc fingers followed the opposite trend, a decrease in predicted to be deleterious variants.

### Example 2 - Bioinformatics analysis of the human A20 locus to identify A20 coding variants

The A20 gene locus is characterized by many SNVs identified not only by GWAS or candidate-gene studies, but also by extensive sequencing studies contained within meta libraries like the ExAC database. The inventors combined variants from these various sources and analysed them by PolyPhen2, revealing a total of ~262 rare missense protein coding SNPs in the OTU domain only.

The inventors also used bioinformatics resources to mine available online resources to make use of all the information on A20's genetic variability. The literature was also consulted for the most up to date variants in the A20 gene locus found through GWAS, NGS and WGS studies. Online databases like Ensembl.org were also extensively consulted for this purpose. For the identification of A20 gene variants identified through GWAS, databases like GWAS central and GWAS catalog were consulted. For a more extensive, non-GWAS centred, consultation of A20 variants, Ensembl online database was consulted. Ensembl is an aggregator of sources of genes information from literature, clinical observations and several other online databases (ExAC, gnomAD, dbSNP, SNPedia, 1000genomes, HapMap). Furthermore, Ensembl provides, where possible, inferences on the deleteriousness of variants through the addition of prediction algorithms information regarding coding variants. In addition to these sources of gene variation, the inventors obtained exome sequencing data from 50 patients with type 1 diabetes (T1D) sequenced at the Garvan Institute of Medical Research. From this analysis, 17,521 variants in *TNFAIP3* gene locus were identified.

Given the difficulty and cost associated with functionally evaluating such a large number of SNVs, a series of filters was applied to reduce the working set of human A20 variants to functionally test (Figure 2). The variants in the *TNFAIP3* gene locus were filtered according to the following characteristics:
- Coding variants
- Rare (<0.1% MAF)
- Non-synonymous
- Predicted to be deleterious by PolyPhen2 (≥ 0.85)

Since the purpose of the functional testing was to assess the impact of A20 variation on protein function, non-coding variants of the A20 gene locus were discarded as a first filter. This 'coding' filter reduced the number of variants to 4580. A further filter based on the frequency of the variants in the population was then applied. In this case rare (<0.1% MAF) SNV were prioritised over common SNV. Rarity would allow the exploration of variants not detectable by GWAS, examining the hypothesis by which complex diseases might be explained by more rare variants in the population. This filter reduced the number of variants to 325.

Variants were then selected only if the point mutation caused a non-synonymous change in the protein sequence, filtering out the redundancy of variants coding for the same amino acid through the different nucleotide triplets. Using this filter, the number of variants was reduced to 205.

Lastly, PolyPhen2 was used to segregate variants that might have an effect on the protein function. PolyPhen2 served two purposes: the first was that it provided an unbiased method to select variants for functional analysis; and the second was that PolyPhen2 would intrinsically test the algorithm's success in correctly determining the functional consequence of a given SNV.

By applying this pipeline, 205 coding variants with a PolyPhen2 score were identified, collected and subsequently plotted against the A20 protein sequence. This offered a visual representation of the distribution of SNV across the A20 protein sequence, as well as a view of the range of functional consequences (benign 0 - 0.15, damaging > 0.15, 0.85, deleterious ≥ 0.85) variants predicted by PolyPhen2 (Figure 3a). This distribution of variants can be filtered with increasing stringency to try enriching the population of variants with real functional consequences *in vitro*/*in vivo.* Filtering out variants which were predicted to be benign (PP2 score < 0.15) highlights how PolyPhen2 scores the functional consequences of an amino acidic change. Variants with a benign score were mostly located in connecting domains, regions of the protein with low evolutionary conservation, which are more prone to amino acid changes tolerance (Figure 3b). Variants with a damaging score (PP2 score ≥ 0.85) polarized in fewer areas (Figure 3c), mostly where functional domains have been predicted. Variants were evenly distributed across the OTU domain and more concentrated around the last four zinc fingers domains. This polarization of variants which are predicted to be damaging may be explained by the fact that these regions are involved in A20's double enzymatic activity (deubiquitination and E3 ubiquitin ligase activity) (Wertz, O'Rourke et al. 2004, Komander and Barford 2008, Shembade, Parvatiyar et al. 2009, Lu, Onizawa et al. 2013, Draber, Kupka et al. 2015, Wertz, Newton et al. 2015).

Together with the previously applied filters shown in Figure 2, a final group of 10 variants emerged that were protein-coding, rare, non-synonymous and predicted to be damaging by PolyPhen2. As shown in Figure 3d, these SNVs were evenly-distributed across the different functional protein domains of A20.

In addition to this set of 10 A20 SNVs, a further A20 variants were chosen for functional evaluation based on the following criteria:
- Rare-benign variants;
- Biochemically defined loss of function (LoF) variants;
- GWAS associated variants;
- A20 variants identified by whole genome sequencing studies; and
- ENU variant with an *in vivo* mouse phenotype.

These criteria a described in further detail below. This selection process brought the total number of variants tested in this study to 25. The 25 SNVs are presented in Table 4 below.

### Rare-benign variants

A group of six SNV that were coding, rare, non-synonymous, but predicted to be benign by PolyPhen2 (score < 0.15) were chosen to compare with the set of rare-deleterious SNV. As well as providing important control data (as these were predicted to have no impact on function), these SNVs tested the PP2 algorithm for errors in predicting false negatives.

### Biochemically defined loss of function (LoF) variants

To draw the upper range of NF-κB activation across the different A20 SNV, a second group comprising two loss-of-function mutants was chosen, namely C103A and S381A. Given previous evidence that these sites may have a role on A20 activity and function, it was predicted that these mutants should define the maximal upper range of responses for any SNV that reduces A20's function in the NF-κB reporter assay.

### GWAS associated variants

A further group of variants includes the only two GWAS variants identified within A20's coding region, namely, A125V and F127C (Musone, Taylor et al. 2008, Lodolce, Kolodziej et al. 2010).

### A20 variants identified by whole genome sequencing studies.

Also included in the panel of variants tested in functional assays were three variants from a cohort of 50 patients with T1D whose exomes had been sequenced. These three variants, namely T108A, I207L and T647P, fulfil the criteria of rarity (MAF < 0.1%) but were predicted to be benign by PolyPhen2 (scores 0.001, 0.132 and 0.000 respectively).

### ENU variant with an in vivo mouse phenotype

The inventors also identified the I325N variant through an *in vivo* ENU-mutagenesis mouse screen for A20 LoF variants. This mutation changes a highly conserved isoleucine in the A20 OTU domain for an asparagine. The I325N mouse model showed a hyper-inflammatory phenotype including an increased frequency of activated T cells, spontaneous immune infiltrates in various organs, and when challenged with LPS these mice suffered premature death. For this study, the I325N variant was introduced into the A20 reference sequence by site-directed mutagenesis. Including this mutant in the panel for functional testing provided a benchmark to highlight whether the variant's effect on NF-κB inhibition could translate into an *in vivo* phenotype.

It was hypothesised that transfecting cells with human A20 variants would affect their ability to inhibit NF-κB upon hTNFα stimulation. Therefore, filtering variants for rarity and deleteriousness was undertaken in order to enrich for variants with an impaired NF-κB inhibitory activity. Variant choice would also serve another purpose, shedding some light on the current complex disease-variants hypothesis. If rare variants were found to confer an *in vitro* NF-κB phenotype, then there may be value in investigating the role of rare variants in complex diseases.

**Table 4 - A20 variants taken forward into functional assays**

| Row | Variant ID | bp change | rs number* | Genome coordinates | Functional Impact** | PolyPhen2 Prediction*** |
|---|---|---|---|---|---|---|
| 1 | R22Q | G/A | rs199876928 | 6:138192429 | none | damaging |
| 2 | S79R | C/G | rs148028402 | 6:138192601 | none | benign |
| 3 | N102S | A/G | rs146534657 | 6:138195991 | LoF | damaging |
| 4 | C103A^ | n/a | n/a | n/a | None | damaging |
| 5 | T108A | A/G | rs376205580 | 6:138196008 | none | benign |
| 6 | V115I | G/A | rs150717698 | 6:138196029 | GoF | benign |
| 7 | A125V | C/T | rs5029941 | 6:138196060 | none | damaging |
| 8 | F127C | T/G | rs2230926 | 6:138196066 | none | benign |
| 9 | R162Q | G/A | rs144788826 | 6:138196171 | GoF | damaging |
| 10 | S184N | G/A | rs147932545 | 6:138196889 | GoF | benign |
| 11 | I207L † | A/C | rs141807543 | 6:138196957 | LoF | benign |
| 12 | C243Y# | G/A | COSM5714695ϕ | 6:137876089 | LoF | damaging |
| 13 | S254N ¥¥ | G/A | rs61756235 | 6: 138238952 | LoF | benign |
| 14 | S254R | C/A | rs1350749287 | 6:138197260 | n.d. | damaging |
| 15 | K301N | G/T | n.a. | 6:137877173 | LoF | damaging |
| 16 | L307V | T/G | rs145745293 | 6:138198326 | none | damaging |
| 17 | I325N *f* | n.a. | n.a. | 6:138198380 | LoF | damaging |
| 18 | S381A^^ | n/a | n/a | n/a | LoF | damaging |
| 19 | V398A | T/C | rs140616865 | 6:138199775 | none | damaging |
| 20 | S466R | C/G | rs200878487 | 6:138199980 | none | benign |
| 21 | T604M | C/G | rs564646155 | 6:138200393 | none | benign |
| 22 | T647P | A/C | rs142253225 | 6:138201240 | none | benign |
| 23 | R706Q ¥¥ | G/A | rs3734553 | 6-138202200 | LoF | benign |
| 24 | R749W | G/A | rs568826568 | 6:138202328 | none | damaging |
| 25 | R763Q | G/A | rs369621216 | 6:138202371 | LoF | damaging |
| 26 | A766T | G/A | rs5029957 | 6:138202379 | LoF | damaging |
| 27 | A766P¥ | G/C | rs5029957 | 6: 138244072 | LoF | damaging |

| | | | | | | |
|---|---|---|---|---|---|---|
| * rs numbers, coordinates and frequencies have been collected from gnomAD, which uses GRCh37/h19 ** Functional Impact = tested in experimental studies. GoF, Gain of function or "cold" variant; LoF Loss of Function or "hot" variant; none, no functional change *** PolyPhen2.0 gene prediction algorithm; SNV marked as 'damaging' achieved a PolyPhen2.0 score of ≥0.8 ^C103A is an engineered alanine substitution of the catalytic cysteine at position 103 predicted to be damaging. However, C103A does not impact A20's NFkB inhibitory function highlighting the novelty of our findings. ϕCOSMID numbers have been collected from ensembl.org, which uses genome build GRCh38 # C243Y is an A20 loss of function mutation identified in a family with heriditary inflammatory disease (REF - PMID: 27175295) ^^S381A is an engineered alanine substitution of serine 381 resulting in a loss of function. S381A was included in our assay to calibrate the sensitivity of our assays to detect A20 loss of function variants (REF PMID: 17709380). *f* I325N is a loss of function A20 mutant that we show confers protective immunity in rodent models of infection † SNV change occurs in both Denisovan hominins and anatomically modern humans ¥ SNV identified in Neandertal hominins ¥¥ SNV change occurs in both Neanderthal hominins and anatomically modern humans | | | | | | |

### Example 3 - Testing A20 variants for their effect in vitro

The next step was to test the A20 variants identified in Example 2 using a multi-model approach to elucidate the role of A20 in the context of human inflammatory diseases.

The impact of the 27 different sequence variants on A20's inhibitory function against NF-κB was tested in an NF-κB-luciferase reporter assay which is recognised as being a standard and robust approach for assessing the effect of A20 on NF-κB inhibition. Essentially, cells expressing a NFkB reporter are treated with TNF to activate the reporter. Reporter activation is then measured by analysing luciferase values. In some cases, cells also express A20, or one of the 27 A20 variants, and NFkB activation is measured after TNF stimulation. Thus, this assay requires transient transfection of mammalian cells with plasmids encoding:
- NF-κB luciferase (expressing luciferase driven by regulatory elements of NF-κB);
- β-galactosidase reporter (to correct for transfection efficiency); and
- PCDNA3 expression vector encoding the empty vector (control), or the reference human-A20 sequence or one of 21 human-A20 variants.

### Luciferase reporter assay in HEK293T cells

The inventors conducted the luciferase reporter assay in HEK293T cells. This undifferentiated cell line was chosen because it is a human cell, originally derived from cultures of human embryonic kidney cells, and its widely reported propensity to transfection. The human derivation of this cell line makes it appropriate to test the function of human A20 variants. Also, the non-specialized nature of this cell line offered a 'neutral' environment for the testing of A20's variants.

Briefly, Lipofectamine 2000 was used to transiently transfect all cells (HEK293T) with NF-κB luciferase plasmid and β-gal reporter to correct for transfection efficiency. Selected wells were also transiently transfected with either an empty PCDNA3 plasmid (serving as non-template control) or human A20 plasmids coding for either the reference or the variants. Cells were stimulated with human-TNFα for 8 hours to stimulate the reporter, at the end of which cell lysates were tested for luciferase and β-galactosidase activity (luminescence) in technical duplicates and the luminescence values were recorded for each of the tested variants (± hTNFα stimulation) (Figure 4). Each individual variant tested was generated through site-directed mutagenesis of the reference human-A20 encoded in PCDNA3, subsequently transformed into competent *E. coli* competent cells, expanded, mini-prepped and finally the sequence and mutation was confirmed through Sanger sequencing

A representative read-out of the assay can be found in Figure 5a. Unstimulated cells transfected with an empty PCDNA3 vector showed basal NF-κB luciferase levels (shown as NF-κB relative units ± standard deviation) with a mean of 132.1 ± 34.41. NF-κB luciferase levels were increased ~4-fold when cells were subjected to 8 hours of hTNFα stimulation (mean 415.3 ± 90.9). In contrast, cells transfected with the reference human-A20 gene report basal NF-κB luciferase levels ~12-folds less than control unstimulated PCDNA transfected cells (mean 11.11 ± 1.715). When stimulated with hTNFα, cells expressing reference human-A20 showed almost complete suppression of NF-κB is luciferase activity when compared to NF-κB luciferase levels of non-template control (NTC) transfected cells. Thus, this data demonstrates the well-described function of A20 to suppress TNF-induced NFkB activation, but also demonstrates the principles of the screening assay, also represented through a hA20 protein expression detection through western blot (Figure 5b).

Results from cells expressing different A20 variants are also presented (Figure 5a). It was noted that cell expressing different A20 variants showed different basal and stimulated NF-κB levels, indicating different effects on A20's normal NF-κB suppressive activity. Some variants exhibited an impaired ability to inhibit TNF-induced NF-κB activity: C243Y (4.477 ± 0.7629), (237.8 ± 44.76) + hTNFα; S381A (11.38 ± 6.758), (380 ± 56.42) + hTNFα; N102S (45.31 ± 5.37), (458 ± 43.47) + hTNFa. Other variants suppressed NF-κB to levels comparable to the reference human-A20: C103A (8.468 ± 2.13), (86.77 ± 47.2) + hTNFα; I325N (4.46 ± 0.4933), (101.9 ± 32.07) + hTNFα; S184N (4.883 ± 0.1174), (92.11 ± 37.21) + hTNFα. Fold changes for the following variants were: hA20-C243Y (~60-fold), hA20-S381A (~82-folds), hA20-N102S (~10-folds, but with basal levels already 3-folds higher than ref-hA20), hA20-C103A (~11-folds), hA20-I325N (25-folds) and hA20-S184N (~20-folds). The differences between ref-hA20+ and C243Y+, ref-hA20+ and S381A+, ref-hA20+ and N102S+ were statistically significant; (**P = 0.0079, **P* = 0.0357 and **P = 0.0079 respectively).

To better facilitate a comparative analysis across the 27 variants, normalization of the data was performed by averaging the technical replicates from each experiment and then scoring each tested variant against each individual experiment's NTC (hTNFα stimulated), the TNF-stimulated NTC result was set to a value of 1, and the reference human-A20 (hTNFα stimulated) result was set to a value of 0. This method guaranteed self-contained consistency of results and allowed for comparability across experiments, increasing the robustness of the approach itself.

The variants reported in the representative bar graph (Figure 5) showed an induction in NF-κB activity upon hTNFα stimulation. The HEK293T cells exhibited 12 to 10-fold less basal NF-κB levels in ref-hA20 transfected cells in the steady state compared to NTC transfected cells in the steady state. The HEK293T cells also exhibited the ability of the A20 variants to suppress NF-κB, whether their activity was comparable to the reference human-A20 (i.e. S184N) or impaired (i.e. C243Y, S381A, N102S).

To better facilitate a comparative analysis across the 27 variants, normalization of the data was performed by averaging the technical replicates from each experiment and then scoring each tested variant against each individual experiment's NTC (hTNFα stimulated), the TNF-stimulated NTC result was set to a value of 1, and the reference human-A20 (hTNFα stimulated) result was set to a value of 0. This method guaranteed self-contained consistency of results and allowed for comparability across experiments, increasing the robustness of the approach itself.

### Observations

The relationship between normalized NF-κB activity of the A20 variants (left y-axis) and their predicted deleteriousness (right y-axis), reported as PolyPhen2 score (data from PolyPhen2 build September 2014), in HEK293T cells is illustrated in Figure 6. On the x-axis the amino acid number of each human A20 variant tested (as they appear in the human-A20 protein sequence) is shown. Through normalization of the NF-κB luciferase activity data it was possible to identify three groups of variants with diverging NF-κB inhibitory activity. The majority of the variants, including variants at positions 22, 79, 103, 108, 125, 127, 307, 398, 466, 604, 647 and 749, showed NF-κB inhibitory activity that did not differ from the reference human-A20 indicating a normal inhibitory activity. A second group of hA20 variants, specifically 115, 162 and 184, showed an enhanced capacity to suppress NF-κB in the luciferase reporter assay when compared to reference hA20. A third group of hA20 variants including 102, 207, 243, 254, 301, 325, 381, 706, 763 and 766 showed an increasing loss of NF-κB inhibitory activity. This was the group with the most dramatic changes, including variants exhibiting only a subtle loss of NF-κB inhibitory activity, barely different to reference human-A20 (i.e. I325N, I207L), but also including variants whose function was severely affected (i.e. S381A, N102S who showed a >50% loss of function when compared to ref-hA20 and NTC transfected cells). In conclusion, these data demonstrate for the first time that different A20 variants can alter function of A20 with regards to the control of NF-κB, and do so in a graduated manner. Further, these data also support the inventors' primary assumption that rare variants in A20 negatively impact its function and that these Loss of Function (LoF) variants could potentially play a role in human disease.

The inventors then compared the functional data with the predictions of PolyPhen2 by plotting the PolyPhen2 scores for each variant against their respective NF-κB activity. Interestingly, hA20 variants with an impaired ability to inhibit NF-κB did not always report concordant deleterious (≥0.85) PolyPhen2 scores. Vice versa, hA20 variants with NF-κB inhibitory activity which is comparable or better to the reference human-A20 did not always report concordant benign (<0.15) PolyPhen2 scores. The relationship between normalized NF-κB activity of the A20 variants (left y-axis) and their predicted deleteriousness (right y-axis), reported as PolyPhen2 score (data from September 2014), in HEK293T cells is shown in Figure 6. The x-axis shows the amino acid number of each human A20 variant as the amino acids appear in the human-A20 protein sequence.

Variants were then re-organized from Figure 6a to generate a relative heatmap (Figure 6b), mapping variants with the highest NF-κB inhibitory activity (on the left) to the most the impaired variants (on the right)(Figure 7). A gradient of inhibitory activity can be seen among the variants evaluated. The normalized activation of NF-κB in HEK293T cells presents, for most of the variants, very little variation among each individual experiment (Figure 7). This is evident in the formation of three easily identifiable groups of variants with better, similar and worse NF-κB inhibitory activity (similar to the earlier observations). In this case the reference human-A20 (value of 0) was positioned left to a set of ten variants, some of which are known to have an effect *in vivo* (i.e. I325N, C243Y). Interestingly, hA20 variant N102S scored even higher than the NTC (value of 1). This suggests not only the complete inability of this variant to suppress NF-κB, but also a potential for this variant to interfere with the activity of the endogenous A20 of these cells.

As is apparent from Figure 8, the variants are distributed in order of their ability to suppress NF-κB and statistical significance. The same three groups of variants previously identified also emerge in Figure 8, with the majority of variants not diverging a great deal from the reference A20 (in green, statistically not significant). Gain of Function (GoF) variants weakly diverge from the reference baseline (in orange, *P-value* ranging from 0.001 to 0.05). This trend may be explained by the additive effect of GoF variants on NF-κB inhibition to the inhibitory function of the endogenous A20 in the cells. Impaired variants were the most statistically significant diverging group (in red, *P-value* < 0.0001). The greater magnitude of effect observed for this group of variants highlights their impact on activity on NF-κB expression, even in the presence of endogenous A20. Taken together, this data show how this experimental setup was skewed towards the detection of dominant-negative variants.

Interestingly, all transfected hA20 variants expressed in comparable levels to the ref-hA20 except for the N102S (Figure 9). The latter had similar levels to the PCDNA transfected cells, inferring that the protein was either not expressed or degraded.

### Example 4 - JNK signalling

TNF is one of the cytokines that activates the NF-κB pathway, which in turn promotes the transcription of pro-inflammatory genes. There are also other molecular actors involved in the inflammatory response that are triggered by TNF. One of these is c-Jun N-terminal kinase (JNK), which belongs to the mitogen-activated protein kinases (MAPKs) family. While NF-κB's function is anti-apoptotic and cyto-protective, JNK's signalling pathway is involved in the mediation of apoptotic cell death in response to a variety of stress stimuli, such as cytokines, ultraviolet irradiation and changes in levels of reactive oxygen species.

When stimulated with TNF, cells can choose which fate to follow, either favouring a cell-protective fate (NF-κB pathway) or cell-death via the JNK pathway. These findings suggest that there are cell-intrinsic mechanisms that are able to discern which fate cells undergo. Studies have identified how the adaptor protein TRAF2 couples the membrane receptor to JNK, but the dynamics of its activation suggest a more complex network than the classic linear kinase cascade.

A20 fulfils both anti-inflammatory and anti-apoptotic roles and it is involved in both NF-κB and JNK pathways, although its inhibitory mechanisms in the latter pathway are unclear. Therefore, to complement the results obtained on NF-κB levels, the inventors used a cell-based assay to dynamically assess the impact of each hA20 variant on JNK activation upon hTNFα stimulation.

For this assay an mCherry Kinase Translocation Reporter HEK293T cell line was used. This cell line constitutively expresses the latter construct, which translocates from the nucleus to the cytoplasm when phosphorylated by JNK (Figures 10 and 11). Using a high-throughput imaging assay based on automated imaging (Thermo Scientific Cellomics Arrayscan machine) to look at single cells, it was possible to investigate JNK activity by measuring the ratio between mCherry present in the nucleus and the cytoplasm of each imaged cell.

The inventors transiently infected HEK293T cells with human-A20 variants using JetPRIME reagent. Briefly, selected wells were transiently transfected with either an empty PCDNA3 plasmid (serving as non-template control) or human A20 plasmids coding for either the reference or the variants. A nuclear localization marker for JNK was given to the cells and then selected wells were stimulated with human-TNFα. Each well of transfected cells was assessed every 5 min for 1 hour. The ratio between cytoplasm JNK and nucleus JNK would be a measure of JNK activation upon hTNFα stimulation. Figure 10 includes a schematic showing a representative view of one of the 25 fields captured by the Thermo Scientific Cellomics Arrayscan machine. The white arrows point at cells where the nuclear marker is either still in the nucleus (left) or has translocated into the cytoplasm (right). The high number (minimum of ~150k cells per well up to ~350k cells) of individual cells measured in each assay, in duplicates and repeated a minimum of two times, returned population behaviour of JNK activation kinetic upon hTNFα stimulation for a period of 60 minutes (5 minutes intervals). A representative image of JNK mCherry marker activation in HEK293T cells transfected with PCDNA and reference-humanA20 is provided in Figure 11. Cells snapshot taken at time 0, prior hTNFα stimulation, and at 20 minutes post-hTNFα stimulation, where PCDNA transfected cells reach their maximum peak of JNK activation.

In Figure 12 a representative experiment shows the dynamics of JNK activation upon hTNFα stimulation. Again, JetPrime reagent was used to transiently transfect all HEK293T cell with either NTC or hA20 (reference or selected variants). As is apparent from Figure 12, non-transfected and NTC transfected cells have similar trends with the highest peak occurring at 20 minutes post-stimulation (Mean ± standard error (SE) = 1.053677 ± 0.020137 and 1.015129 ± 0.01626 respectively). Reference human-A20 transfected cells still show an induction after hTNFα stimulation but JNK activity plateaus after 10 minutes, and the recorded mean ± SE value at 20 minutes post-stimulation was 0.782451 ± 0.010964. A20 variants with reported impairing effects in previous assays displayed a similar trend in this assay too. Mean ± SE of this group of variants were the following: C243Y (0.85324 ± 0.01716), S381A (0.86703 ± 0.01228) and N102S (1.05010 ± 0.01864). A20 variants reporting ref-hA20 comparing function also displayed a similar trend in this assay. Mean ± SE of this group of variants were the following: C103A (0.77158 ± 0.03143), I325N (0.73734 ± 0.01214) and S184N (0.68184 ± 0.02260).

After the A20 variants were tested in this assay, values were normalized to be able to rank the variants based on their ability to suppress JNK activity at the 20 minute time point. The normalized activation of JNK in cells have been represented as a heatmap in Figure 13, which shows the NTC transfected cells peaking at 20 minutes (Mean ± SE = 1.49119 ± 0.05318) followed by a lower-plateaued JNK activity for the rest of the assay (Mean ± SE = 1.28400 ± 0.01637). Reference human-A20 exerts a stronger inhibitory function on JNK activity when compared to the NTC transfected cells. This translated in a reduction of the JNK activation peak at 20 minutes (Mean ± SE = 1.133136 ± 0.008768) to the basal levels displayed throughout the duration of the experiment (Mean ± SE = 1.120043 ± 0.017716). A20 variants at the lower end of the JNK activation ranking (e.g. V398A) displayed even lower trends than ref-hA20 throughout the experiment, therefore reporting a better JNK inhibitory function than the rest of the variants tested. A20-N102S instead places itself at the other end of this heatmap with values that surpass NTC transfected cells. This fundamental impairment of this variant's function is carried on in this assay highlighting the dominant-negative nature of this A20 variant.

### Ability of A20 variants to inhibit JNK

When considering the results obtained for the HEK293T cells in Example 3 for the dynamic inhibition of JNK although variants were ranked by their normalized JNK activity values at 20 minutes post-stimulation it is important to notice how some variants allow JNK hyper activation through time. The physiological kinetic of non-transfected, and NTC transfected, cells shown in Figure 12 shows JNK activation peak at 20 minutes and the subsequent decrease and plateauing of its activity past that time point. Some of the tested A20 variants do not display the latter kinetic (e.g. C103A, V115L, T647P), instead JNK activation for this variants increases through time.

When considering a static snapshot of the variants ranked by their JNK activity values at 20 minutes it was possible to observe a polarization of the variants in respect to the ref-hA20. The previous NF-κB luciferase reporter assay measured the transcriptional responses accumulated after TNF stimulation (8 hours post-stimulation) while this assay reported real-time dynamic activation of JNK over a period of 60 minutes. Although there is a fundamental difference in the way each of the functional assay tests A20 effect on NF-κB or JNK, some of the most impaired variants (e.g. N102S, C243Y) reconfirmed their impairing nature in this assay, adding a dynamic aspect to the screening of human-A20 variants. Interestingly, the GoF variants from the NF-κB assay did not score less than the ref-hA20, one of which (V115L) was ranked 4^{th} least protective. This might suggest a different role for these variants depending on their involvement according to the structural conformations the A20 protein needs to assume through time and across different pathways and molecular actors involved.

### Example 5 - Correlation between functional results and polyphen2 predictions

### Normalized NF-κB. luc scores and PolyPhen2 predictions

The inventors used the PolyPhen2 prediction algorithm as one of the filters applied to the final selection of the variants tested in this study. The *in vitro* functional consequences of amino acidic changes for each variant were then compared to PolyPhen2 predictions. This analysis was conducted on the HEK293T data due to the clarity of the subdivisions between variants' effects. Variants' normalized NF-κB luciferase values were ordered based on their ability to suppress NF-κB. This allowed for the identification of three groups of variants in the HEK293T cells: variants with GoF activity (highlighted in blue and ranging from -0.162 to - 0.085), ref-hA20 comparable variants (highlighted in green and ranging from -0.018 to - 0.0012) and variants with an impairing NF-κB inhibitory activity (highlighted in red and ranging from 0.005 to 1.105) (Figure 14)..

Each variant was checked for conservation in the *TNFAIP3* gene of 7 species (human, chimpanzee, cow, pig, dog, mouse, chicken and zebrafish) and an asterisk used (in Figure 14) to indicate each species in which the respective variant was conserved. The rarity of each variant in the general population was reported as a YES (if MAF < 0.1%), NO (if MAF ≥ 0.1%) or N/A, and this was based on the frequencies collected from gnomAD database. PolyPhen2 scores were also reported in green if below 0.85 (benign or possibly damaging consequences) and in red (predicted to be deleterious) if equal or above 0.85. When comparing NF-κB suppression ability of each variant and their relative conservation no obvious correlation was observed. On the other hand, PolyPhen2 scores ≥ 0.85 perfectly correlated with the rarity of variants in the general population, indicating part of the logic behind the call for deleteriousness of a variant through this algorithm.

The GoF group included two common variants predicted to be benign (S184N and V115L) and a rare variant predicted to be deleterious (R162Q), and their normalized NF-κB luciferase activity values. The ref-hA20 comparable group included most of the variants tested in this study (11 variants), yet no obvious correlation between conservation and effect emerged. The impairing variants (9 variants) were overall the most conserved among species and the most rarely observed in the general population (this group also included I325N and S381A variants, which have never been observed in the general population, most likely due to their LoF effect).

### Concordance between prediction algorithms and actual functional impact of variants

One of the main challenges with the ever so increasing amount of variants discovered in the human genome is how to functionally annotate them. Being able to discern between what is disease causal and what is not will ultimately result in a better understanding of how the human genome works.

In this study, one of the filters used by the inventors to possibly enrich for the discovery of deleterious variants was using PolyPhen2 prediction algorithm. Aside from PolyPhen2, many other algorithms have been developed to help predict the functional effect of a protein non-synonymous missense mutation (Butkiewicz and Bush 2001, Boyko, Williamson et al. 2008, Di, Chan et al. 2009, Neale, Rivas et al. 2011, Adzhubei, Jordan et al. 2013, Itan and Casanova 2015, Miosge, Field et al. 2015), yet, their ability to correctly predict functional consequences is still at best ~50% (Di, Chan et al. 2009, Miosge, Field et al. 2015). As new prediction algorithms are implemented it is interesting to see what is the concordance they display when compared to functional data and how these predictions change through time. In this section, the concordance between the functional results of the *in vitro* testing of the variants and PolyPhen2 predictions is explored.

The PolyPhen2 algorithm has been updated three times since the beginning of this study (September 2014, April 2017 and July 2017) but for consistency of results, variants were 'locked' with PolyPhen2 scores based on the September 2014 release. These PolyPhen2 predictions have been compared to predictions from other two extensively used online prediction tools, namely PROVEAN and SIFT, collected at the same time. These latter two prediction algorithms also assess the deleteriousness of an amino acidic substitution in a similar way to PolyPhen2, but have different degrees of strictness (Ng and Henikoff 2003, Choi and Chan 2015).

The A20 variants tested were then ordered according to their NF-κB inhibitory activity (Figure 15), with variants having the highest NF-κB inhibitory activity ranked at the top of Figure 15 and A20 variants exhibiting the most impaired NF-κB inhibitory activity ranked at the bottom. Briefly, each A20 variant was compared to the predictions of three deleteriousness assessing algorithms: PROVEAN, SIFT and PolyPhen2. Concordance between the prediction algorithms and functional effect was recorded if the algorithms predicted a variant to have neutral, tolerated or benign consequences for the blue and green groups and deleterious or damaging consequences for the red group. These predictions were made according to the cutoff score for each algorithm (shown in Figure 15). PolyPhen2's accuracy changed through time. PolyPhen2 prediction at the beginning of this study showed a 52.4% concordance with the functional results. The percentage of concordance increased with later builds of the algorithm, increasing to 61.9% (April 2017) and 66.66% concordance in July 2017. In comparison, PROVEAN concordance with the functional results was 71.4%, while SIFT was 66.66%. The lack of full concordance between the functional data and the predictive algorithms highlight the need for further development of the predictive algorithms and the need for functional studies to test the validity of variants selected using a bioinformatics approach.

Although it was encouraging to see a positive increasing trend in the concordance of the PolyPhen2 scores when compared to the functional data, these studies were made on a relatively small set of variants. Therefore the observed concordance percentages might not reflect the reality when testing larger numbers of variants and the other filtering criteria used in the study are modified or removed. The concordance percentages would most likely also be strictly related to the type of assay conducted. Some of the variables that may have affected the accuracy of predictive algorithms with respect to functional analysis are listed and discussed in Table 5.

This analysis highlights another problem, the level of any given variants 'deleteriousness' can change over time, in the case of A125V, the call for this variant switched from benign to deleterious and back to benign with each new release of PolyPhen2. While the changes to PolyPhen2 have increased the overall level of concordance of the algorithm with the functional studies, at the same time the fluctuations in the specific calls hampers the scientific community from forming reliable assumptions about individual variants.

**Table 5. Confounding variables in the assays conducted in this study and the possible implications of such variables.**

| **Variables** | **Possible consequences** |
|---|---|
| The assay looked at the accumulation of NF-κB through luficerase expression | Variants might have shown differences non-detectable through this assay |
| | Tested variants might have drastically different effects on other components on the pathway |
| The assay only considered one final time point (8 hours post-stimulation) | The dynamic response phenotype of each variant is masked by capturing results at a single time point. This is highlighted by the JNK-assay where the dynamic response was assessed. |
| Cells were stimulated with a physiological, optimized, concentration of hTNFα | Other stimulants (such as different cytokines that activate NF-κB like IL-1beta) might have revealed new differences between variants compared to TNF stimulation |
| Cells transfection was not 100% | Although HEK293T cells' transfection efficiency is reportedly >70% stochastically it cannot be 100% every time and this could have masked the differences between variants with more subtle effects compared to the reference A20 |
| Test cells express endogenous A20 | This might have masked the impact of recessive A20 alleles - the current assay conditions may select for dominant-negative variants |

Improvements in the way these algorithms predict deleteriousness are constantly made and similar tools have emerged (Yachdav, Kloppmann et al. 2014). Another online prediction tool (PredictProtein) was used to visualise all the possible amino acidic substitution for the A20 protein (Figure 16). Using PredictProtein it was possible to map the predicted effect of a point mutation in A20 with its location within A20. As shown in Figure 16, the most permissive areas of the protein coincide with the connecting domains. By contrast, areas in red are enriched with deleterious effects for point mutations, perfectly aligning with the known functional domains of the protein. This additional information is useful when trying to predict the functional effect of point mutations.

A step further could be including insights on the spatial location of the mutation within the 3D architecture of the protein and information about the role each amino acid assumes in the protein context (catalytic sites, phosphorylation sites, site for docking of molecular partners, etc.).

Unfortunately not every protein has been fully biochemically characterized and/or has a solved protein structure. Even if a protein structure has been solved structure on Protein Data Bank (PDB) might have low definition protein structure (> 2.5 Å), which allows for a general visualization of the protein shape and secondary structures such as α-helices and β-sheets motifs spatial location. Higher resolutions (≤ 2.5 Å) can give information on side-chains and electron densities (useful for molecular dynamics studies).

In the case of A20, a partial structures of the protein (the OTU domain - amino acid 1 to 360) has been solved by other members within this laboratory team e.g. PDB ID: 4ZS5. In total three structures have been deposited on PDB, and they have resolutions of 2.5, 2.7 and 3.2 Å respectively. This information was used to localize the tested variants within the OTU domain and to correlate their effects on NF-κB inhibitory activity with their position within the protein. The position of each of the tested variants is annotated in Figure 17. In Figure 18, A20 variants were found to be located either on protuberances on the 3D protein structure or internally to the OTU protein structure. No clear pattern emerged for the GoF and ref-hA20 comparable variants. On the other hand, 4 out of 6 of the functionally impaired variants were located either internally (I207L) or in deep pockets of the protein structure (I325N, C243Y, N102S).

In respect to the position of the variants to their location on either α-helices or β-sheets motifs, GoF or ref-hA20 comparable variants were only found to be located on α-helices. Impaired variants were located either in the middle of a β-sheets motif (I325N) or at the junction between an α-helix and a β-sheet (I207L, C243Y, N102S). No clear pattern emerged in respect to the amino acid change, although the properties of each amino acid are shown in Figure 17. A closer look at the most deleterious A20 variants across all assays revealed that the mutation from an asparagine (N) to a serine (S) at position 102 might suggest an interaction loss with two other A20's residues (A117 and A123) (Figure 19).

To further investigate this aspect, the inventors performed molecular dynamics (MD) simulations for both the N102S and another deleterious candidate A20-C243Y. The first (N102) is of interest as it sits adjacent to the catalytic cysteine, C103. This is of further interest since the role of A20 variant C103A is of debate in the literature (Lu, Onizawa et al. 2013, De, Dainichi et al. 2014). The second (C243Y) might have a more obvious implication regarding local folding, this might be due to the substitution with a larger side chain.

A readout of the MD simulations is presented in Figure 20. These simulations were performed in triplicates and represent a total of 500 ns of dynamics simulation at 300 kelvin. These simulations were performed based on the assumption that the variants will reach folding maturity. As A20's crystal structure was solved mostly from homodimers crystals these simulations were conducted on the two OTU subunits of A20, here referred as Chain A and Chain B. Figure 20a shows the root-mean-square-deviation (RMSD), a measure of the average distance between atoms of superimposed proteins. From this analysis A20-WT and N102S exhibited minor differences in the homodimer fluctuations, however C243Y exhibited some large overall fluctuations to how the dimers are oriented to each other. The latter could suggest an increased conformational flexibility that may frustrate binding to substrates or other molecular partners.

The root-mean-square-fluctuation (RMSF), a measure of the amplitude of atom motions, is presented in Figure 20b. These simulations were plot across the homodimers merging the two chains (Chain A = residues 1-360, Chain B = residues 361-720). Again A20-WT and N102S plots were very similar, suggesting similar levels of dynamics (peaks). On the other hand, the results for C243Y showed an asymmetric reduction in loop dynamics (circled in red). The radius of gyration is plotted in Figure 20c, which is a measure of compactness of the entire homodimer. Once again A20-WT and N102S plots were very similar, while C243Y showed a slight reduction in gyration, which might signify an increase in overall compactness of the homodimer.

Principal component analyses (PCA) of the simulation trajectories for the WT and the mutants are shown in Figure 20d to visualise the conformational landscape both for the homodimers and each individual chain. Overall, A20-WT showed a smoother sampling of conformational space, in which smooth transitions are also seen within the individual chains. Interestingly, both N102S and C243Y showed reduced conformational sampling. The transitions of both the homodimers and the individual chains of these mutants are also less smooth, indicating that they spend less time transitioning, with the N102S being less constrained than the C243Y mutant.

These behaviours were visualised by mapping the PCA onto an interaction network visualization of the chain structures (Figure 20e). The mobility network of A20-WT was overall fairly constrained in its dynamics with the exception of the loops (in blue) surrounding the active site (~157, ~182, ~221).

The two mutants exhibited behaviours that are at the two opposite sides of the A20-WT mobility dynamics. N102S exhibits a reduced loop dynamics, which is likely to frustrate either binding to ubiquitin or disrupt the catalytic activity even if the active site geometry is still maintained, therefore, resulting in such a severe phenotype *in vitro.* C243Y also exhibited a reduced loop dynamics, which was even more than N102S, however, the regions surrounding the mutation (~34, ~280, ~318) showed a slightly increased amount of non-native flexibility. The latter flexibility introduced near these loops could affect phosphorylation, hence disrupting protein function.

### Example 6 - Observations of A20 variants on immunity in mouse experiments

### I325N OTU variant confers heightened immunity in mice.

The A20 I325N allelic variant was identified in a genome-wide mouse ENU-mutagenesis screen segregating with increased frequencies of circulating CD44^{hi} activated/memory T cells and regulatory T cells (Figure 21) in otherwise healthy adult mice. Detailed analysis revealed the I325N mutation also increased numbers of B cells in the spleen and peritoneal cavity, and diminished IκBα within B cells, CD8 T cells, NK cells and dendritic cells (Figures 21b and 22). Macrophages immortalized from bone marrow of I325N mutant mice produced more inflammatory cytokines in response to lipopolysaccharide (LPS) than controls from wild-type mice (Figure 23). In thymocytes, I325N increased TNFα-induced NF-κB signaling in ways consistent with diminished A20-mediated inhibition (Figure 24).

In wild-type mice transplanted with mixtures of mutant and wild-type A20 bone marrow, the I325N mutation acted cell autonomously to increase B cell activation and proliferation by LPS or antigen receptors, and to increase TCR and CD28-dependent formation of FOXP3⁺ CD4⁺ regulatory T cells and their Helios⁺ FOXP3⁻ precursors within the thymus (Figures 21C, D and Figure 25). Surprisingly, I325N had a greater effect than the C103A OTU domain mutation analyzed in parallel bone marrow transplant recipients, despite C103A completely inactivating the polyubiquitin protease activity of A20 *(15, 20),* indicating I325N must diminish additional inhibitory mechanisms.

Consistent with heightened cellular markers of immunity, I325N mutant mice had greater resistance to Coxsackievirus B4 strain E2, a virus isolated from a human neonate with a disseminated fatal infection causing extensive pancreatic necrosis (35, 36). A virus dose that was lethal for 90% of wild-type C57BL/6 mice was not lethal for *Tnfaip3*^{I325N/I325N} littermates, and caused less mortality in *Tnfaip3*^{I325N/+} mice (Figure 21E and Figure 26A). Mutant mice had less infectious virus and viral RNA in the pancreas, less mRNA encoding immune response cytokines IL-1β and IFNβ, less pancreatic necrosis, higher serum IL-6, and preserved body-weight and euglycemia (Figures 21F, G and Figure 26B-G).

The homogeneous genetic background of I325N mutant mice allowed testing if heightened immunity imposed a subclinical cost or altered the insulin anabolic axis. *Tnfaip3*^{I325N/+} mice were healthy, of normal weight, and fertile, producing homozygous mutant offspring at the expected ratio. Homozygotes also appeared healthy, although their body weight was 5-20% less than heterozygous or wild-type littermates (Figure 27A), with histological analysis revealing low-grade inflammation of the pancreatic islets, colon, kidney, and liver (Figures 27B-C and 28). Pancreatic insulitis in I325N homozygotes was associated with a 50% reduction in beta cell mass (Figure 27D), although random blood glucose levels and glucose tolerance tests were normal (Figure 29). Isolated *Tnfaip3*^{I325N/I325N} islets exhibited normal basal insulin output but reduced insulin secretion when stimulated *in vitro* (Figure 29F). Islet transplant and culture experiments showed the A20 mutation acted within islet cells, exaggerating canonical and non-canonical NF-κB signaling, lowering insulin secretion, and increasing inflammatory cytokine production (Figures 27E-H, 29G-I and 30).

### A20 I325N mice are susceptible to LPS injection and poxvirus infection

In the rodent counterpart of smallpox, infection with the orthopoxvirus ectromelia virus was tolerated and controlled by wild-type mice, yet resulted in high mortality and higher viral titres in I325N homozygotes (Figure 31).

### Diminished tolerance to self-antigen caused by mutant A20

In bone marrow chimeric mice with a higher frequency of pancreatic islet-reactive CD4⁺ T cells , autoreactive T cells escaped deletion and precipitated diabetes when half the hematopoietic cells were homozygous for I325N (Figure 32A-C). The rogue islet-reactive T cells were nevertheless derived equally from A20 wild-type or I325N precursors, whereas I325N acted cell autonomously to increase MHC II and the T cell costimulator CD86 on B cells and dendritic cells and to increase frequencies of germinal centre B cells (Figure 32D-F).

### I325N mice reject transplanted grafts faster

Figure 33 shows that I325N mice reject transplanted grafts faster.

### Example 7 - A20 variant mice with heightened immunity to microbial stimulation.

Mice (C57BL/6) were engineered using CRISPR/Cas9 to express either the human I207L A20 variant or the C243Y A20 variant. Mice which were homozygous and heterozygous for each human A20 variants were produced for comparison to normal C57BL/6 mice.

Mice homozygous, heterozygous, or wild type for the three A20 variants I207L, I325N, or C243Y were challenged with a gram negative microbial stimuli of 2.5 mg/kg of LPS and serum IL-6 was measured as a marker of immune activation at time zero, 2 and 6 hours post treatment. Figure 34 shows that mice harboring the I325N and C243Y A20 variants produce more IL-6 than their wild type counterparts. Thus A20 loss of function variants confer heightened immune responsiveness to microbial challenge.

### Example 8 - A20 variant mice with heightened T cell immunity

In an additional study, splenocytes were harvested from the I207L A20 variant mice at 6-8 weeks of age and CD4 and CD8 cells were analysed for CD44 expression. As will be apparent from Figure 35, mice which were homozygous for I207L showed increased frequency of activated (CD44 high) CD4 and CD8 immune cells.

Thus, like the A20 I35N allelic variant mice described in Example 6, the A20 I207L allelic variant segregated with increased frequencies of circulating CD44^{hi} activated/memory T cells and regulatory T cells in otherwise healthy adult mice. CD44 is a marker of immune activation and indicates heightened immunity in these mice.

### Example 9 - A20 variants and the effect on immune response in humans

Using whole genome sequencing, the inventors identified in human subjects (parent and offspring trios) a pair of missense variants *in cis,* p.(T108A) and p.(I207L), which formed part of a larger haplotype consisting of multiple coding and non-coding variants (called 'hap'; see Figure 36). Both missense variants lay within the OTU domain of A20 (see Figure 36), and were rare within public variant databases (gnomAD v2.1.1 allele frequency 0.0001169-0.0001202). These variants are described herein and are also been described in Zammit *et al.,* (2019) *JCI Insight,* 4(21);e131028, the full contents of which is incorporated herein by reference. In particular, Zammit *et al.* (which is a publication by the inventors) validates that A20 variant expression inhibits human islet inflammation in transplants. The inventors also identified unique deletions within the *TNFAIP3* locus in some of the human subjects. These heterozygous *TNFAIP3* deletion mutations included; one deletion (del1) that spanned the entire *TNFAIP3* locus, while a second (del2) was a 2bp frameshift deletion (p.(N371Sfs*17)). A third non coding mutation found in the 3'UTR region of some subjects (see figure 36) was not investigated. Thus, the inventors have identified human subjects with unique deletions and gene variants within the *TNFAIP3* locus (present in both parent and offspring trios).

These human subjects were then used to assess whether *TNFAIP3* variants can tune human immune responses. Peripheral blood mononuclear cells (PBMNC) were taken from subjects and exposed to TNF stimulation in cell culture experiments. At 0, 2 and 4 hours after TNF stimulation, the levels of inflammatory mRNAs were measured in the PBMNC by RT-PCR. The results of the RT-PCR analysis for *TNFAIP3, TNF, ICAM1* and *CXCL2* gene expression in PBMCs from Trios are shown in Figure 37A and B. These data show that subjects harbouring the *TNFAIP3* hap exhibit stronger/increased responses to TNF stimulation.

It will be appreciated by persons skilled in the art that numerous variations and/or modifications may be made to the above-described embodiments, without departing from the broad general scope of the present disclosure. The present embodiments are, therefore, to be considered in all respects as illustrative and not restrictive.

The following numbered paragraphs disclose aspects and embodiments of the present disclosure.
1. A method of stratifying a subject according to the strength of their immune response, said method comprising:
   (i) determining the nucleotide sequence of the A20 gene in the subject;
   (ii) comparing the nucleotide sequence of the A20 gene determined at (i) with a reference A20 gene nucleotide sequence; and
   (iii) predicting the strength of the subject's immune response on the basis of the subject's A20 gene nucleotide sequence being identical to the reference sequence at one or more nucleotide positions or on the basis of one or more differences in the nucleotide sequence of the A20 gene in the subject relative to the reference sequence.
2. The method of paragraph 1, wherein the presence of a A20 single nucleotide variant (SNV) in the subject relative to the reference A20 nucleotide sequence is indicative that the subject's immune response will be increased or decreased relative to if the subject had the reference A20 nucleotide sequence.
3. The method of paragraph 2, wherein the presence of a A20 SNV corresponding to an A20 variant set forth in Table 1 in the subject is indicative that the subject's immune response will be increased or decreased relative to if the subject had a nucleotide sequence encoding the reference A20 protein.
4. The method of paragraph 3, wherein the presence of an A20 SNV corresponding to an A20 variant set forth in Table 2 in the subject is indicative that the subject's immune response will be increased relative to if the subject had a nucleotide sequence encoding the reference A20 protein; and/or.
5. The method of paragraph 3, wherein the presence of an A20 SNV corresponding to an A20 variant set forth in Table 3 in the subject is indicative that the subject's immune response will be decreased relative to if that subject had a nucleotide sequence encoding the reference A20 protein.
6. A method of modifying the strength of an immune response in a subject, the method comprising administering to, or expressing in, the subject an A20 variant which will increase or decrease the strength of the subject's immune response relative to the subject's immune response in the absence of the A20 variant being administered or expressed.
7. The method of paragraph 6 , comprising stratifying the subject according to the strength of their immune response based on the subject's A20 gene nucleotide sequence prior to administering to, or expressing in, the subject the A20 variant.
8. The method of paragraph 7, wherein stratifying the subject according to the strength of their immune response is performed according to the method of any one of paragraphs 1 to 5.
9. The method of any one of paragraphs 6 to 8, wherein the A20 variant administered to, or expressed in, the subject is set forth in Table 1.
10. The method of any one of paragraphs 6 to 9, comprising administering to the subject an expression vector comprising a nucleic acid coding for an A20 variant set forth in Table
11. The method of any one of paragraphs 6 to 10, comprising administering to the subject the A20 variant protein.
12. The method of any one of paragraphs 6 to 10, comprising administering to the subject a cell modified to express the A20 variant.
13. The method of paragraph 12 , wherein the cell which has been modified to express the A20 variant is an autologous cell.
14. The method of paragraph 12 , wherein the cell which has been modified to express the A20 variant is an allogeneic cell.
15. The method of any one of paragraphs 12 to 14, wherein the cell has been modified to express the A20 variant using a meganuclease, a zinc finger nuclease (ZFN), a transcription activator-like effector-based nuclease (TALEN) or a clustered regularly interspaced short palindromic repeats (CRISPR/Cas) system.
16. The method of any one of paragraphs 12 to 15, wherein the cell which has been modified to express the A20 variant is a T cell.
17. The method of paragraph 16, wherein the T cell is a chimeric antigen receptor (CAR) T cell.
18. The method of any one of paragraphs 6 to 17, wherein the A20 variant is set forth in Table 2 and the strength of the subject's immune response is increased relative to if the subject expressed a wildtype A20 only or the subject expressed an A20 allele comprising the reference A20 nucleotide sequence only.
19. The method of paragraph 18, wherein an increase in the subject's immune response comprises an increase in NF-KB and/or c-Jun amino-terminal kinase (JNK) signalling relative to if the subject expressed a wildtype A20 allele only or the subject expressed an A20 allele comprising the reference A20 nucleotide sequence only.
20. The method of paragraph 18 or paragraph 19 , wherein the subject is suffering from cancer, or wherein the subject is suffering from cancer and is receiving treatment by immunotherapy or has been prescribed a treatment by immunotherapy.
21. The method of paragraph 18 or paragraph 19, wherein the subject is suffering from, or is at risk of, infection by a pathogen.
22. The method of paragraph 21 , wherein the pathogen is a disease-causing bacteria, virus, protist or fungus.
23. The method of any one of paragraphs 6 to 15, wherein the A20 variant is set forth in Table 3 and the subject's immune response is decreased relative to if the subject expressed a wildtype A20 allele only or the subject expressed an A20 allele comprising the reference A20 nucleotide sequence only.
24. The method of paragraph 23 , wherein a decrease in the subject's immune response comprises a decrease in NF-KB and/or c-Jun amino-terminal kinase (JNK) signalling relative to if the subject expressed a wildtype A20 allele only or the subject expressed an A20 comprising the reference A20 nucleotide sequence only.
25. The method of paragraph 23 or paragraph 24 , wherein the subject has an autoimmune disease.
26. The method of paragraph 23 or paragraph 24, wherein the subject has undergone or is about to undergo organ or tissue transplantation.
27. A method of treating cancer in a subject in need thereof, comprising increasing a subject's immune response by performing the method of paragraph 18 or paragraph 19 and administering to the subject an immunotherapeutic agent.
28. The method of paragraph 27, wherein the immunotherapeutic agent is selected from the group consisting of checkpoint inhibitors, monoclonal antibodies, chimeric antigen receptor (CAR) T cells, and small molecule immune agonists.
29. A method of treating an autoimmune disease in a subject, comprising repressing a subject's immune response by performing the method of paragraph 23 or paragraph 24.
30. An isolated nucleic acid molecule comprising a nucleotide sequence encoding an A20 variant set forth in Table 1.
31. The isolated nucleic acid molecule of paragraph 30, comprising a nucleotide sequence encoding an A20 variant set forth in Table 2.
32. The isolated nucleic acid molecule of paragraph 30, comprising a nucleotide sequence encoding an A20 variant set forth in Table 3.
33. An expression vector comprising the isolated nucleic acid molecule of any one of paragraphs 30 to 32.
34. An isolated protein comprising an A20 variant set forth in Table 1.
35. An isolated protein comprising an A20 variant set forth in Table 2.
36. An isolated protein comprising an A20 variant set forth in Table 3.
37. A T cell which is modified to express an A20 variant selected from Table 2.
38. The T cell of paragraph 37, wherein said cell is a chimeric antigen receptor (CAR) T cell.
39. A pharmaceutical composition comprising (i) one or more of an isolated nucleic acid of any one of paragraphs 30 to 32 or an expression vector of paragraph 33 or an isolated protein of any one of paragraphs 34 to 36 or a T-cell of paragraph 37 or 38, and (ii) a pharmaceutically acceptable carrier, diluent or excipient.
40. A vaccine comprising an A20 protein selected from Table 2 or an isolated nucleic acid molecule comprising a nucleotide sequence encoding an A20 variant set forth in Table 2 or expression vector comprising same.

## Claims

1. A method of stratifying a subject according to the strength of their immune response, said method comprising:
(i) determining the nucleotide sequence of the A20 gene in the subject;
(ii) comparing the nucleotide sequence of the A20 gene determined at (i) with a reference A20 gene nucleotide sequence; and
(iii) predicting the strength of the subject's immune response on the basis of one or more differences in the nucleotide sequence of the A20 gene in the subject relative to the reference sequence, wherein the presence of A20 single nucleotide variant (SNV) corresponding to A20 variant 1 set forth in Table 1 in the subject relative to the reference A20 nucleotide sequence is indicative that the subject's immune response will be increased elative to if the subject had the reference A20 nucleotide sequence.

2. An isolated nucleic acid molecule comprising a nucleotide sequence encoding A20 variant 1 set forth in Table 1.

3. An expression vector comprising the isolated nucleic acid molecule of encoding an A20 variant protein comprising an amino acid sequence which is at least about 90% identical to the sequence set forth in SEQ ID NO: 1 and comprising an asparagine (N) at a position corresponding to amino acid 102 in the sequence set forth in SEQ ID NO: 1.

4. The expression vector of claim 3, comprising a polynucleotide sequence encoding an A20 variant comprising an amino acid sequence which is at least about 95% identical to the sequence set forth in SEQ ID NO: 1 and comprising an asparagine (N) at a position corresponding to amino acid 102 in the sequence set forth in SEQ ID NO: 1,
optionally wherein the polynucleotide sequence encodes an A20 variant comprising an amino acid sequence which is at least about 99% identical to the sequence set forth in SEQ ID NO: 1 provided that it comprises an asparagine (N) at a position corresponding to amino acid 102 in the sequence set forth in SEQ ID NO: 1,
optionally wherein the polynucleotide sequence encodes an A20 variant comprising the amino acid sequence set forth in SEQ ID NO: 1, with the exception of an asparagine (N) substitution at a position 102 of the sequence set forth in SEQ ID NO: 1.

5. An isolated protein comprising an amino acid sequence which is at least about 90% identical to the sequence set forth in SEQ ID NO: 1 and comprising an asparagine (N) at a position corresponding to amino acid 102 in the sequence set forth in SEQ ID NO: 1.

6. The isolated protein of claim 5, comprising an amino acid sequence which is at least about 95% identical to the sequence set forth in SEQ ID NO: 1 and comprising an asparagine (N) at a position corresponding to amino acid 102 in the sequence set forth in SEQ ID NO: 1,
optionally comprising an amino acid sequence which is at least about 99% identical to the sequence set forth in SEQ ID NO: 1 provided that it comprises an asparagine (N) at a position corresponding to amino acid 102 in the sequence set forth in SEQ ID NO: 1,
optionally comprising the amino acid sequence set forth in SEQ ID NO: 1, with the exception of an asparagine (N) substitution at a position 102 of the sequence set forth in SEQ ID NO: 1.

7. An isolated cell expressing the nucleic acid according to claim 2 or expressing the protein according to claim 5 or 6, and/or comprising the expression vector according to claim 3 or 4.

8. The isolated cell of claim 7, wherein said cell is a T cell, optionally wherein the T cell is a chimeric antigen receptor (CAR) T cell.

9. A pharmaceutical composition comprising (i) one or more of an isolated nucleic acid of claims 2 or an expression vector of claim 3 or 4, or an isolated protein of claim 5 or 6, or a cell according to claim 7 or 8, and (ii) a pharmaceutically acceptable carrier, diluent or excipient.

10. The isolated nucleic acid according to claim 2, the expression vector according to claim 3 or 4, the isolated protein according to claim 5 or 6, the cell according to claim 7 or 8 or the pharmaceutical composition according to claim 9 for use in a method of modifying the strength of an immune response in a subject, the method comprising administering to, or expressing in, the subject A20 variant 1 to increase the strength of the subject's immune response relative to the subject's immune response in the absence of A20 variant 1 being administered or expressed, optionally wherein increasing the strength of the subject immune response of the subject comprises increasing NF-κB and/or c-Jun amino-terminal kinase (JNK) signalling relative to if the subject expressed a wildtype A20 allele only or the subject expressed an A20 allele comprising the reference A20 nucleotide sequence only.

11. The isolated nucleic acid, the expression vector, the isolated protein, the cell, or the pharmaceutical composition for use according to claim 10, wherein:
i) the subject is suffering from cancer, or wherein the subject is suffering from cancer and is receiving treatment by immunotherapy or has been prescribed a treatment by immunotherapy; or
ii) wherein the subject is suffering from, or is at risk of, infection by a pathogen, optionally wherein the pathogen is a disease-causing bacteria, virus, protist or fungus.

12. The isolated nucleic acid, the expression vector, the isolated protein, the cell, or the pharmaceutical composition for use according to claim 10 or 11 for use in treating cancer in a subject in need thereof in combination with an immunotherapeutic agent, optionally wherein wherein the immunotherapeutic agent is selected from the group consisting of checkpoint inhibitors, monoclonal antibodies, chimeric antigen receptor (CAR) T cells, and small molecule immune agonists.

13. A method of modifying an isolated cell, the method comprising administering to, or expressing in, the cell A20 variant 1 to increase the strength the immune response of that cell relative to the immune response of the cell in the absence of A20 variant 1 being administered thereto or expressed therein, optionally wherein the increased strength of the immune response comprises increased NF-κB and/or c-Jun amino-terminal kinase (JNK) signalling relative to a corresponding cell expressing a wildtype A20 allele only or comprising the reference A20 nucleotide sequence only.

14. The method of claim 13, wherein the isolated cell is a T cell.

15. The method of claim 13 or 14, wherein the isolated cell is modified to express A20 variant 1 using a meganuclease, a zinc finger nuclease (ZFN), a transcription activator-like effector-based nuclease (TALEN) or a clustered regularly interspaced short palindromic repeats (CRISPR/Cas) system.
